# EUROPÄISCHE PATENTSCHRIFT

(11) **EP 2 256 196 B1**
(45) Veröffentlichungstag und Bekanntmachung des Hinweises auf die Patenterteilung: **13.07.2011**
(21) Anmeldenummer: 10172588.5
(22) Anmeldetag: 01.04.2006
(51) Int. Cl.: C12N 15/10, C12N 1/06, C12Q 1/68

(54) **Verfahren zur Behandlung einer Biomoleküle enthaltenden Probe**
Method for treating a sample that contains biomolecules
Procédé pour traiter un échantillon contenant des biomolécules

(30) Priorität: 01.04.2005 DE 102005015005
(43) Veröffentlichungstag der Anmeldung: 01.12.2010
(62) Teilanmeldung aus: 09155467.5
(73) Patentinhaber: Qiagen GmbH, 40724 Hilden (DE)
(72) Erfinder: Korfhage, Christian, 40724 Hilden (DE); Wilmer, Friederike, 40724 Hilden (DE); Löffert, Dirk, 40724 Hilden (DE); Himmelreich, Ralf, 40724 Hilden (DE); Fritz, Claudia, 40724 Hilden (DE); Rieske, Kathleen, 51399 Burscheid (DE)

(56) Entgegenhaltungen:
- EP-A- 1 044 984
- WO-A-02/00600
- WO-A-90/12881
- WO-A-94/18156
- WO-A-02/056030
- WO-A-02/057478
- WO-A-02/059360
- WO-A-03/102184
- WO-A-2005/019235
- WO-A2-2006/094974
- US-A- 5 010 183
- US-A1- 2002 197 637

## Beschreibung

Die vorliegende Erfindung betrifft ein Verfahren zur Behandlung einer Biomoleküle enthaltenden Probe. Die Erfindung betrifft ferner ein Verfahren zur Lyse einer biologischen Probe, ein Verfahren zur Stabilisierung von Nukleinsäuren und/oder Proteinen, ein Verfahren zur Verminderung von inhibitorischen Effekten in einer Nukleinsäuren und/oder Proteine enthaltenden Probe, ein Verfahren zur differentiellen Maskierung von Nukleinsäuren sowie Analyseverfahren, die auf den vorgenannten Verfahren aufbauen bzw. diese inkorporieren.

Es ist seit langem bekannt, dass die genetische Herkunft und funktionelle Aktivität einer Zelle durch Studien von Biomolekülen wie z.B. ihrer Nukleinsäuren oder Proteine bestimmt und untersucht werden kann. Die Analysen dieser Moleküle ermöglichen den direkten Zugriff auf die Ursache der Aktivitäten von Zellen. Sie sind somit indirekten, konventionellen Methoden, wie z.B. dem Nachweis von Stoffwechselprodukten, potentiell überlegen. Dies hat in den zurückliegenden Jahren zu einer starken Verbreitung von Nukleinsäure- und Proteinanalysen geführt. So werden molekularbiologische Analysen bereits in vielen Bereichen eingesetzt, z.B. in der medizinischen und klinischen Diagnostik, in der Pharmazie bei der Entwicklung und Evaluierung von Arzneimitteln, in der Lebensmittelanalytik sowie bei der Überwachung der Lebensmittelherstellung, in der Agrarwirtschaft bei der Züchtung von Nutzpflanzen und Nutztieren sowie in der Umweltanalytik und in vielen Forschungsgebieten.

Zum Beispiel lassen sich durch die Analyse der RNA, speziell der mRNA in Zellen, die Aktivitäten von Genen direkt bestimmen. Die quantitative Analyse von Transkriptmustern (mRNA-Mustern) in Zellen durch moderne molekularbiologische Methoden, wie z.B. Echtzeit-Reverse Transkriptase PCR ("Real-Time RT-PCR") oder Genexpressions-Chip-Analysen ermöglicht z.B. die Erkennung fehlerhaft exprimierter Gene, wodurch z.B. Stoffwechselkrankheiten, Infektionen oder die Entstehung von Krebs erkannt werden können. Die Analyse der DNA, z.B. aus Zellen, durch molekularbiologische Methoden, wie z.B. PCR, RFLP, AFLP oder Sequenzierung ermöglicht z.B. den Nachweis genetischer Defekte oder die Bestimmung des HLA-Typs sowie anderer genetischer Marker.

Die Analyse genomischer DNA und RNA wird auch zum direkten Nachweis von infektiösen Erregern, wie Viren, Bakterien usw. eingesetzt.

Voraussetzung für alle Analyseverfahren von Biomolekülen aus biologischen Proben ist, dass diese Biomoleküle, wie z.B. DNA, RNA oder Proteine dem entsprechenden Analyseverfahren zugänglich gemacht werde. Inhaltsstoffe von Zellen oder Organismen lassen sich in der Regel nur dann analysieren, wenn die Inhaltsstoffe im Analysemedium vorliegen, d.h. z.B. aus der Zelle oder dem Organismus in das Analysemedium überführt werden. Zu diesem Zweck werden die Zellen/Organismen aufgeschlossen, so dass die Inhaltsstoffe nicht mehr innerhalb, sondern außerhalb des Organismus oder der Zelle vorliegen und somit die Inhaltstoffe des Organismus oder der Zellen für die Analyse frei zugänglich sind. Der Aufschluss von Organismen oder Teilen von Organismen (z.B. Zellen) wird auch als Lyse bezeichnet, die aufgeschlossenen Organismen werden auch als Lysate bezeichnet.

Der Aufschluss von Proben, z.B. Organismen, sollte in zweierlei Hinsicht möglichst vollständig erfolgen: (1) Es sollten alle Bereiche der Probe, z.B. des Organismus, aufgeschlossen werden, so dass die Inhaltsstoffe möglichst aller Teile eines Organismus in der Probe freigegeben werden. (2) Der Aufschluss der Probe, z.B. der Organismen, ist erst dann abgeschlossen, wenn auch die Inhaltsstoffe der Analyse zugänglich gemacht wurden. Solange die Organismen aufgeschlossen wurden, die Inhaltsstoffe jedoch noch verdeckt werden, z.B. durch Zellkompartimente, kann eine Analyse der Inhaltsstoffe nicht quantitativ durchgeführt werden.

Derzeit sind verschiedene Lyseverfahren bekannt. Den meisten Lyseverfahren schließt sich eine Reinigung der zu analysierenden Inhaltsstoffe an, um die Inhaltsstoffe von allen Stoffen zu befreien, die einer Analyse entgegenwirken. Einige Beispiele sollen im Folgenden beschrieben werden:
(A) Lyse mit Detergentien: Detergentien sind amphipatische Moleküle, die die hydrophobe Membran von Zellen auflösen, so dass sich die Inhaltsstoffe der Zellen in die Umgebung ergießen. Zu diesen gehören z.B. nicht-ionische und ionische Detergentien. Weite Anwendung beim Aufschluss von Organismen haben die Detergentien Triton-X100, Nonidet-P40, Natriumdodecylsulfat (Sodiumdodecylsulfate, SDS), oder auch kationische Detergentien wie z.B. N-Cetyl-N,N,N-trimethyl-ammoniumbromid (CTAB) u. a. Viele Verfahren mit kationischen Detergentien wurden zur Lyse von Organismen und zur Isolierung von Nukleinsäuren (NA) beschrieben und patentiert. Nukleinsäuren können z.B. durch kationische Detergentien zu deren Schutz und Reinigung komplexiert werden. Zu diesen Verfahren zählen die in den nachfolgend genannten US-Patenten und US-Patentanmeldungen beschriebenen Verfahren: US 6,602,718, US 6,617,170, US 5,010,183, US 5,985,572, US 5,300,635, US 5,728,822, und US 2002/0146677 A1, US 2004/0048384 A1, US 2004/0115689 A1, US 2004/0014703 A1.
   Die Lyseverfahren mit Ammoniumsalzen wie z.B. N-Cetyl-N,N,N-trimethyl-ammoniumbromid (CTAB) verwenden amphipatische Ammoniumsalze, die eine längere Kohlenwasserstoffkette von mindestens 6 C-Atomen enthalten.
B) Lyse mit Wasser: Bei der Lyse mit Wasser nutzt man die Eigenschaften von semipermeablen Membranen von Zellen, die diese umhüllen. Diese Membranen sind durchlässig für Wasser, jedoch nicht für Salze oder größere Moleküle. Wird eine Zelle mit ihren Inhaltsstoffen aus Salzen und anderen, größeren Molekülen in eine Flüssigkeit überführt, die eine geringere Salzkonzentration aufweist als das Zellinnere, so nimmt die Zelle solange Wasser auf, bis die Salzkonzentration innerhalb und außerhalb der Zelle ausgeglichen ist. Ist der Konzentrationsunterschied zwischen Zellinnenraum und der äußeren Flüssigkeit ausreichend groß, so nimmt die Zelle solange Wasser auf, bis sie platzt.
C) Lyse mit organischen Lösungsmitteln: Die hydrophoben Membranen von Zellen können durch organischen Lösungsmittel zerstört werden. Hierbei werden die Lipide der Membranen wie auch lipophile Proteinbestandteile der Membranen in die organische Phase aufgenommen. Die Zellinhaltsstoffe verbleiben meist in der hydrophilen Phase oder an der Grenzfläche zwischen lipophiler und hydrophiler Phase. Bei dieser Art der Lyse findet Phenol besonders häufig Anwendung.
D) Lyse mit chaotropen Salzen: Chaotrope Salze zerstören die auf der Bildung von Wasserstoff-Brückenbindungen beruhende Struktur von Wasser, so dass die Doppelschichtstruktur von Membranen nicht mehr aufrechterhalten werden kann. Dadurch werden diese Membranen aufgelöst und die Lyse erfolgt, wobei sich die Zellinhaltsstoffe in das chaotrope Milieu ergießen. Diese Methode wird bei einer Reihe von Verfahren zur Isolierung von Nukleinsäuren und Proteinen angewendet, z.B. in den von der Firma QIAGEN GmbH, Hilden, Deutschland, kommerziell angebotenen RNeasy®-, QIAamp®-Verfahren oder dem denaturierenden Aufschluss von Zellen zur Proteinreinigung.

Für die nachfolgenden Aufreinigungsschritte oder Nachweisreaktionen, die mit dem erhaltenen Lysat durchgeführt werden, ist es wünschenswert, dass die relevanten Inhaltsstoffe der Zelle möglichst vollständig in das Lysat überführt werden, da dadurch die Menge, die zum Nachweis oder für die Isolierung einer vorgegebenen Menge eines Biomoleküls, wie DNA oder RNA benötigt wird, weiter reduziert werden kann.

Bei der quantitativen als auch der qualitativen Analyse von Biomolekülen wie z.B. Nukleinsäuren und Proteinen ist der Erhalt deren Integrität von großer Bedeutung. Insbesondere Nukleinsäuren wie DNA und in noch höherem Maße RNA unterliegen nach der Entfernung der biologischen Proben aus ihrer natürlichen Umgebung sowie nach der Lyse verschiedensten Einflüssen, die zu einer Veränderung oder einem Abbau der DNA oder RNA führen können. Beispielhaft werden hier der enzymatische Abbau dieser Nukleinsäuren oder auch der Zerfall von DNA unter dem Einfluss von in der Probe während der Lyse auftretenden Scherkräften genannt.

Es ist bekannt, dass der Erhalt der Integrität von Nukleinsäuren und Proteine durch (A) Reinigung der NA bzw. Proteine, (B) Dehydrierung, (C) Schutz vor degradierenden Enzymen oder (D) Komplexbildung erreicht werden kann. Dies wird nachfolgend kurz beschrieben:
(A) Reinigung der Nukleinsäuren bzw. Proteine: Bei der Reinigung werden Nukleinsäuren bzw. Proteine von allen Substanzen oder Molekülen befreit, die in der biologischen Probe enthalten sind und die Integrität der Nukleinsäuren bzw. Proteine dauerhaft schädigen könnten. Zu diesen Reinigungsverfahren zählen z.B. Affinitätschromatographie, Protein-Aussalzverfahren, Reinigung von Nukleinsäuren bzw. Proteinen an Festphasen. Die Festphase kann z. B ein Ionenaustauscher sein, wie er z.B. in den US-Patentschriften US 5,990,301, und US 6,020,186 beschrieben wird. Alternativ ist die Verwendung von porösen Matrices beschrieben, z.B. in den US Patenten US 6,180,778 oder US 5,496,562.
(B) Dehydrierung: Eine Dehydrierung der Nukleinsäure bzw. Proteine führt dazu, dass schädigende Prozesse, die ablaufen können, wenn die Probe mit den Nukleinsäuren bzw. Proteinen in Wasser gelöst sind, blockiert bzw. verhindert werden. Zu diesen schädigenden Prozessen gehört z.B. die enzymatische Degradierung durch Proteasen oder Nukleasen. So kann z.B. die isolierte Nukleinsäure durch Fällung (Präzipitation) mittels Salz und Alkohol dehydriert werden (Current protocols in molecular biology, z.B. S. 1.6.1. Alkaline Lysis Miniprep). Nukleinsäuren können auch in biologischen Proben wie z.B. Gewebestücken oder mikroskopischen Schnitten durch Dehydrierung vor Abbau geschützt werden. Dies ist beispielsweise in der US-Patentschrift US 6,528,641 beschrieben. In dem dort beschriebenen Verfahren werden die Reagenzien in die intakte Probe infiltriert, wobei nicht nur eine Dehydrierung, sondern auch eine Fällung von Proteinen wie z.B. Nukleasen bewirkt wird, die dann inaktiv in der dehydrierten Probe vorliegen. Insbesondere wird dort die Verwendung von Ammoniumsulfat als dehydrierendes Agens beschrieben. Eine Präzipitation von DNA wird auch in dem in der Patentanmeldung US 2002/0197637 beschriebenen Reinigung von DNA bewirkt, wobei Polyaminen zur Reinigung verwendet werden und die Polyamine zu einer Kondensation der DNA führt, wodurch bei mechanischem Aufschluss von Zellen eine Schädigung der DNA durch Scherung vermieden werden soll.
(C) Schutz vor degradierenden Enzymen: Schon lange ist bekannt, dass Proteasen und Nukleasen (Nukleinsäure abbauende Enzyme) spezifisch gehemmt werden können. So kann die DNase I z.B. durch Mg²⁺- oder Ca²⁺-Komplexbildner gehemmt werden. RNasen können z.B. durch spezifische RNase-Inhibitoren oder durch reduzierende Agenzien gehemmt werden. Solche Inhibitoren sind in den US-Patentanmeldungen US 5,552,302 und US 6,777,210 beschrieben.
(D) Komplexierung: Eine andere Variante der Reinigung sieht zum Schutz der Nukleinsäuren eine Komplexierung mit Micellen bildenden quartären Ammoniumsalzen vor. So führt z.B. die Komplexierung der Nukleinsäuren zum Schutz vor Nukleasen. Dabei werden quartäre Ammoniumsalze verwendet, die als kationische Detergenzien wirken und Micellen bilden. Dazu ist es erforderlich, dass das quartäre Ammoniumsalz zumindest eine langkettige Kohlenstoffkette als Substituenten trägt. Zu diesen oder ähnlichen Verfahren zählen auch die US-Patente US 6,602,718, US 6,617,170, US 5,010,183, US 5,985,572, US 5,300,635, US 5,728,822 und die US-Patentanmeldungen US 2002/0146677A1, US 2004/0048384A1, US 2004/0115689, und US 2004/0014703A1.

Auf ähnliche Weise beschreibt das US-Patent US 6,821,752 eine Reinigung und Extraktion von Proteinen in Gegenwart von amphipatischen Aminen, d.h. von als Detergenzien wirkenden Verbindungen.

Die zur Abtrennung oder Komplexierung der Nukleinsäuren bzw. Proteine vorgesehenen Verbindungen ist dadurch eingeschränkt, dass sie sich hinsichtlich des nachfolgend durchgeführten Isolierungsveifahrens bzw. Analyseverfahrens möglichst inert verhalten sollten, d.h. die nachgelagerte Isolierung bzw. Analyse nicht nachteilig beeinflussen sollten. Ansonsten müssen sie in einem weiteren Aufarbeitungsschritt dekomplexiert werden.

Der oben genannte Stand der Technik zeigt, dass die ausreichende Stabilisierung von Nukleinsäuren und Proteinen aus biologischen Proben in der Regel zusätzliche Aufarbeitungsschritte erfordert, durch welche die Probe aufgetrennt wird. Vorteilhaft sind somit Verfahren zur Stabilisierung, die solche zusätzliche Aufarbeitungsschritte nicht erforderlich machen und die möglichst vielseitig verwendbare stabilisierende Reagenzien einsetzen.

Ein weiteres Problem, das bei der Analyse von Biomolekülen wie Nukleinsäuren bzw. Proteinen enthaltenden Proben auftreten kann, ist die Tatsache, dass in solch einer Probe Biomoleküle eines ersten Typs durch Biomoleküle eines zweiten Typs beeinträchtigt werden können. Manchmal sollen aber auch Biomoleküle des zweiten Typs analysiert werden, wobei Biomoleküle des ersten oder eines anderen Typs dann störend bei der Analyse wirken. Um dies zu verdeutlichen, sei hier ein Beispiel unter vielen genannt: Ein Probe enthält Zellen eines bestimmten Organismus. So kann z.B. ein Nachweis einer Nukleinsäure dieser Zellen durch ein Oligonukleotid in Gegenwart von Nukleinsäure bindenden Proteinen nachteilig beeinflusst werden. Unter der Annahme, dass im vorliegenden Beispiel eine bestimmte Spezies DNA nachgewiesen werden soll, stellen die DNA bindenden Proteine eine inhibitorische Substanz dar. In einem anderen Beispiel, z.B. der Analyse von DNA bindenden Proteinen, kann aber eine DNA, die an die DNA-bindende Proteine binden kann, einen nachteiligen Einfluss auf das Analyseergebnis nehmen, da in diesem Fall die Nukleinsäure als inhibitorische Substanz wirkt und zu einer Verfälschung des Ergebnisses der Analyse führt.

Gängige Verfahren, um solche nachteiligen Wechselwirkungen zwischen verschiedenen Biomolekülen auszuschließen, sehen vor, die sich gegenseitig beeinflussenden Biomoleküle voneinander zu trennen, um Störungen zu vermeiden. Diese Verfahren sehen somit meistens einen Reinigungsschritt vor, bei denen bestimmte Spezies von Biomolekülen aus einer Probe entfernt werden. Dabei wird durch das Trennungsverfahren die Konzentration der Biomoleküle untereinander verändert, so dass die gewünschten Biomoleküle angereichert, die anderen jedoch in ihrer Konzentration reduziert werden.

Bekannte Verfahren zum Trennen von Biomolekülen schließen die folgenden Verfahren ein:
(A) Trennung von Biomolekülen durch Aussalzverfahren: Aussalzverfahren werden verwendet, um Teile der biologischen Probe, die sich durch eine bestimmte Menge an Salzen fällen lassen, aus der Probe abzutrennen. Zum einen können diese Verfahren angewendet werden, um den Teil, der gereinigt werden soll, mit Salz zu fällen. Ein solches, bekanntes Verfahren stellt die Ammoniumsulfat-Fällung von Proteinen dar. Zum anderen kann dieses Verfahren aber auch in umgekehrter Richtung angewendet werden, so dass das Material, welches gereinigt werden soll, von einer Vielzahl kontaminierender Moleküle befreit wird, selber aber nicht gefällt wird. Ein Beispiel hierzu ist die Proteinfällung durch Kaliumacetat bei der Isolierung von DNA.
(B) Trennung von Biomolekülen durch Chromatographie: Biomoleküle können durch verschiedene Chromatographieverfahren aufgrund ihrer Eigenschaften gereinigt und getrennt werden. Diese Eigenschaften können ihre Größe, ihre Ladung, ihre Hydrophobizität oder ihre Affinität zu bestimmten Oberflächen oder Haptenen betreffen, um nur einige Möglichkeiten zu benennen. Chromatographie sei hier am Beispiel der Ionenaustauschchromatograpie erläutert: Für eine Reihe von chemischen Biomolekülen bestehen zwei Möglichkeiten, durch Ionenaustauschchromatographie gereinigt zu werden. Zum einen kann das zu isolierenden Biomolekül am Ionenaustauchchromatographiematerial gebunden werden, wohingegen die kontaminierende Substanzen nicht gebunden werden und auf diese Weise von dem zu isolierenden Biomolekül abgetrennt werden können (z.B. Anionaustauschchromatographie zur Reinigung von negativ geladenen Nukleinsäuren). Zum anderen können aber auch kontaminierende Substanzen am Chromatographiematerial gebunden werden und das zu isolierende Biomolekül kann dann im Durchbruch oder im Waschpuffer aufgefangen werden (z.B. Kationenaustauschchromatographie zur Reinigung der negativ geladenen Nukleinsäuren). Beispiele eines solchen Verfahrens sind in den US-Patenten 5,990,301 und WO0248164 beschrieben.
(C) Trennung von Biomolekülen an festen Oberflächen: Eine Reihe von Oberflächen haben die Eigenschaft, in einem definierten Bindungsmilieu bestimmte Biomoleküle binden zu können. Diese Bindungseigenschaften können für die Reinigung von Biomolekülen ausgenutzt werden. Dies soll hier am Beispiel der Reinigung von Nukleinsäure an Silica-Oberflächen verdeutlicht werden. Silica-Oberflächen, ob als Mikropartikel oder als Membranen, können in Gegenwart hoher Konzentrationen chaotroper Salze Nukleinsäure binden. Andere Moleküle wie z.B. Proteine binden jedoch nicht an diese Oberflächen und werden so abgetrennt. Nach dem Waschen der Silica-Oberfläche kann die Nukleinsäure in reiner Form gewonnen werden. Beispiele hierzu sind beschreiben in den US-Patentschriften US 5,990,301, US 6,020,186 und US 6,180,778.
(D) Trennung von Biomolekülen durch selektive Komplexierung: Einige Trennungsverfahren komplexieren Biomoleküle selektiv, so dass diese durch einen Zentrifugationschritt oder Filtrierschritt von den anderen Inhaltsstoffen der Probe getrennt werden können. Beispiele hierzu sind in den US-Patentschriften US 5,728,822 und US 5,985,572 beschrieben.

In der US-Patentanmeldung US 2002/0115851 wird die Verwendung von Ammoniumsulfat zur Neutralisierung von inhibitorischen Effekten an RNA-haltigen Proben beschrieben. Diese Proben enthalten aufgereinigte Mengen an RNA. Allerdings ist in der Literatur beschrieben, dass anorganische Ammoniumsalze wie z.B. Ammoniumsulfat den Nachteil haben, dass bestimmte Teilaktivitäten von Polymerasen (z.B. 3'-5'exonuklease Aktivität) verändert werden können und sich somit die Gegenwart von Ammoniumsulfat nachteilig auf die zur nachfolgenden Analyse durchgeführte Polymerase-Reaktionen auswirken kann (Tsurumi et al., "Functional Expression and Characterization of the Epstein-Barr Virus DNA Polymerase Catalytic Subunit", Journal of Virology, Vol 67, No. 8, 1993, p. 4651-4658)

Die beschriebenen Verfahren erfordern eine vorgelagerte Abtrennung aus der Probe der sich auf die Nachweisreaktion nachteilig auswirkenden Biomoleküle oder setzen diese voraus. Es besteht somit ein Bedürfnis nach einem vereinfachten Verfahren, mit dem die inhibitorische Wirkung bestimmter Biomoleküle in Proben in dem Maße vermindert werden kann, dass eine zuverlässige Nachweisreaktion mit der Probe durchgeführt werden kann.

Ein weiteres Problem, das bei der Trennung von Nukleinsäuren häufig auftritt, liegt in der Tatsache begründet, dass Ribonukleinsäure (RNA) und Desoxyribonukleinsäure (DNA) Biomoleküle darstellen, die von ihren chemischen Eigenschaften sehr ähnlich sind. Daher besteht häufig die Schwierigkeit, diese voneinander zu trennen. Für eine Vielzahl von Applikation ist es jedoch von großer Bedeutung DNA-freie RNA oder RNA-freie DNA herzustellen: So kann beispielsweise kontaminierende genomische DNA in RNA-Präprarationen zu quantitativ falschen Ergebnissen in RT-PCR Experimenten führen.

Derzeit existieren eine Reihe von Verfahren, die es ermöglichen, RNA oder DNA differentiell anzureichern. Prinzipiell kann man verschiedene Verfahren unterscheiden:
Zum einen können Enzyme, die spezifisch DNA oder RNA abbauen, eingesetzt werden. Diese Enzyme nennt man Nukleasen. Zu diesen Nukleasen gehören die RNA abbauenden Enzyme, die RNasen, und die DNA-abbauenden Enzymen, die DNasen. Soll z.B. DNA gereinigt werden, so kann während des Reinigungsverfahrens eine RNase eingesetzt werden, die RNA-Moleküle in kleine Bruchstücke zerlegt. Diese Verfahren werden allgemein eingesetzt z.B. für Plasmid-Isolation oder Reinigung genomischer DNA. DNA-Kontaminationen in RNA-Präparationen werden alternativ z.B. mit DNase I hydrolysiert. Auch dieses Verfahren ist allgemein bekannt.
Zum anderen können chemische Verfahren verwendet werden, welche die Unterschiede in den chemischen Eigenschaften von RNA oder DNA auszunutzen. Hierzu zählt eine Reihe von Festphasen-Reinigungsverfahren. Die Festphase kann z. B ein Ionenaustauscher sein, wie beispielsweise in den US-Patenten US 5,990,301 und US 6,020,186 beschrieben, oder kann eine poröse Matrix sein, wie sie z.B. in den US-Patenten US 6,180,778 und US 5,496,562 beschrieben ist.
Andere Reinigungsverfahren betreffen das unterschiedliche Löslichkeitsverhalten von RNA oder DNA. Hierzu zählt z.B. die Reinigung von RNA in Gegenwart von wässriger Lösung eines chaotropen Salzes und saurem Phenol, wobei genomische DNA sich in der Interphase anreichert und RNA in wässriger Lösung verbleibt (Chomczynski P. & Sacchi N., 1987, Anal Biochem. 1987 Apr; 162(1):156-9, "Single-step method of RNA isolation by acid guanidinium thiocyanate-phenol-chloroform extraction").
Andere Verfahren basieren auf der selektiven Präzipitation von RNA oder DNA. Beispiele hierzu wurden bereits oben im Zusammenhang mit der Dekomplexierung von inhibierenden Biomolekülen oder der Stabilisierung von Biomolekülen beschrieben.

Auch diese Verfahren beinhalten einen separaten Verfahrensschritt, mit dem die relative Zusammensetzung der verschiedenen Biomoleküle, insbesondere der verschiedenen Nukleinsäuren oder Proteine, wesentlich verändert wird, um den gewünschten Effekt zu erzielen. Auch hier kann die Anwendbarkeit des Verfahrens davon abhängen, ob das zur Abtrennung eingesetzte Reagenz sich nachteilig auf die gewünschte Nachweisreaktion auswirkt, wodurch die Anzahl der für eine bestimmte Probenpräparation, wie z.B. für ein Analyseverfahren, zur Verfügung stehenden Reagenzien eingeschränkt wird. Auch birgt jeder Verfahrensschritt zur Auftrennung einer Probe, neben den zusätzlichen Kosten, die er erzeugt, ein Kontaminationsrisiko in sich, was ein extrem sauberes und kontrolliertes Arbeiten erforderlich macht.

Es besteht somit hinsichtlich verschiedener Aspekte ein Bedürfnis nach einer weiteren Verbesserung der Probenvorbereitung von Biomolekülen enthaltenden Proben sowie nach verbesserten Prozessierungs-, Präparations-, und Analyseverfahren von Biomolekülen.

WO-94/26867 A beschreibt ein Verfahren zur direkten Lyse von Virion enthaltenen Proben mittels eines Gemischs aus Tris- Puffer, 0,001 % SDS und 1 µg Proteinase K. Durch den Einsatz von Proteinase K wird jedoch das Protein verdaut und somit die Komplexität des Systems verringert. Dies bedeutet, dass eine gleichzeitige Analyse von Proteinen und anderer Inhaltsstoffe, wie beispielsweise Nukleinsäuren nicht mehr möglich ist.

Weiterhin beschreiben verschiedene Dokumente im Stand der Technik ganz allgemein Verfahren zur Probenaufarbeitung (beispielsweise US-2002/197637 A1). Weiterhin beschreiben WO02/059360 und W002-056030 Ribonukleinsäure (RNA) und Moleküle, welche an besagte RNA binden und die Verhinderung besagter Bindungen. WO-02/00600 und WO2006/094974 beschreiben weitere Verfahren zur Stabilisierung von RNA.

Allen diesen Lehren ist jedoch gemein, dass entweder das Protein oder die Nukleinsäuren abgetrennt werden, z. B. durch Präzipitation, oder durch die Zerstörung einer der Komponenten (Denaturierung, Verdau, Abbau). Dies bedeutet, dass sich die relative Konzentration der Nukleinsäure und Proteinen zueinander durch die Zugabe der Stickstoffhaltigen Verbindung enthaltenden Zusammensetzung verändert und eine gleichzeitige Analyse von Proteinen und anderer Inhaltsstoffe, wie beispielsweise Nukleinsäuren nicht mehr möglich ist.

Diese Aufgabe löst die vorliegende Erfindung mit dem Verfahren zur Stabilisierung von Nukleinsäuren und/oder Proteinen gemäß Anspruch 1.

Weitere vorteilhafte Aspekte, Details und Merkmale der vorliegenden Erfindungen ergeben sich aus den abhängigen Ansprüchen, der Beschreibung, den Beispielen sowie den Figuren.

Bevor die einzelnen Aspekte der vorliegenden Erfindung beschrieben werden, werden nachfolgend die folgenden Begriffe erläutert, wie sie in der vorliegenden Erfindung verstanden werden.

Organismen: Organismen sind definiert als jegliche Form von Hüllen, die Nukleinsäuren und/oder Proteine enthalten. Dazu gehören z.B. Viren, Phagen, Zellen, Zellverbände oder ganze Organismen. Diese Organismen können lebend, tot oder in ihrem Ruhezustand eingesetzt werden. Diese Organismen können in Lösung, pelletiert oder auch mit festen Phasen assoziiert oder gebunden sein. Wenn im Rahmen der vorliegenden Erfindung von "Organismen" gesprochen wird, können mehrere Organismen derselben Art, mehrere Organismen unterschiedlicher Arten oder auch ein einzelner Organismus gemeint sein. Die Organismen können dem Ursprung nach Archaebakterien, Prokaryonten oder Eukaryonten sein. Sie können tierischen, pflanzlichen oder endosymbiontischen (z.B. auch Mitochondrien oder Plastiden) Ursprungs sein. Zu den Organismen im Sinne der vorliegenden Erfindung zählen z.B. einzelne Zellen, Zellverbände, Gewebe oder ganze Tiere oder ganze Pflanzen, kultivierte Zellen, Absonderungsprodukte oder Sekrete, z.B. stabilisiertes und nicht stabilisiertes Blut, Serum, Plasma, Gewebsflüssigkeiten, Sperma, Abstriche, Sputum, Saliva, Tränenflüssigkeit, Urin, Kot, Haare bzw. Haarwurzeln, Hautschuppen, Buccal Swabs, Buffy Coat, etc.

Zelluläre Substanz: Unter zellulärer Substanz wird im Rahmen der vorliegenden Erfindung ein heterogenes Stoffgemisch verstanden, das innerhalb von Organismen vorkommt oder von diesen in die Umgebung abgegeben werden kann wie z.B. Gewebsflüssigkeiten, Plasma, Saliva, Sputum, Sekrete, etc. Die zelluläre Substanz beinhaltet Zellinhaltstoffe wie z.B. Nukleinsäuren, Proteine und andere Polymere und Metaboliten. Dazu gehören auch solche Substanzen, die der Umhüllung des Organismus zuzuordnen sind wie z.B. Hüllen, insbesondere Membranen, Capside, Zellwände, extrazelluläre Matrices, etc.

Biomoleküle: Biomoleküle sind Moleküle, die aus Organismen stammen oder deren im Rahmen eines Nachweisverfahrens wie z.B. durch eine Amplifikation enthaltene Umwandlungsprodukte. Zu den Biomolekülen gehören z.B. Nukleinsäure oder Proteine oder andere Moleküle aus Organismen. Die Biomoleküle können z.B. durch die Lyse von Organismen gewonnen werden.

Nukleinsäure: Nukleinsäure (NA) wird im Rahmen der vorliegenden Erfindung als Desoxyribonukleinsäure (DNA), Ribonukleinsäure (RNA) oder Peptidnukleinsäure (PNA) verstanden. Desoxyribonukleinsäure (DNA) und Ribonukleinsäure (RNA) kommen natürlicherweise in Organismen vor, können aber auch außerhalb von Organismen vorkommen oder diesen hinzugefügt worden sein. Die Länge der Nukleinsäure kann unterschiedlich sein. Die Nukleinsäure kann durch Veränderungen modifiziert sein. Z.B. können eine oder mehrere der Nukleobasen der Nukleinsäure modifiziert sein. Auch die Zuckereinheiten in der Nukleinsäure können modifiziert (z.B. durch Methoxygruppen) oder gar ersetzt sein, wie beispielsweise in PNA. Die Nukleinsäure kann Basenanaloga wie z.B. non-Purin oder non-Pyrimidin Analoga oder Nukleotidanaloga wie z.B. PNA enthalten. Die DNA und/oder RNA kann Anhänge enthalten wie z.B. Proteine oder Aminosäuren.
Der Begriff DNA, wie er in der vorliegenden Erfindung verstanden wird, lässt sich weiter unterteilen in natürlich vorkommende und nicht natürlich vorkommende Arten, wobei auch nicht-natürlich vorkommende Arten in Organismen gelangen können (Beispielsweise durch Transfektion oder Transformation). Genomische DNA (gDNA) enthält die Sequenzinformation, die den Bauplan des Organismus enthält. Im Rahmen der vorliegenden Erfindung werden auch Teilbereiche, auch sehr kleine Teilbereiche, aus dieser Sequenz als genomische DNA bezeichnet, die nicht der vollständigen Sequenz entsprechen. Unter genomischer DNA werden im Rahmen der vorliegenden Erfindung auch deren modifizierte Formen verstanden. Unter den Begriff "DNA" fällt hier auch Plasmid-DNA. Plasmid DNA ist eine extrachromosomale DNA mit einem eigenen "orign of replication". Unter Plasmid-DNA werden im Rahmen der vorliegenden Erfindung auch deren modifizierte Formen verstanden. Unter den Begriff "DNA", wie er hier verstanden wird, fallen auch mtDNA oder ptDNA. mtDNA oder ptDNA ist die genomische DNA aus Mitochondrien oder Plastiden. Unter mtDNA oder ptDNA werden hier auch deren modifizierte Formen verstanden. Unter den Begriff "RNA", wie er hier verstanden wird, fallen die natürlich vorkommenden und nicht natürlich vorkommenden Arten, wobei auch nicht-natürlich vorkommende Arten in Organismen gelangen können (z.B. durch Transfektion). mRNA, hnRNA und rRNA sind große RNA-Moleküle (50 nt bis viele kB groß), die als Abschrift aus einem codierenden Bereich erstellt werden können. Unter mRNA bzw. rRNA werden im Rahmen der vorliegenden Erfindung auch deren modifizierte Formen verstanden.

tRNA, miRNA, siRNA etc. sind kleine RNA-Moleküle, die bei der Translation wirksam sind. Unter tRNA, miRNA oder siRNA werden hier erneut auch deren modifizierte Formen verstanden.

Protein: Als Protein wird jede Aminosäure (AS), die mit weiteren Aminosäuren über einen Peptidbindung in einer linearen oder auch verzweigten Anordnung verknüpft ist. Das Protein kann als Monomer, als Dimer oder Multimer vorkommen, wobei Homomere oder Heteromer gebildet werden können. Protein können natürlich vorkommende Proteine sein oder auch synthetische Proteine sein. Die Proteine können modifiziert vorliegen. Diese Proteine können z.B. Enzyme, strukturelle Proteine, Rezeptoren, Ionenkanäle und andere Transporter, Extrazellulär-Matrix-Proteine, Transkriptionsregulatoren, Nukleinsäure-bindende Proteine, Hüll- und Schutzproteine, Speicherproteine etc. sein.

Lyse: Unter dem Begriff "Lyse" wird im Rahmen der vorliegenden Erfindung ein Prozess verstanden, der dazu führt, dass Nukleinsäuren und/oder Proteine aus Organismen heraus in die Umgebung abgegeben werden. Dabei kann die Struktur der Organismen zerstört werden, z.B. die Hülle des Organismus aufgelöst werden. Unter dem Begriff "Lyse" wird im Rahmen der vorliegenden Erfindung auch verstanden, dass zelluläre Substanzen aus den Organismen durch kleine Öffnungen, z.B. Poren etc. in der Hülle des Organismus austreten können, ohne dass dabei die Struktur der Organismen zu zerstören. Beispielsweise können durch Lysereagenzien Poren erzeugt werden. Des Weiteren ist im Rahmen der vorliegenden Erfindung unter dem Begriff "Lyse" zu verstehen, dass Nukleinsäuren und/oder Proteine von Organismen, die schon strukturell zerstört erscheinen oder kleine Öffnungen aufweisen, durch die Verwendung eines Additivs herausgespült werden können. Durch die Lyse wird ein Lysat erzeugt. Der Prozess der Lyse kann durch enzymatische, chemische oder physikalische Lyse-Verfahren erfolgen.

Enzymatische, chemische oder physikalische Lyse-Verfahren: Unter dem Begriff "enzymatische Lyse-Verfahren" wird im Rahmen der Erfindung ein Prozess verstanden, der die Lyse von Organismen oder die Freisetzung von Biomolekülen mittels Enzymen unterstützt. D.h. die Enzyme für enzymatische Lyse-Verfahren sind dadurch gekennzeichnet, dass sie eine Lyse von Organismen oder eine Freisetzung von Biomolekülen unterstützt. Zu solchen Enzymen gehören z.B. Proteasen, Lysozyme, Glucanasen, Peptidasen, Lipasen, Hyaluronidasen, Kollagenasen, Nukleasen, Amylasen, Hydrolasen, Pectinasen, etc. Dem einschlägigen Fachmann sind weitere Enzyme bekannt, die im Sinne der vorliegenden Erfindung eine Lyse von Organismen oder eine Freisetzung von Biomolekülen unterstützen. Diese Enzyme können auf klassische Weise aus Organismen oder durch biotechnologische Verfahren gewonnen werden. Zu diesen Enzymen gehören auch genetisch oder chemisch veränderter Enzyme, sowie hitzelabile oder hitzestabile Enzyme.
Unter dem Begriff "chemische Lyse-Verfahren" wird im Rahmen der Erfindung ein Prozess verstanden, der die Lyse von Organismen oder die Freisetzung von Biomolekülen mittels Chemikalien unterstützt. Hierzu zählen z.B. Detergentien, Säure, Lauge, organische Lösungsmittel, chaotrope Substanzen etc.
Unter dem Begriff "physikalische Lyse-Verfahren" wird im Rahmen der Erfindung ein Prozess verstanden, der die Lyse von Organismen oder die Freisetzung von Biomolekülen mittels physikalischer Verfahren unterstützt. Hierunter werden mechanische, thermische, aber auch solche Verfahren verstanden, die auf der Wirkung von Wellen oder Druckdifferenzen beruhen. Mechanische Lyse-Verfahren sind z.B. Verfahren, die eine Scherung oder Zerkleinerung herbeiführen wie z.B. Mühlen, Messer, Quetschen, enge Kanülen, Siebe, Mörser oder Beschuss mit Teilchen oder Verfahren, die zu einer Homogenisierung der Probe führen können. Bei thermischen Lyse-Verfahren führt eine Temperaturänderung zu dem Aufschluss von Organismen oder der Freisetzung von Biomolekülen. Diese können z.B. auf Erhitzung oder auf Abkühlung, z.B. unter den Gefrierpunkt, beruhen. Verfahren, die auf die Wirkung von Wellen beruhen, sind z.B. Behandlungen mit Schall- oder elektromagnetischen Wellen. Auch Druckveränderungen können die Lyse unterstützen wie z.B. in Form von Quetschen oder plötzlich Druckveränderungen (z.B. French-Press).

Dem einschlägigen Fachmann sind weitere enzymatische, chemische oder physikalische Lyse-Verfahren bekannt und es ist auch bekannt, dass diese Verfahren in unterschiedlichen Kombinationen verwendet werden können.

Lysepuffer: Unter dem Begriff "Lysepuffer" wird im Rahmen der vorliegenden Erfindung allgemein eine zur Lyse verwendete Flüssigkeit verstanden. Diese Flüssigkeit kann eine Lösung aus verschiedenen Lysereagenzien darstellen. Der Lysepuffer kann, muss aber nicht ein Puffersystem zur Einstellung des pH umfassen. Z.B. stellen die in den erfindungsgemäßen Verfahren verwendeten flüssigen Zusammensetzungen "Lysepuffer" dar.

Reaktionssystem: Unter Reaktionssystem wird im Rahmen der vorliegenden Erfindung jede Form von System verstanden, mit dem eine Komponente, die in einer Probe enthalten ist, modifiziert, identifiziert oder in einer biologischen, chemischen oder physikalischen Reaktion verändert werden kann. Das Reaktionssystem enthält zumindest eine nachweisbare Menge der zu analysierenden Komponente, z.B. DNA und/oder RNA.

Differentiellen Analyse: Die differentielle Analyse ist ein Reaktionssystem, in dem eine Probe verwendet wird, in der zumindest zwei unterschiedliche Komponenten, z.B. die Nukleinsäuren DNA und RNA, enthalten sind, und in dem mindestens eine der Komponenten, z.B. eine der oben genannten Nukleinsäuren nicht analysiert werden soll.

Nukleinsäure-Präparationen bzw. Protein-Präparationen: Nukleinsäure-Präparationen sind Nukleinsäuren, die durch Präparation einer nukleinsäurehaltigen Probe oder eines nukleinsäurehaltigen Stoffgemisches erhalten wurden, wobei die Präparation ein Verfahren darstellt, mit dem die relative Konzentration unterschiedlicher in der Probe oder dem Stoffgemisch enthaltener Nukleinsäure und Proteinspezies beeinflusst wird, so dass eine oder mehrere bestimmte Spezies Nukleinsäuren in der Nukleinsäure-Präparation angereichert werden. Unter die Präparationsverfahren fallen somit z.B. Aufreinigungverfahren, wie z.B. die Reinigung von Nukleinsäuren aus einer Probe über eine Silica-Membran (z.B. RNeasy oder QIAamp®, erhältlich von der Firma QIAGEN GmbH, Hilden, Deutschland), wodurch die Konzentration von Proteinen gegenüber der Nukleinsäure verglichen mit der Probe oder dem Stoffgemisch vor dem Präparationsverfahren deutlich abnimmt. Analoges gilt für Protein-Präparationen.

Im Folgenden werden verschiedene Nukleinsäure-Hybrdisierungs- bzw. Sequenzierungs- und Synthesemethoden beschrieben, wie sie im Rahmen der erfindungsgemäßen Analyseverfahren zur Anwendung kommen können. Bei diesen Methoden handelt es sich um gängige Methoden, wie sie z.B. in den Anmeldungen W0 01/498802 oder US 2002/0115851 beschrieben sind.

Bindereaktion: Eine Bindereaktion ist eine Reaktion, in der mindestens zwei Bindepartner (z.B. zwei Nukleinsäure-Moleküle in einer Hybridisierung oder zwei Proteine in einer Antikörper-Antigen Wechselwirkung oder Protein und Nukleinsäure in einer Bindereaktion) miteinander wechselwirken. Bindereaktionen werden z.B. durchgeführt, um Nachweisreaktionen oder Quantifizierung durchzuführen oder diese vorzubereiten.
Eine Probe wird mit einer Sonde in Kontakt gebracht, so dass die Probe von der Sonde gebunden werden kann. Die Sonde kann so modifiziert sein, dass ein einfacher Nachweis der Sonde möglich ist. Solche Modifikationen können z.B. Kopplungen von Fluorophoren, radioaktiven Substanzen oder Enzymen sein.

Der Nukleinsäure-Erststrang ist der Nukleinsäurestrang, der während der primerabhängigen Nukleinsäure-Synthese durch ein Enzym, wie z. B. DNA oder RNA-Polymerase, Ligase etc. gebildet wird und komplementär zur Sequenz einer Zielnukleinsäure ist. Der Nukleinsäure-Zweitstrang ist der Nukleinsäurestrang, welcher während der primerabhängigen Nukleinsäure-Synthese durch ein Enzym gebildet wird und komplementär zur Sequenz des Erststrangs ist.

Als eine Zielnukleinsäure wird eine Nukleinsäure verstanden, die in einem Reaktiossystem reagiert z.B. spezifisch einen definierten Primer oder Oligonucleotid bindet, d. h. mit diesem hybridisieren, kann. In diesem Beispiel dient die Zielnukleinsäure nach der Primer-Bindung bei der primerabhängigen Nukleinsäure-Synthese durch ein Enzym, z. B. DNA- oder RNA-abhängige DNA-Polymerasen, als Matrize des zu synthetisierenden Nukleinsäure-Strangs, wobei die Sequenz des zu synthetisierenden Nukleinsäure-Strangs komplementär zur Sequenz der Zielnukleinsäure ist. Spezifische Bindung bedeutet dabei nicht, dass 100% Komplementarität zwischen Zielnukleinsäure und hybridisierendem Teil des Primers oder Oligonucleotid vorliegen muss. Bis zu maximal 50% der Basen im hybridisierenden Bereich zwischen Primer und Zielnukleinsäure dürfen nicht-komplementär sein, um noch brauchbare Ergebnisse zu erzielen. Gute bis sehr gute Ergebnisse können erreicht werden, wenn nicht mehr als 30% der Basen des hybridisierenden Teils des Primers und der Zielnukleinsäure nicht komplementär sind. Je höher der Grad an Komplementarität zwischen dem hybridisierenden Teil des Primers und der Zielnukleinsäure ist, desto spezifischer und effektiver ist der Primer.

Die Zielsequenz ist zum einen die Sequenz, welche die Primer-Bindestelle enthält, und zum anderen die Sequenz, die in 3'-Richtung (stromabwärts) von der Primer-Bindestelle liegt. Bei der Primer-abhängigen Nukleinsäure-Synthese wird eine Nukleinsäuresequenz gebildet, die komplementär zur Zielsequenz ist.

Primer sind Starter für die Nukleinsäure-Synthese. Es handelt sich meist um kurzkettige, einzelsträngige Oligoribo- oder Oligodesoxyribonukleotide, die zu einem Bereich auf einem einzelsträngigen Nukleinsäuremolekül komplementär sind (siehe oben) und mit diesem zu einem Doppelstrang reagieren können. Das freie 3'-Hydroxy-Ende in diesem Doppelstrang dient als Substrat für Nukleinsäure-Polymerasen, wie beispielsweise DNA-Polymerasen, und als Startstelle für die Aufpolymerisierung des gesamten Einzelstrangs zum Doppelstrang. Primer sind allgemein definiert als ein oligomeres Startermolekül, das sequenzspezifisch an die Zielnukleinsäure binden kann. Die Sequenz der Zielnukleinsäure, die den Primer bindet, wird als Primer-Bindestelle bezeichnet. Dabei kann ein Primer durchaus auch verschiedene Zielnukleinsäuren binden, wenn diese alle die gleiche oder eine ähnliche Sequenz als Primer-Bindestelle enthalten. Ein erster oder primärer Primer P1 wird als "anti-sense Primer" und ein zweiter oder sekundärer Primer P2 wird als "sense Primer" definiert.

Als Zweitstrangsynthese-Primer wird ein "sense Primer" verstanden, der entweder ein sekundärer, der Reaktion von außen zugeführter Primer P2 oder ein durch die Rückfaltung des Nukleinsäure-Erststrangs gebildeter Primer (sog. "Hairpin Loop") ist.

Eine Primer-Bindestelle ist die Sequenz der Zielnukleinsäure, die den Primer durch Hybridisierung binden kann. Die Sequenz der Primer-Bindestelle ist zu mindestens 30%, bevorzugt mindestens 50%, besonders bevorzugt 100%, komplementär zur Sequenz des Primers.

Der hybridisierende Anteil des Primers ist der Sequenzteil des Primers, der an das primäre Zielmolekül hybridisiert und zu der Sequenz der Primer-Bindestelle des primären

Zielmoleküls in mindestens 50% der Basen komplementär ist. Das primäre Zielmolekül ist das Nukleinsäuremolekül, das in die enzymatische Reaktion eingesetzt wird. Es ist nicht Produkt dieser Reaktion.

Die durch Enzyme, wie insbesondere DNA- und RNA-abhängige DNA-Polymerasen katalysierte, Primer-abhängige Nukleinsäure-Synthese ist eine Schlüsselreaktion bei der cDNA-Synthese, DNA-Sequenzierung und bei Amplifikationsverfahren wie z. B. der Polymerase-Kettenreaktion (PCR)/RT-PCR oder den isothermalen Amplifikationsverfahren NASBA (Nucleic Acid Sequence Based Amplification), 3SR (Self-Sustained Sequence Replication; beinhaltet die Verwendung von RNase H), 2SR (Self-Sustained Sequence Replication; ähnlich wie 3SR, aber ohne Verwendung von RNase H), TMA (Transcription Mediated Amplification), SDA (Strand Displacement Amplification), LCR (Ligase Chain Reaction) und verwandte Methoden. Die Effizienz der Primer-abhängigen Nukleinsäure-Synthese wird beeinflusst durch die Aktivität des Enzyms für die Nukleinsäure-Polymerisation (z. B. einer DNA-Polymerase), durch die Zielnukleinsäure und durch Effizienz und Spezifität der Primer-Hybridisierung. Nachfolgend werden einige Anwendungsbeispiele für primer-abhängige Nukleinsäure- Syntheseprozesse näher beschrieben.

Primer-abhängige Nukleinsäure-Synthese-Reaktionen findet man z. B. bei Erst- und Zweitstrang-cDNA-Synthesen, bei der DNA-Sequenzierung, bei auf Primer-Bindung basierenden Mutageneseprozessen und anderen Verfahren. Dabei werden sequenzspezifisch gestartete Nukleinsäure-Synthesereaktionen durchgeführt unter Einsatz von Enzymen, wie z. B. RNA- oder DNA-abhängigen DNA-Polymerasen, wobei die sequenzspezifisch gestarteten DNA-Synthesereaktionen folgende Schritte enthalten:
1. Sequenzspezifische Hybridisierung eines ersten Primers P1 an eine zu diesem komplementäre Sequenz der Zielnukleinsäure (RNA oder DNA); und
2. Verlängerung des Primers P1 durch den katalysierten Einbau von Desoxyribonukleotiden an das freie 3'-OH Ende des zu verlängernden Primers P1 mittels eines Enzyms, wie z. B. einer RNA-abhängigen oder DNA-abhängigen DNA-Polymerase, wobei die Zielnukleinsäure als Matrize dient, und der Primer P1 insgesamt oder zumindest im 3'- Bereich eine Sequenz enthält, die komplementär zur einer bestimmten Sequenz der Zielnukleinsäure ist und sequenzspezifisch an diese Zielsequenz hybridisieren kann und wobei der Primer P1 im 5'-Bereich eine Sequenz enthalten kann, die nicht komplementär zur Zielnukleinsäure ist und nicht an der Zielnukleinsäure hybridisieren kann und zusätzliche Funktionen enthalten kann. Die Synthese läuft bis zum Ende der Zielnukleinsäure (Matrize) oder kann vor dem Ende unterbrochen werden, womit die Erststrang-Nukleinsäure-Synthese beendet ist.
3. Die Nukleinsäure-Zweitstrangsynthese beginnt durch die sequenzspezifische Hybridisierung eines Zweitstrangsynthese-Primers (ZP). Der Zweitstrangsynthese-Primer kann ein separater, der Reaktion zugeführter Primer P2, ein Degradierungsprodukt der Zielnukleinsäure oder ein durch die Rückfaltung des Nukleinsäure-Erststrangs gebildeter Primer (Hairpin Loop) sein.
4. Verlängerung des ZP durch den katalysierten Einbau von Nukleotiden an das freie 3'-OH Ende des zu verlängernden ZP mittels eines Enzyms, wobei der Nukleinsäure-Erststrang der Zielnukleinsäure als Matrize dient; der ZP enthält insgesamt oder zumindest im 3'- Bereich eine Sequenz, die komplementär zu einer bestimmten Sequenz der Zielnukleinsäure ist und sequenzspezifisch an diese Zielsequenz hybridisieren kann und der ZP kann im 5'-Bereich eine Sequenz enthalten, die nicht komplementär zur Zielnukleinsäure ist und als Folge davon nicht an der Zielnukleinsäure hybridisieren kann, zusätzliche Funktionen enthalten kann und wobei der ZP die gleiche oder eine zu dem Primer P1 unterschiedliche Sequenz aufweisen kann.

Die primerabhängige Synthese-Reaktion ist auch bei der Sequenzierung eine Schlüsselreaktion. Das Verfahren der Nukleinsäure-Sequenzierung folgt im Wesentlichen dem schon vorstehend unter primerabhängige Nukleinsäure-Synthese-Reaktion beschriebenen Verfahren und beschränkt sich auf die Schritte (1) und (2), wobei im Falle einer DNA-Synthese anstelle der Desoxyribonukleotide eine Mischung aus Desoxyribonukleotiden und Didesoxyribonukleotiden zum katalysierten Einbau durch Enzyme, wie z. B. RNA- oder DNA-abhängige DNA-Polymerasen, verwendet wird.

Dabei werden die sequenzspezifisch gestarteten Nukleinsäure-Synthesereaktionen zur Sequenzierung durch Enzyme durchgeführt, wie beispielsweise RNA- oder DNA-abhängige DNA-Polymerasen, wobei die sequenzspezifisch gestarteten DNA-Sequenzreaktionen folgende Schritte enthalten:
1. Sequenzspezifische Hybridisierung eines Primers P1 an eine zu diesem komplementäre Sequenz der Zielnukleinsäure (RNA oder DNA).
2. Verlängerung des Primers P1 durch den katalysierten Einbau von Desoxyribonukleotiden und Didesoxyribonucleotiden an das freie 3'-OH Ende des zu verlängernden Primers P1 durch ein Enzym, z. B. eine RNA-abhängige oder DNA-abhängige DNA-Polymerase, wobei die Zielnukleinsäure als Matrize dient, der Primer P1 insgesamt oder zumindest im 3'-Bereich eine Sequenz enthält, die komplementär zur einer bestimmten Sequenz der Zielnukleinsäure ist und sequenzspezifisch an diese Zielsequenz hybridisieren kann und der Primer P1 im 5'-Bereich eine Sequenz enthalten kann, die nicht komplementär zur Zielnukleinsäure ist, nicht an der Zielnukleinsäure hybridisieren kann und zusätzliche Funktionen enthalten kann.

Primerabhängige Nukleinsäure-Synthese-Reaktionen findet man ebenfalls bei Erst- und Zweitstrang-DNA-Synthesen der Polymerase-Kettenreaktion (PCR). Dabei werden die sequenzspezifisch gestarteten DNA-Synthesereaktionen durchgeführt durch hitzestabile RNA- und/oder DNA-abhängige DNA-Polymerasen, wobei die sequenzspezifisch gestarteten DNA-Synthesereaktionen folgende Schritte enthalten:
1. Initiale thermische Denaturierung der Zielnukleinsäure;
2. Sequenzspezifische Hybridisierung zweier Primer P1 und P2 an eine zu diesen komplementäre Sequenz der Zielnukleinsäure;
3. Verlängerung der Primer P1 und P2 durch den katalysierten Einbau von Desoxyribonukleotiden an das freie 3'-OH Ende der zu verlängernden Primer P1 und P2 durch eine hitzestabile RNA-abhängige oder DNA-abhängige DNA-Polymerase, wobei die Zielnukleinsäure als Matrize dient;
4. die Primer-Verlängerungsprodukte ihrerseits nach Eingang in Schritt (1) wieder als Matrize zur Primer-Hybridisierung der Primer P1 und P2 dienen; und
5. die Schritte (1) bis (4) beliebig häufig wiederholt werden können und so die Nukleinsäure mit den gewünschten Eigenschaften synthetisiert werden kann, wobei die Primer P1 und P2 insgesamt oder zumindest im 3'-Bereich eine Sequenz enthalten, die komplementär zu einer bestimmten Sequenz der Zielnukleinsäure ist und sequenzspezifisch an diese Zielsequenz hybridisieren kann und wobei die Primer P1 und P2 im 5'-Bereich eine Sequenz enthalten können, die nicht komplementär zur Zielnukleinsäure ist und nicht an der Zielnukleinsäure hybridisieren kann und zusätzliche Funktionen enthalten kann.

Sequenzspezifisch gestartete Nukleinsäure-Synthesereaktionen sind des weiteren Bestandteil von auf in-vitro Transkription basierenden, isothermalen, exponentiellen Nukleinsäure-Amplifikationsverfahren wie z. B. NASBA (Nucleic Acid Sequence Based Amplification), 3SR (Self-Sustained Sequence Replication), 2SR (Self-Sustained Sequence Replication ähnlich 3SR, jedoch ohne Verwendung von RNase H), TMA (Transcription-Mediated Amplification), und ähnlichen Verfahren. Bei diesen Methoden verwendet man Primer, RNA- und DNA-abhängige DNA-Polymerasen, RNA- Polymerasen und geeignete Reaktionsbedingungen, wobei die exponentiellen Nukleinsäure- Amplifikationsverfahren folgende Schritte umfassen:
1. Herstellung eines einzigen Reaktionsmediums, welches eine Zielnukleinsäure, einen ersten Primer P1, einen zweiten ZP, eine RNA-abhängige DNA Polymerase, eine DNA-abhängige DNA-Polymerase, eine DNA-abhängige RNA-Polymerase, Ribonukleotide und Desoxyribonukleotide enthält;
2. Einstellung von Reaktionsbedingungen, so dass Amplifikationszyklen aufrechterhalten werden können, wobei
3. die Erststrang-DNA-Synthese komplementär zur Zielnukleinsäure durchgeführt wird durch die Hybridisierung eines Primers P1 an eine komplementäre Zielsequenz, gefolgt von einer Verlängerung des Primers P1 durch Desoxyribonukleotide unter Verwendung einer RNA- oder DNA-abhängigen DNA-Polymerase, wobei die Zielnukleinsäure entweder eine DNA oder eine RNA sein kann, und
4. die Synthese der Zweitstrang-DNA komplementär zur Erststrang-DNA durchgeführt wird durch die enzymatische, thermische oder chemische Denaturierung oder Degradation der Zielnukleinsäure von der Erststrang-DNA und die Hybridisierung eines ZP an die komplementäre Erststrang-DNA gefolgt von einer Verlängerung des ZP durch Desoxyribonukleotide unter Verwendung einer RNA- oder DNA-abhängigen DNA-Polymerase, wobei die Primer P1 und ZP im 3'-Bereich eine Sequenz enthalten, die komplementär zur einer bestimmten Sequenz der Zielnukleinsäure ist und sequenzspezifisch an diese Zielsequenz hybridisieren kann und wobei mindestens einer der Primer P1 oder ZP oder beide Primer im 5'-Bereich eine Sequenz enthalten, die nicht komplementär zur Zielnukleinsäure ist und nicht an der Zielnukleinsäure hybridisieren kann und eine DNA-abhängige RNA-Polymerase Promotorsequenz enthält,
5. von dem aus eine in-vitro Transkription des in den Schritten (1) bis (4) synthetisierten DNA-Moleküls durch die Verwendung von DNA-abhängiger RNA-Polymerase und Ribonukleotiden durchgeführt werden kann, wobei die entstehenden in-vitro Transkripte wiederum als Matrize dienen und unter sequenzspezifischer Hybridisierung von Primer P1 und ZP erneut Eingang in DNA-Erst- und Zweitstrang-Synthese finden, gefolgt von in-vitro Transkription. Auf diese Weise wird eine exponentielle Amplifikation der Nukleinsäure erreicht.

Sequenzspezifisch gestartete Nukleinsäure-Synthesereaktionen sind außerdem Bestandteil von auf in-vitro Transkription basierenden, linearen, isothermalen Nukleinsäure-Amplifikationsverfahren. Diese Methoden werden unter Verwendung sequenzspezifisch bindender Primer, von RNA- und DNA-abhängigen DNA-Polymerasen, und RNA-Polymerasen bei geeigneten Reaktionsbedingungen durchgeführt, wobei isothermale, lineare Nukleinsäure-Amplifikationen folgende Schritte umfassen:
1. Herstellung eines einzigen Reaktionsmediums, welches eine Zielnukleinsäure, einen ersten Primer P1, einen zweiten ZP, eine RNA-abhängige DNA Polymerase, eine DNA-abhängige DNA-Polymerase, eine DNA-abhängige RNA-Polymerase, Ribonukleotide und Desoxyribonukleotide enthält,
2. Durchführung der Erststrang-DNA-Synthese komplementär zur Zielnukleinsäure durch die Hybridisierung eines Primers P1 an eine komplementäre Zielsequenz gefolgt von einer Verlängerung des Primers P1 durch Desoxyribonukleotide unter Verwendung einer RNA- oder DNA-abhängigen DNA-Polymerase, wobei die Zielnukleinsäure entweder eine DNA oder eine RNA sein kann;
3. die Synthese der Zweitstrang-DNA wird komplementär zur Erststrang-DNA durchgeführt durch die enzymatische, thermische oder chemische Wegnahme der Zielnukleinsäure von der Erststrang-DNA und die Hybridisierung eines ZP an die komplementäre DNA des Erststrangs. Die Nukleinsäure-Zweitstrang-Synthese beginnt durch die sequenzspezifische Hybridisierung eines zweiten ZP, eines Degradierungsprodukts der Zielnukleinsäure oder durch die Rückfaltung des Nukleinsäure-Erststrangs an die zu diesem komplementäre Sequenz des Nukleinsäure-Erststrangs der Zielnukleinsäure. Darauf folgt eine Verlängerung des ZP durch Desoxyribonukleotide unter Verwendung einer RNA- oder DNA-abhängigen DNA-Polymerase, wobei die Primer P1 und ZP im 3'-Bereich eine Sequenz enthalten, die komplementär zu einer bestimmten Sequenz der Zielnukleinsäure ist und sequenzspezifisch an diese Zielsequenz hybridisieren kann und wobei mindestens einer der Primer P1 oder ZP oder die Primer P1 und ZP im 5'-Bereich eine Sequenz enthalten, die nicht komplementär zur Zielnukleinsäure ist und nicht an der Zielnukleinsäure hybridisieren kann, und eine DNA-abhängige RNA-Polymerase eine Promotorsequenz enthält, von der aus eine in-vitro Transkription des in den Schritten (1) bis (3) synthetisierten DNA-Moleküls durch die Verwendung von DNA-abhängiger RNA-Polymerase und Ribonukleotiden durchgeführt werden kann. Auf diese Weise wird eine lineare Amplifikation der Nukleinsäure erreicht.

Strand-Displacement-Amplification (SDA): SDA ist ein Oberbegriff für eine Reihe von Methoden, die zur Amplifikation von Nukleinsäure führen. Die Reaktionen enthalten mindestens die zu amplifizierende Nukleinsäure, einen geeigneten Puffer, Primer, dNTPs und eine Polymerase. Die Reaktion führt zu einer Nukleinsäure-Synthese, wobei ein Doppelstrang entwunden wird, um einen so entstandenen Einzelstrang als Matrize nutzen zu können. Die Entwindung kann durch die Polymerase selbst durchgeführt werden (ursprüngliche SDA). Alternativ kann die Entwindung des Doppelstranges mittels einer Helicase durchgeführt werden (Helicase-abhängige Amplifikation). Werden Primer eingesetzt, die eine Zufallssequenz aufweisen, so nennt man die Reaktion Multiple-Displacement. Amplification (MDA). Von VanNess wurde eine Methode entwickelt, bei der die Primer durch Einzelstrang-Brüche der zu amplifizierenden Zielnukleinsäure entstehen (US-Patentanmeldung 2003/138800, "Exponential amplification of nucleic acids using nicking agents"). Von der Firma NuGen (USA) wurde eine Methode entwickelt, bei RNA/DNA Fusionsprimer verwendet werden (SPIA). Eine andere SDA-Reaktion verwendet circuläre DNA zur Amplifikation (Rolling Circle Amplifikation). Hierbei wird DNA synthetisiert. Eine ähnliche Methode amplifiziert RNA (Rolling Transcription Amplification).

Primerunabhängige Synthesereaktionen: Primerunabhängige Synthesereaktionen sind Reaktionen, die keinen Primer benötigen. Ein Beispiel einer primerunabhängigen Synthesereaktion ist die Transkriptionsreaktion. Bei einer Transkriptionsreaktion wird ausgehend von einem Gen, das einen geeigneten Promotor für eine RNA-Polymerase enthält, in Gegenwart von Nukleotiden, einem geeigneten Reaktionsmilieu und RNA-Polymerase ein Transkript synthetisiert. Dieses findet ohne die Verwendung eines Primers statt. Es wird eine Vielzahl von Transkripten von einem Gen abgelesen und synthetisiert.

Ein weiteres Beispiel für eine primerunabhängige Synthesereaktion ist die Translationsreaktion. Hierbei wird ein RNA-Transkript mit einem Translationsapparat in Kontakt gebracht. Dieser besteht aus Ribosomen und allen weiteren Komponenten, die nötig sind, um Proteine oder Polypeptide herzustellen. Dazu gehören z.B. tRNAs, AminoacyltRNA-Synthetasen etc. Wird ein Transkript mit dieser Reaktionslösung in Kontakt gebracht, können mittels dieser primerunabhängigen Reaktion Proteine hergestellt werden.

Im Folgenden werden weitere Reaktionssysteme beschrieben, wie sie im Rahmen der erfindungsgemäßen Analyseverfahren zur Anwendung kommen können.

Enzymatische Tests sind Reaktionssysteme, in denen katalytisch wirksame Biomoleküle mithilfe ihrer katalytischen Aktivität gemessen werden können. Hierzu zählen z.B. Tests von Enzymen wie Dehydrogenasen, Hydrolasen, Polymerasen, Phosphorylasen, Phosphatasen, Kinasen, etc.

Nutzung von Oberflächen von Biomolekülen: Hierunter wird verstanden eine Quantifizierung, Detektion, Anreicherung oder auch Abreicherung von Biomolekülen unter Nutzung der spezifischen Merkmale ihrer Oberflächen. Hierzu zählen z.B. Nachweise von Biomolekülen mit Hilfe von Antikörpern, Aptameren oder Bindepartnern, Kofaktoren, Proteinen aus Multienzymkomplexen oder auch Protein oder Nukleinsäuren für Nachweise von Nukleinsäure-Protein-Interaktionen.

Degradierung von Biomolekülen. Hierunter wird die Zerstörung mindestens einer Eigenschaft eines Biomoleküls verstanden. Z.B können Enzyme die Größe von Biomolekülen nur dann reduzieren, wenn die Enzyme die Struktur des Zielmoleküls erkennen. Auch hierzu können die erfindungsgemäßen stickstoffhaltigen Verbindungen beitragen.

Allgemein betrifft die vorliegende Erfindung ein Verfahren zur Probenvorbereitung für ein nachfolgendes Präparations-, Prozessierungs- oder Analyseverfahren einer zumindest eine Spezies Nukleinsäure und/oder eine Spezies Protein enthaltenden Probe, wobei das Verfahren die folgenden Schritte umfasst:
A) Bereitstellen einer Probe, die zumindest eine Spezies einer Nukleinsäure und/oder eines Proteins umfasst,
B) In-Kontakt-Bringen der Probe mit einer flüssigen oder festen Zusammensetzung, um eine flüssige Probenzubereitung zu erzeugen, wobei die Zusammensetzung zumindest eine stickstoffhaltige Verbindung enthält, die ausgesucht ist aus der Gruppe bestehend aus a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen heterocyclischen Verbindungen umfassen, d) Aminen vom Typ R¹R²NR³_{,} wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, j) stickstoffhaltige Verbindung ausgewählt aus den unter a-i beschriebenen Gruppen mit einer zusätzliche Derivatisierung, sowie Mischungen aus zwei oder mehreren dieser Verbindungen.

Dabei kann die Probe ein Organismus oder ein Nukleinsäure und/oder Protein enthaltendes Stoffgemisch sein. Die in Schritt B) erzeugte flüssige Probenzubereitung weist in allen Ausführungsformen des erfindungsgemäßen Verfahrens einen pH zwischen 7,1 und 14, bevorzugt einen pH zwischen 7,1 und 12 und ganz besonders bevorzugt einen pH zwischen 7,4 und 10 auf.

Unter einem Prozessierungsverfahren soll hier jede enzymatische, chemische oder physikalische Umsetzung der in der Probe vorliegenden Biomoleküle verstanden werden. Spezielle Aspekte dieses Verfahrens werden nachfolgend mit Bezug auf besondere Ausgestaltungen als weitere Aspekte dieser Erfindung erläutert. Diese speziellen Ausgestaltungen betreffen wie nachfolgend erläutert ein Verfahren zur Lyse einer biologischen Probe, ein Verfahren zur Stabilisierung von Nukleinsäuren und/oder Proteinen, ein Verfahren zur Verminderung von inhibitorischen Effekten in einer Probe enthaltend Nukleinsäuren und/oder Proteine, ein Verfahren zur selektiven Maskierung von Nukleinsäuren in einer Probe sowie die jeweils auf diesen Verfahren aufbauenden Analyseverfahren.

Demgemäß betrifft ein Aspekt der vorliegenden Erfindung ein Verfahren zur Lyse einer biologischen Probe, die zumindest eine Spezies einer Nukleinsäure und/oder zumindest eine Spezies eines Proteins in einer Hülle enthält. Als Hülle kommen hier insbesondere biologische Hüllen wie z.B. Membranen, Capside oder Zellwände in Frage, die Hüllen sind aber nicht darauf beschränkt. Die Probe wird zur Erzeugung eines Lysats mit einer flüssigen oder festen Zusammensetzung in Kontakt gebracht, die zumindest eine stickstoffhaltige Verbindung enthält, die ausgesucht ist aus der Gruppe bestehend aus:
a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen heterocyclischen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindenden Antibiotika, i) Verbindungen, die in der kleinen Grube der DNA binden, j) stickstoffhaltige Verbindung ausgewählt aus den unter a-i beschriebenen Gruppen mit einer zusätzliche Derivatisierung. sowie Mischungen aus zwei oder mehreren dieser Verbindungen. Unter "Probe, die zumindest eine Spezies einer Nukleinsäure und/oder zumindest eine Spezies eines Proteins in einer Hülle enthält" fallen Organismen, wie sie im Rahmen der vorliegenden Erfindung verstanden werden. Ob die Probe mit einer flüssigen oder festen Zusammensetzung in Kontakt gebracht wird, hängt davon ab, ob die Probe ausreichend Flüssigkeit enthält, dass durch die Lyse ein für die weitere Verwendung ausreichend flüssiges Lysat erhalten wird. Vorzugsweise wird die Zusammensetzung flüssig sein. Dabei kann die stickstoffhaltige Verbindung in einem Lösemittel gelöst oder suspendiert vorliegen.

Durch das erfindungsgemäße Lyseverfahren wird es möglich, im Vergleich mit herkömmlichen Lyseverfahren höhere Mengen an Nukleinsäuren und/oder Proteinen in das Lysat zu überführen. Es wird vermutet, dass die stickstoffhaltige Verbindung zu einer effektiveren Auflösung der Hüllen der in der Probe enthaltenen Organismen führt, wodurch höhere Mengen der Zellinhaltstoffe in das Lysat gelangen können.

In einer bevorzugten Ausführungsform dieses Verfahrens wird die Lyse so durchgeführt, dass die mindestens eine Spezies der Nukleinsäure und/oder des Proteins in dem erzeugten Lysat gelöst oder suspendiert wird. Um zu erreichen, dass bei der Lyse die mindestens eine Spezies der Nukleinsäure und/oder des Proteins in dem erzeugten Lysat gelöst oder suspendiert wird, sind die Lysebedingungen so zu wählen, dass eine Präzipition der zumindest einen Nukleinsäure und/oder des Proteins nicht eintritt. Mit den Begriffen "gelöst" und "suspendiert" ist im Rahmen der vorliegenden Erfindung gemeint, dass die zumindest eine Spezies Nukleinsäure bzw. Protein in der flüssigen Phase der Probenzubereitung, beispielsweise des Lysats, verbleiben und damit einer nachfolgenden Analyse durch ein entsprechendes Nachweis- oder Analyseverfahren zugänglich sind. Dies steht im Gegensatz zu den präzipitierten Bestandteilen des Lysats, die nicht unmittelbar einem Nachweis- oder Analyseverfahren zugeführt werden können, sondern erst durch entsprechende Verfahrensschritte wieder in die flüssige Phase überführt werden müssen. Hierbei ist die Art der Probe, mit der die Lyse durchgeführt wird, zu berücksichtigen. Mögliche weitere Komponenten in der Zusammensetzung, welche die stickstoffhaltige Verbindung enthält, sind hierbei ebenfalls zu berücksichtigen und gegebenenfalls die geeigneten Bedingungen mittels einfacher Routineuntersuchungen für ein bestimmtes System aus Probe und Zusammensetzung zu ermitteln. Dies kann beispielsweise dadurch erfolgen, dass beobachtet wird, ob bei der Lyse eine Präzipitation erfolgt. Ist dies der Fall, so kann das Präzipitat unter Verwendung von standardmäßig angewendeten Analyseverfahren und -methoden auf seine Zusammensetzung untersucht werden, insbesondere dahingehend ob das Präzipitat die zu lösende/suspendierende Spezies Nukleinsäure und/oder Protein enthält. Insbesondere ist in dieser Ausführungsform die Konzentration der stickstoffhaltigen Verbindung in der Zusammensetzung so zu wählen, dass die Nukleinsäure in dem Lysat gelöst oder suspendiert bleibt und die stickstoffhaltige Verbindung somit keine Präzipitation dieser Spezies Nukleinsäure und/oder Protein in dem Lysat bewirkt.
Dadurch, dass die zumindest eine Spezies Nukleinsäure und/oder Protein in dem Lysat gelöst und/oder suspendiert bleibt, ist es beispielsweise möglich, dass das Lysat direkt in einem Reaktionssystem einer nachfolgenden Analyse der Spezies Nukleinsäure und/oder des Proteins unterzogen werden kann. Andere Zellinhaltsstoffe, die nicht analysiert werden sollen, können während der Lyse präzipitiert werden und so aus dem flüssigen Probenvolumen entfernt werden. Die zumindest eine Spezies Nukleinsäure und/oder Protein ist somit eine Spezies, die für ein nachfolgendes Präparations-, Prozessierungs- oder Analyseverfahren vorgesehen ist.

In einer weiteren Ausführungsform wird die Lyse in Gegenwart eines Trägermaterials zur Immobilisierung der mindestens einen Spezies Nukleinsäure oder Protein durchgeführt. Dabei können herkömmliche Trägermaterialien, wie sie üblicherweise in der Nukleinsäure- bzw. Proteinanalytik zum Einsatz kommen, z.B. Microarrays oder so genannte "Beads". Unter "Beads" werden hier Mikropartikel verstanden, die eine Oberfläche aufweisen, an die Moleküle anbinden können. Eine solche Oberfläche kann z.B. durch eine entsprechende Oberflächenbehandlung erreicht werden. Beispiele solcher Materialien sind Oligotex® und Liquichip®, erhältlich von der Firma QIAGEN, Hilden, Deutschland.

In einer weiteren Ausführungsform des erfindungsgemäßen Verfahrens enthält die Probe zumindest zwei Spezies aus der Gruppe bestehend aus Nukleinsäuren und Proteinen. Die Lyse wird derart durchgeführt, dass die zwei oder mehrere bestimmte Spezies Nukleinsäure und/oder Proteine in dem erzeugten Lysat enthalten sind, insbesondere derart, dass diese in dem Lysat gelöst oder suspendiert werden. Beispielsweise können in dem Verfahren Nukleinsäuren wie DNA und RNA, insbesondere gDNA und RNA, in dem Lysat enthalten sein. Alternativ können z.B. selektiv Proteine in Lösung/Suspension gehalten werden. Wie oben beschrieben sind bei der Auswahl der Lysebedingungen die Art der Probe sowie die verschiedenen in der zur Lyse verwendeten Zusammensetzung ggf. enthaltenen Lysereagenzien zu berücksichtigen, insbesondere die Menge der eingesetzten stickstoffhaltigen Verbindung.

In noch einer weiteren Ausführungsform des erfindungsgemäßen Verfahrens enthält die Probe zumindest zwei Spezies aus der Gruppe bestehend aus Nukleinsäuren und/oder Proteinen und die Lyse wird derart durchgeführt, dass eine Mehrheit, vorzugsweise im Wesentlichen alle, der in der biologischen Probe enthaltenen Spezies Nukleinsäuren und/oder Proteine im erzeugten Lysat im Wesentlichen enthalten, insbesondere gelöst und/oder suspendiert werden. Dabei können entweder die in der Probe enthaltenen Spezies Nukleinsäuren oder alle in der Lösung enthaltenen Spezies Proteine gelöst und/oder suspendiert werden. Dies bedeutet, dass während der Lyse im Wesentlichen keine Präzipitation, wie sie z.B. zur Entfernung von Nukleinsäuren oder Proteinen aus Lysaten eingesetzt wird, erfolgt. Enthält die Probe mehrere Spezies Nukleinsäuren und/oder Proteine und sollen zwei, mehrerer oder alle dieser Spezies nach der Lyse in dem Lysat gelöst und/oder suspendiert bleiben, ist es besonders bevorzugt, dass sich die relative Konzentration dieser verschiedenen Spezies Nukleinsäuren und/oder Proteine zueinander durch die Lyse nicht verändert und somit die relative Konzentration dieser Spezies im Lysat gegenüber der relativen Konzentration dieser Spezies in der Probe im Wesentlichen unverändert bleibt. Erneut gilt, dass wie oben bereits ausgeführt, die Art der Probe sowie die verschiedenen ggf. verwendeten Lysereagenzien und die Art der verwendeten stickstoffhaltigen Verbindung bei der Durchführung der Lyse zu berücksichtigen sind und geeignete Routinetests gegebenenfalls durchzuführen sind, um geeignete Bedingungen für eine vorgegebene Probenart zu ermitteln.

Durch die oben beschriebenen Lyseverfahren, in denen zumindest zwei Spezies Nukleinsäure und/oder Proteine im Lysat gelöst und/oder suspendiert werden, ist es möglich das Lysat, das diese Spezies enthält zur Analyse bzw. Nachweis einer jeder dieser Spezies in einem nachfolgenden Analyse- bzw. Nachweisverfahren einzusetzen.
Dabei kann die stickstoffhaltige Verbindung neben einer effektiveren Lyse, die zu einer erhöhten Konzentration der gewünschten Spezies Nukleinsäure und/oder Proteins im Lysat führt, auch eine stabilisierende Wirkung auf die im Lysat gelösten und/oder suspendierten Spezies Nukleinsäure und/oder Protein haben. Darüber hinaus können durch die Verwendung der stickstoffhaltigen Verbindung in der zur Lyse verwendeten Zusammensetzung inhibierende Wechselwirkungen und Effekte in dem Lysat vermindert oder unterdrückt werden. Weiterhin ist es durch die Verwendung der stickstoffhaltigen Verbindung möglich, eine bestimmte Spezies Nukleinsäure in dem Lysat zu maskieren, so dass diese Spezies Nukleinsäure keine nachteiligen Auswirkungen auf das Analyse- bzw. Nachweisverfahren einer weiteren in dem Lysat enthaltenen Spezies nimmt. Auf diese speziellen zusätzlichen Aspekte wird nachfolgend noch detaillierter eingegangen.

In einer weiteren bevorzugten Ausführungsform des Verfahrens werden als Nukleinsäurespezies DNA und/oder RNA in dem Lysat gelöst und/oder suspendiert. In einer anderen Ausführungsform werden ein oder mehrere Spezies Protein in dem Lysat gelöst und/oder suspendiert.

In einer weiteren bevorzugten Ausführungsform des erfindungsgemäßen Lyseverfahrens umfasst die zur Lyse eingesetzte Zusammensetzung neben der stickstoffhaltigen Verbindung weitere Lysereagenzien zumindest ein Reagenz aus der Gruppe der Komplexbildner, Detergentien, Substanzen zur Volumeneinengung, und/oder Lösemittel enthält. Als Komplexbildner kommen insbesondere EGTA (Ethylenglykol-bis(2-aminoethylether)-N,N,N',N'-tetraessigsäure) und EDTA (Ethylendinitrilotetraessigsäure) in Frage. Mögliche Detergenzien sind insbesondere Triton X 100 (Polyethylenglykol-tert.-octylphenylether), Nonidet-P40 (Nonylphenyl-polyethylenglykol), n-Ocytlglucosid und N-Cetyl-N,N,N-trimethyl-ammoniumbromid. Als Substanzen zur Volumeneinengung kommen insbesondere Polyethylenglykole verschiedener Kettenlängen in Frage. Als Lösemittel werden vorzugsweise H₂O oder Phenol oder Mischungen daraus eingesetzt. Vorzugsweise werden die Komplexbildner in einer Menge eingesetzt, dass sie jeweils in der Probenzubereitung, z.B. dem Lysat in einer Konzentration von 0,1 bis 10 mM vorliegen. Werden als zur Lyse eingesetzten Zusammensetzungen flüssige Zusammensetzungen eingesetzt, also ein Lysepuffer, so liegen die Komplexbildner vorzugsweise jeweils in einer Konzentration von 0,1 bis 10 mM vor. Die Detergentien werden vorzugsweise in einer Menge eingesetzt, dass sie in der Probenzubereitung, z.B. dem Lysat mit einer Konzentration von 0,01-10 Vol% vorliegen. Wird ein Lysepuffer verwendet, so machen die Detergentien vorzugsweise 0,01 bis 10 Vol-% des Lysepuffers aus. Vorzugsweise werden die Reagenzien zur Volumeneinengung in einer Menge eingesetzt, dass sie in der Probenzubereitung in einer Konzentration von 0,01 bis 5 Vol-% vorliegen. Wird ein Lysepuffer verwendet, wo enthält dieser vorzugsweise zwischen 0,01 und 5 Vol-% Reagenzien zur Volumeneinengung. Die Lysereagenzien werden bevorzugt als Lösung, können aber auch anders z.B. in Form einer Festsubstanz zugegeben werden. Für die vorliegende Erfindung werden als Zusammensetzungen für die Lyse insbesondere Lysepuffer verwendet, bestehend aus H₂O als Lösemittel, zumindest einer der oben genannten stickstoffhaltigen Verbindungen als Additiv und optional Komplexbildnern, Detergentien, und/oder Substanzen zur Volumeneinengung.
Insbesondere können Lysepuffer verwendet werden, die alle dieser Komponenten aufweisen. Dementsprechend betrifft ein weiterer Aspekt der vorliegenden Erfindung Zusammensetzungen für die Lyse einer biologischen Probe, wobei die Probe zumindest eine der im Rahmen der verschiedenen erfindungsgemäßen Verfahren verwendete stickstoffhaltige Verbindung sowie zumindest ein weiteres Lysereagenz, wie sie im Zusammenhang mit dem erfindungsgemäßen Lyseverfahren oben beschrieben sind.
Des Weiteren kann die Lyse unter mechanischer Einwirkung oder mit enzymatischer Unterstützung durchgeführt werden. Als Mittel zur mechanischen Unterstützung können Mörser, das Anlegen hoher Drücke, enge Kapillare sowie die Verwendung von Filtereinheiten in Frage. Eine enzymatische Unterstützung der Lyse kann beispielsweise durch die Verwendung von Proteasen, Lysozymen, Cellulasen, Pektinasen unterstützt werden, wodurch der Abbau der Struktur der Organismen in der Probe weiter unterstützt wird.
Durch diese Unterstützung kann die Lyse gegebenenfalls noch effektiver gestaltet werden, d.h. die Ausbeute an Nukleinsäure und/oder Protein in dem Lysat erhöht oder die Lyse beschleunigt werden. Des Weiteren ist es möglich, dass die Probe vor der Lyse mit einem Waschpuffer, insbesondere einem hypotonen Waschpuffer, gewaschen wird.

In einer bevorzugten Ausführungsform des erfindungsgemäßen Lyseverfahrens wird die Menge der eingesetzten stickstoffhaltigen Verbindung so gewählt, dass die Konzentration der stickstoffhaltigen Verbindung in dem erzeugten Lysat zwischen 0,001 mM bis 1 M beträgt, vorzugsweise zwischen 0,001 bis 100 mM, insbesondere bevorzugt 0,001 bis 30 mM, 0,001 bis 20 mM, und speziell 0.001 bis 19 mM oder 0.001 bis 15 mM. Bei Verwendung von heterocyclischen Verbindungen als der stickstoffhaltigen Verbindung, insbesondere von Imidazol, kann die Menge der Verbindung so ausgewählt werden, dass in dem Lysat eine Konzentration von 0,01 bis 20 mM, besonders bevorzugt von 0,01 bis 15 mM an Imidazol eingestellt wird. Für Verbindungen, die Aminofunktionalitäten aufweisen gilt hier in der Regel, dass die Konzentration der stickstoffhaltigen Verbindung um so geringer sein kann, umso mehr Aminofunktionalitäten in der stickstoffhaltigen Verbindung enthalten sind, die mit der Nukleinsäure oder dem Protein wechselwirken können.

In einer weiteren bevorzugten Ausführungsform des erfindungsgemäßen Lyseverfahrens ist die zumindest eine Nukleinsäurespezies eine DNA-Spezies. In einer weiteren bevorzugten Ausführungsform des erfindungsgemäßen Lyseverfahrens ist die zumindest eine Nukleinsäurespezies eine RNA-Spezies. In einer weiteren bevorzugten Ausführungsform des erfindungsgemäßen Lyseverfahrens ist die zumindest eine Spezies von Biomolekül eine Protein-Spezies.

In weiteren bevorzugten Ausführungsformen des erfindungsgemäßen Lyseverfahrens ist die stickstoffhaltige Verbindung ausgewählt aus der Gruppe bestehend aus:
a) Polyaminen, die vorzugsweise ausgesucht sind aus der Gruppe bestehend aus offenkettigen und cyclischen Polyaminen mit 2, 3, 4, 5 oder 6 Aminogruppen. Unter dem Begriff "Polyamin", werden im Rahmen der vorliegenden Erfindung insbesondere Verbindungen verstanden, die eine gesättigte Kohlenstoffkette mit endständigen Aminogruppen umfassen. Die endständigen Aminogruppen können primäre (H₂N-), sekundäre (R₁NH-) oder tertiäre Aminogruppen (R₁R₂N-) sein und können Teil einer cyclischen Gruppe sein. Die Reste R₁ und R₂ können hier unabhängig voneinander C₁-C₅-Alkylgruppen sein. Vorzugsweise sind die endständigen Aminogruppen primäre oder sekundäre Aminogruppen. Die gesättigte Kohlenstoffkette kann mit einer wechselnden Anzahl von sekundären (-NH-) oder tertiären (-NR₁-), vorzugsweise sekundären Aminogruppen unterbrochen sein. R₁ kann hier erneut eine C₁-C₅-Alkylgruppe sein. Die gesättigte Kohlenstoffkette kann beispielsweise offenkettig unverzweigt, offenkettig verzweigt oder zyclisch sein. Vorzugsweise handelt es sich bei der Kohlenstoffkette oder den einzelnen, die verschiedenen Aminogruppen verbindenden Kohlenstoffketten um Alkylengruppen -(CH₂)ₙ-, wobei n eine ganze Zahl von 1 bis 6, vorzugsweise von 2 oder 3 ist. Die zylclischen Kohlenstoffketten können sowohl reine Kohlenwasserstoffringe als auch gesättigte, stickstoffhaltige Ringgruppen wie z.B. Piperidin oder Piperazin sein. Die Ringe enthalten dabei vorzugsweise 4 bis 6 Ringatome. Die stickstoffhaltigen Ringgruppen können an mindestens einem der im Ring enthaltenen Stickstoffatome mit einer gesättigten Kohlenstoffkette mit endständigen Aminogruppen substituiert sein. Diese Kohlenstoffkette kann wieder durch sekundäre oder tertiäre, vorzugsweise sekundäre, Aminogruppen unterbrochen. Auch hier sind die Kohlenstoffketten vorzugsweise Alkylengruppen -(CH₂)ₙ-, die wobei n eine ganze Zahl von 2 bis 6 sein kann und vorzugsweise 2 oder 3 ist. Obwohl Polyamine mit 2 bis 6 Aminogruppen bevorzugt sind, können die Polyamine auch mehr Aminogruppen enthalten. Insbesondere werden unter dem Begriff Polyamine auch polymere geradekettige oder verzweigte Polyamine wie z.B. Polyethylenimine oder Vinylamine verstanden.
   Als Polyamine können insbesondere können Ethylendiamin, Trimethylendiamin bzw. Putrescin, Spermidin, Cadaverin, Diethylentriamin, Spermin, Triethylentetramin, Tetraethylenpentamin, Pentaethylenhexamin, 1,4-Bis(3-aminopropyl)-piperazin, 1-(2-Aminoethyl)piperazin, 1-(2-Aminoethyl)piperidin, 1,4,10,13-Tetraoxa-7,16-diazacyclooctadecan, Poly(1-vinylpyrrolidon-co-2-dimethylaminoethylmethacrylat) und Tris(2-aminoethyl)amin) und ähnliche verwendet werden. Besonders bevorzugt ist die Verwendung von unverzweigten Polyaminen mit 2 bis 6 Aminogruppen, insbesondere Ethylendiamin, Trimethylendiamin bzw. Putrescin, Spermidin, Diethylentriamin, Spermin, Triethylentetramin, Tetraethylenpentamin, Pentaethylenhexamin, wobei die Verwendung von Spermidin insbesondere bevorzugt ist.
b) Aminosäuren, insbesondere α-Aminosäuren. Dabei können die Aminosäuren sowohl in der D- oder L-Form oder als Racemat vorliegen. Es können proteinogen und nicht proteinogene, aber insbesondere proteinogene Aminosäuren verwendet werden. Es können sowohl polare als auch apolare Aminosäuren verwendet werden. Dabei umfassen die apolaren Aminosäuren sowohl die aliphatischen (z.B. Glycin, Alanin, Valin, Leucin, und Isoleucin) und aromatische Aminosäuren (z.B. Phenylalanin, Tyrosin, Tryptophan). Polare Aminosäuren können sowohl neutrale, basische als auch saure Aminosäuren umfassen, bzw. die Amide der sauren Aminosäuren. Die neutralen Aminosäuren können z.B. ausgesucht sein aus den Iminosäuren wie Prolin oder den Aminosäuren mit Hydroxy-Gruppen wie z.B. Serin und Threonin oder den schwefelhaltigen Aminosäuren wie z.B. Cystein und Methionin. Weiterhin können die Aminosäuren ausgesucht sein aus den basischen Aminosäuren wie z.B. Lysin, Arginin und Histidin. Die Aminosäuren können auch ausgesucht sein aus den sauren Aminosäuren wie z.B. Asparaginsäure, Glutaminsäure oder deren Amiden wie z.B. Asparagin oder Glutamin. Vorzugsweise werden Aminosäuren wie Arginin, Prolin, Tryptophan und Glutaminsäure als stickstoffhaltige Verbindung verwendet. In einer weiteren Ausführungsform werden Aminosäuren als stickstoffhaltige Verbindung eingesetzt, die keine reduzierend wirkende Thiogruppe aufweisen.
c) Heterocyclischen Verbindungen, die ausgesucht sind aus der Gruppe der fünf- oder sechsgliedrigen Ringe bzw. der sechsgliedrigen Ringe mit anelliertem fünfgliedrigen Ring, wobei der fünfgliedrige Ring, der sechsgliedrige Ring und/oder der anellierte fünfgliedrige Ring 1 bis 3 Stickstoffatome aufweist. Die fünf- oder sechsgliedrigen Ringe und die anellierten Ringe können ungesättigt, teilweise ungesättigt oder aromatisch sein. In den Ringverbindungen können die jeweiligen Ringglieder Substituenten aufweisen, die ausgesucht sind aus der Gruppe bestehend aus H, C₁-C₆-Alkylgruppen, =O, -OH, =S, -SH, =NH, -NH₂, Alkyl-O-, Alkyl-S-, Alkylamino- und Dialkylaminogruppen, wobei diese Alkylgruppen (d.h. die Alkylgruppen in den Alkyl-O-, Alkyl-S-, Alkylamino- und Dialkylaminogruppen) C₁-C₅-Alkylgruppen, vorzugsweise C₁-C₃-Alkylgruppen sind. Des Weiteren können die Ringglieder mit F-, Cl-, Br- oder J substituiert sein. Die heterocyclischen Verbindungen können in den jeweiligen fünf- oder sechsgliedrigen Ringgruppen als weiteres Heteroatom ein oder mehrere O- oder S- Atome aufweisen. Bevorzugte heterocylische Verbindungen sind aromatische stickstoffhaltige 5-gliedrige Ringverbindungen der allgemeinen Formel I: 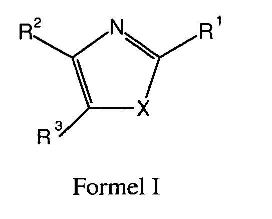 wobei X ausgesucht ist aus NH oder S, R¹, R², und R³ unabhängig voneinander ausgesucht sind aus der Gruppe bestehend aus -H, -F, -Cl, -Br, -J, -OH, -SH, -NH₂, -C(=O)OH, - C(=O)NH₂, Alkyl-O-, Alkyl-S-, Alkylamino- und Dialkylaminogruppen, wobei diese Alkylgruppen C₁-C₅-Alkylgruppen, vorzugsweise C₁-C₃-Alkylgruppen, sind Besonders bevorzugt sind die Verbindungen Imidazol, Thiazol und Aminothiazole, insbesondere 2-Aminothiazol.
   Weiterhin bevorzugte heterocyclische Verbindungen sind aromatische sechsgliedrige Ringverbindungen der allgemeinen Formel II: 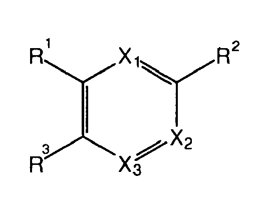 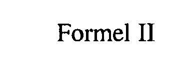 wobei X₁ ausgesucht ist aus der Gruppe bestehend aus N, O, S sowie CR⁴, X₂ ausgesucht ist aus der Gruppe bestehend aus N, O, S sowie CR⁵ und X₃ ausgesucht ist aus der Gruppe bestehend aus N, O, S sowie CR⁶, wobei zumindest eine der Gruppen X₁, X₂ oder X₃ N darstellt, und R¹, R², R³, R⁴, R⁵ und R⁶ unabhängig voneinander ausgesucht sind aus der Gruppe bestehend aus H, C₁-C₆-Alkylgruppen, -OH, -SH, -NH₂, -F, -Cl, -Br, -I, Alkyl-O-, Alkyl-S-, Alkylamino- und Dialkylaminogruppen, wobei diese Alkylgruppen (d.h. die Alkylgruppen in den Alkyl-O-, Alkyl-S-, Alkylamino- und Dialkylaminogruppen) C₁-C₅-Alkylgruppen, vorzugsweise C₁-C₃-Alkylgruppen, sind. Vorzugsweise stellen X, X und/oder X₃ N dar. Weiterhin bevorzugt ist, dass entweder nur X, N, oder X₁ und X₂ oder X₁ und X₃ N sind. R¹ bis R⁵ sind vorzugsweise ausgesucht aus der Gruppe bestehend aus H und C₁-C₃-Alkyl, und ist vorzugsweise H oder Methyl. Besonders bevorzugt sind die Verbindungen 2,3-Dimethylpyrazin, Pyridin, und Pyrimidin.
   Weiterhin bevorzugt sind Verbindungen gemäß der allgemeinen Formel III: 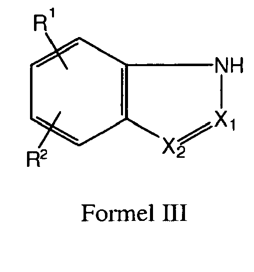 wobei X₁ ausgesucht ist aus der Gruppe bestehend aus N, O, S und CR³, X₂ ausgesucht ist aus der Gruppe bestehend aus N, O, S und CR⁴ und R¹, R², R³ und R⁴ unabhängig voneinander ausgesucht sind aus der Gruppe bestehend aus H, C₁-C₆-Alkylgruppen, -OH, -SH, -NH₂, -F, - Cl, -Br, -I, Alkyl-O-, Alkyl-S-, Alkylamino- und Dialkylamino-, wobei diese Alkylgruppen (d.h. die Alkylgruppen in den Alkyl-O-, Alkyl-S-, Alkylamino- und Dialkylaminogruppen) C₁-C₅-Alkylgruppen, vorzugsweise C₁-C₃-Alkylgruppen, sind. Vorzugsweise stellt entweder X₁ oder X₂ N dar. Weiterhin kann wenn X₁ CR⁴ ist X₂ CR⁵ sein. Vorzugsweise stellen R¹, R², R³ und R⁴ H dar.
   Besonders bevorzugte Verbindungen sind Indazol und Benzimidazol.
   Weiterhin bevorzugt als stickstoffhaltige Verbindungen sind die heterocyclischen Verbindungen ausgesucht aus der Gruppe der Nukleobasen. Diese umfassen insbesondere die Verbindungen Adenin, Cytosin, Guanin, Inosin, Hypoxanthin, Thymin, Uracil und Xanthin oder solche Verbindungen, die als Analoge oder Derivate in Nukleinsäure eingebaut werden können, wobei die Struktur dieser Verbindungen auch im Ring Unterschiede aufweisen kann. Besonders bevorzugt sind hier die Verbindungen Adenin, Cytosin, Guanin und Thymin.
   In der vorliegenden Erfindung zeigen die Verbindungen Imidazol und 2,3-Dimethylpyrazin, Pyrimidin, Guanin und Guanosin besonders gute Wirksamkeit.
   Weiterhin umfasst als stickstoffhaltige heterocyclische Verbindungen sind Homo- oder Heteropolymere, die diese stickstoffhaltigen Verbindungen enthalten.
d) Aminen vom Typ R¹R²NR³, wobei die Reste R¹, R² und R³ unabhängig voneinander ausgewählt sind aus der Gruppe bestehend aus H und C₁-C₃-Alkylgruppen, wobei gilt, dass R¹ R² und R³ nicht gleichzeitig H sind. Insbesondere bevorzugt ist die Verwendung von Methylamin, Ethylamin, n-Propylamin, Dimethylamin, Diethylamin, Di(n-propyl)amin, Di(isopropyl)amin, Trimethylamin, Triethylamin, Tri(n-propyl)amin, und Tri(isopropyl)amin.
e) Carbonsäureamiden, welche die Struktur X-C(=O)NH₂ aufweisen. Dabei ist X ausgesucht ist aus der Gruppe bestehend aus: -NH₂, C₁-C₅-Alkyl, C₂-C₅-Alkenyl, C₂-C₅-Alkinyl oder Aryl, vorzugsweise Phenyl oder ein aminosubstituiertes Aryl, H₂NC(=O)-Y-, wobei Y eine Alkylengruppe von Typ -(CH₂)ₙ- sein kann und wobei n eine ganze Zahl aus dem Bereich 0 bis 10, vorzugsweise 0 bis 5 ist, oder Y eine C₁C₁₀-Alkenylengruppe, vorzugsweise eine C₁-C₆-Alkenylengruppe, oder eine Arylgruppe ist. Wenn Y eine C₁-C₁₀-Alkenylengruppe ist, kann diese Gruppe eine oder mehrere Olefinbindungen umfassen, wobei die Olefinbindungen in der Kohlenstoffkette isoliert oder konjugiert vorliegen können. Wenn Y ein Arylrest ist, kommen insbesondere Phenyl- oder Biphenylgruppen in Frage. Vorzugsweise stellt X -NH₂ oder 2-Aminophenyl dar.
f) Anorganischen Ammoniumsalzen, die ausgesucht sind aus der Gruppe bestehend aus Ammoniumsulfat, Ammoniumcarbonat und Ammoniumhydrogenphoshat;
g) Ammoniumgruppen enthaltenden inneren Salzverbindungen, die ausgesucht sind aus Betain, Ectoin und Trimethylaminoxid;
h) Nukleinsäure bindenden Antibiotika, die ausgesucht sind aus der Gruppe bestehend aus Distamycinen, insbesondere Distamycin D, Mitomycinen, Norfloxacin, Streptozocin, Duocarmycinen, Streptozocin, Actinomycinen, und Aminoglycisiden; und
i) Verbindungen, die in der kleinen Grube der DNA binden und ausgesucht sind aus der Gruppe bestehen aus Thiazotropsin, Tri-Imidazol und Chromomycinen.
j) stickstoffhaltige Verbindung ausgewählt aus den unter a-i beschriebenen Gruppen mit einer zusätzliche Derivatisierung. Die Derivatisierung kann z.B durch eine Verknüpfung der stickstoffhaltigen Verbindungen mit anorganischen oder organischen Resten wie Zuckern, Phosphaten, Alkoholen, Sulfaten, Glutathion, Lipiden etc. durchgeführt sein. Besonders bevorzugt sind hier die Verbindungen Guanosin, Adenosin, Cytosin, und Thymindin oder auch Antibiotika.

Die oben beschriebenen bevorzugten stickstoffhaltigen Verbindungen können in allen der nachfolgend noch erläuterten, weiteren erfindungsgemäßen Aspekte der Erfindung verwendet werden. Allgemein können die stickstoffhaltigen Verbindungen, die in der vorliegenden Erfindung Verwendung finden, in freier Form oder in Form von geeigneten Salzen vorliegen. Ob eine Verbindung als freie Verbindung oder als Salz eingesetzt wird kann davon abhängig gemacht werden, ob die Zusammensetzung in flüssiger oder fester Form vorliegen soll.

Unter C₁-C₆-Alkyl-Gruppen werden im Rahmen der vorliegenden Erfindung insbesondere die folgenden Gruppen verstanden: Methyl, Ethyl, Propyl, Isopropyl, Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und/oder 1-Ethyl-2-methyl-propyl.

Unter C₂-C₅-Alkenyl-Gruppen werden im Rahmen der vorliegenden Erfindung insbesondere die Gruppen Ethenyl (Vinyl), 2-Propenyl (Allyl), 2-Butenyl, 3-Butenyl, 1-Methyl-2-propenyl, 2-Methyl-2-propenyl, 2-Pentyl, 3-Pentyl, 4-Pentyl, 1-Methyl-2-butenyl, 2-Methyl-3-butenyl, 3-Methyl-3-butenyl, 1,1-Dimethyl-2-propenyl, 1,2-Dimethyl-2-propenyl, und 1-Ethyl-2-propenyl verstanden.
Unter C₂-C₅-Alkinyl-Gruppen werden im Rahmen der vorliegenden Erfindung insbesondere Ethinyl, 2-Propinyl (Propargyl), 2-Butinyl, 3-Butinyl, 2-Pentinyl, 3-Pentinyl, 4-Pentinyl, 3- und Methyl-2-butinyl verstanden.

Vorzugsweise wird die Wahl der stickstoffhaltigen Verbindung, die zur Lyse verwendet wird, auf das in dem Lysepuffer verwendete Lösemittel abgestimmt. Dabei ist es bevorzugt, dass die stickstoffhaltige Verbindung in einem Maße in dem Lösemittel löslich ist, dass Konzentrationen der Verbindung in dem Lösemittel in dem jeweils gewünschten Bereich möglich sind.

In einem weiteren Aspekt betrifft die vorliegenden Erfindung ein Analyseverfahren zum Nachweis zumindest einer Spezies Nukleinsäure und/oder zumindest einer Spezies Protein in einer Probe, umfassend die Schritte:
A) Erzeugung eines Lysats gemäß dem erfindungsgemäßen Lyseverfahren, und
B) Verwendung des Lysats in einer zum Nachweis der zumindest einen Spezies Nukleinsäure und/oder der einen Spezies Protein geeigneten Reaktion oder Reaktionssequenz.

Durch die verbesserte Lyse wird die Nachweisgenauigkeit des Analyseverfahrens verbessert, da im Vergleich mit herkömmlichen Analyseverfahren höhere Konzentrationen der jeweiligen Nukleinsäure und/oder Proteins in der Probe erreicht werden.

Vorzugsweise wird das Lysat direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen durchgeführt werden, oder dass weitere Schritte zur Entfernung von Stoffen, die den Abbau von Nukleinsäuren und/oder Proteinen bewirken, aus der Probenzubereitung durchgeführt werden müssen, bzw. dass solche Stoffe inaktiviert werden müssen. Vorzugsweise wird dabei das Lysat ohne jeden weiteren Aufarbeitungsschritt direkt zur Analyse bzw. zur Herstellung einer zur Analyse geeigneten Reaktionslösung verwendet.

In anderen Fällen kann die Lyse unterstützt werden durch die oben genannten Verfahren zur physikalischen, chemischen oder enzymatischen Lyse. Insbesondere haben sich Lyse-Verfahren mit physikalischer Unterstützung durch Erhitzen oder Homogenisierung des Lysats, mit chemischer Unterstützung durch Verwendung von Detergentien oder enzymatische Unterstützung durch Verwendung von Proteasen wie z.B. Protease K (wenn nicht Protein nachgewiesen werden soll), Lysozym oder auch Nucleasen (z.B. DNase; in diesem Fall kann DNA nicht nachgewiesen werden) als tauglich erwiesen.

Dabei kann die zum Nachweis der zumindest einen Nukleinsäure geeignete Reaktion oder Reaktionssequenz ausgesucht sein z.B. aus der Gruppe der Nukleinsäure-Bindereaktionen, insbesondere der Nukleinsäure-Hybridisierungen, insbesondere Northern-, Southern-Blotting oder auch Hybridisierung von Oligonukleotiden und Sonden, insbesondere DNA-Sonden, der Gruppe der enzymatischen Modifikationen bzw. Polymerisationen von Nukleinsäuren, insbesondere Sequenzierungsreaktionen, in-vitro Transkription, Restriktionsendonukleasespaltungen, der Gruppe der Amplifikationsreaktionen, insbesondere PCR (Polymerase-Kettenreaktion), Real-Time-PCR (als Sonden basierendes Verfahren wie auch als Verfahren, dass abhängig ist von einem sequenzunspezifisch bindenden Detektormolekül wie. z.B. SybrGreen), RT-PCR (Reverse Transkription Polymerase-Kettenreaktion), Real-time-RT-PCR (als Sonden basierendes Verfahren wie auch als Verfahren, dass abhängig ist von einem sequenzunspezifisch bindenden Detektormolekül wie. z.B. SybrGreen), Multiplex PCR, Multiplex RT-PCR, die sondenbasierenden Verfahren der Real-Time Multiplex PCR und Real-Time Multiplex RT-PCR, NASBA (Nucleic Acid Sequence Based Amplification), 3SR (Sequence Sustained Self Replication), 2SR, TMA (Transcription Mediated Amplification), MDA (Multiple Displacement Amplification) Rolling Circle Amplification, Whole-Transcriptome-Amplification, Whole-Genome-Amplification und Rolling Transcription Amplification oder Loop-mediated isothermal amplification (LAMP).
Die zum Nachweis des zumindest einen Proteins geeignete Reaktion oder Reaktionssequenz kann ausgesucht sein aus der Gruppe der Protein-Bindereaktionen, insbesondere Protein-Erkennung, insbesondere durch andere Proteine, Reaktionen beruhend auf der enzymatischen Aktivität des Proteins, Antikörper, Aptamere, Liganden, Nukleinsäuren, insbesondere Western-Blotting, oder andere Substanzen wie Glutathion und NAD, oder ausgesucht ist aus der Gruppe der Proteinmodifikation oder -prozessierung, insbesondere (De)Phosphorylierung, (De)Glycosylierung, und Spaltung durch Proteasen.
Alle der oben genannten Verfahren können entweder in Lösung, Suspension oder Festphase durchgeführt werden.

In einem weiteren Aspekt betrifft die vorliegende Erfindung ein Verfahren zur Stabilisierung von Nukleinsäuren und/oder Proteinen, wobei dieses Verfahren die folgenden Schritte umfasst:
A) Bereitstellen einer Probe, die zumindest eine Spezies einer Nukleinsäure und/oder eines Proteins umfasst,
B) In-Kontakt-Bringen der Probe mit einer flüssigen oder festen Zusammensetzung, um eine flüssige Probenzubereitung zu erzeugen, wobei die Zusammensetzung zumindest eine stickstoffhaltige Verbindung enthält, die ausgesucht ist aus der Gruppe bestehend aus
   a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, j) stickstoffhaltige Verbindungen ausgewählt aus den unter a-i beschriebenen Gruppen mit einer zusätzliche Derivatisierung sowie Mischungen aus zwei oder mehreren dieser Verbindungen.

In einer weiteren Ausführungsform dieses Verfahrens wird die Stabilisierung von Nukleinsäuren durch eine oder mehrere der beanspruchten Substanzen durchgeführt vor während oder nach einer enzymatischen, physikalischen oder chemischen Lyse, bevorzugt unter Verwendung von Detergenzien und Protease wie. z.B Proteinase K. So werden z.B. Detergenzien in einer Konzentration von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.

In einer weiteren Ausführungsform dieses Verfahrens wird die Stabilisierung von RNA durch eine oder mehrere der beanspruchten Substanzen durchgeführt nachfolgend nach einem enzymatischen Abbau durch eine Endonuklease wie z.B. DNase. So kann die DNase I in einer Konzentration von 0,003 U/µl bis 0,5 U /µl in der stablisierten Probe enthalten sein.

In einer weiteren Ausführungsform dieses Verfahrens wird die Stabilisierung von Protein durch eine oder mehrere der beanspruchten Substanzen durchgeführt nachfolgend nach einem enzymatischen Abbau durch von Nukleinsäuren durch Nukleasen wie z.B. RNase, DNasen, Benzonasen etc.

In einer weiteren Ausführungsform dieses Verfahrens wird die Stabilisierung von Biomolekülen in der Probe z.B. von Nukleinsäure oder Protein durchgeführt durch eine oder mehrere der beanspruchten Substanzen ausgewählt aus z.B Arginin, Imidazol, Prolin, Lysin, Spermin, Spermidin, Glutaminsäure, Indazol, Thymin, Thymidin, Guanin, Guanosin, Adenin, Histidin, Tryptophan, Ammoniumsulfat oder durch eine Mischung dieser.

In einer weiteren Ausführungsform dieses Verfahrens wird die zumindest eine Spezies Nukleinsäure oder Protein vor, während oder nach der Zugabe der stickstoffhaltigen Verbindung auf einem Trägermaterial immobilisiert. Als Trägermaterialien können hier die bereits oben im Zusammenhang mit der erfindungsgemäßen Lyse erwähnten Materialien verwendet werden.

In einer alternativen Ausführungsform des erfindungsgemäßen Stabilisierungsverfahrens wird die Probenzubereitung derart durchgeführt, dass die zumindest eine Spezies der Nukleinsäure und/oder des Proteins in der flüssigen Probenzubereitung gelöst und/oder suspendiert wird. Dies kann insbesonders durch die Zugabe geeigneter Mengen der stickstoffhaltigen Verbindung zu der Probe bewirkt werden. Wird durch die Zugabe der Zusammensetzung eine Lyse einer biologischen Probe bewirkt, so kann dementsprechend die Lyse derart durchgeführt werden, dass die zumindest eine Spezies der Nukleinsäure und/oder des Proteins in der flüssigen Probenzubereitung gelöst und/oder suspendiert wird.

Unter einer Probe wird hier neben Organismen, die zum Zwecke des Aufschlusses einer Lyse unterzogen werden, jedes Stoffgemisch verstanden, das zumindest eine Spezies einer Nukleinsäure und/oder eines Proteins aufweist. Dabei kann es sich um ein Stoffgemisch handeln oder auch nur um die reine Nukleinsäure bzw. das Protein. Solche Stoffgemische können z.B. durch Nukleinsäure- oder Proteinsäure-Präparationen erhalten werden. Auch können als Proben Trägermaterialien verwendet werden, auf denen die zumindest eine Spezies Nukleinsäure und/oder Protein immobilisiert wurde. Die Probe kann dabei als Feststoff oder Lösung vorliegen bzw. es kann sich um reine Organismen bzw. um Suspensionen von Organismen in einem flüssigen Medium, wie z.B. Zellkulturen handeln. In Abhängigkeit von der Art der Probe kann die Zusammensetzung der Probe als Flüssigkeit, z.B. als Pufferlösung oder als Feststoff zugegeben werden. Unter einer flüssigen Probenzubereitung fallen hier Probenzubereitungen, die zumindest eine flüssige Phase enthalten. Die Probenzubereitung kann z.B. vollständig gelöst sein oder auch suspendierte Bestandteile und feste Bestandteile umfassen. Beispielsweise können Zellbestandteile suspendiert oder ungelöst in der Probenzubereitung vorliegen. Auch kann die Probenzubereitung ein Trägermaterial zur Immobilisierung von Nukleinsäuren und/oder Proteinen umfassen.
Durch die Zugabe der stickstoffhaltigen Verbindung kann die zumindest eine Spezies Nukleinsäure bzw. Protein in der Probenzubereitung stabilisiert werden, so dass eine unmittelbare Abtrennung dieser Spezies von Bestandteilen in der Probenzubereitung, die ohne die Stabilisierung durch die stickstoffhaltige Verbindung diese Spezies abbauen oder zersetzen würden, nicht erforderlich ist bzw. gegebenenfalls zu einem späteren Zeitpunkt als üblich erfolgen kann. Besonders bevorzugt ist es jedoch, wenn auf eine nachfolgende Aufreinigung der Probenzubereitung vollständig verzichtet werden kann und die Probenzubereitung direkt in einem Analyse- bzw. Nachweisverfahren eingesetzt werden kann. Dadurch wird zum einen der Nachweis der Spezies Nukleinsäure oder Protein verbessert, da diese Spezies in der Analyselösung stabilisiert vorliegt und seine Konzentration in dieser Analyselösung nicht oder in nur geringerem Maße als in einem Vergleichssystem ohne stickstoffhaltige Verbindung abnimmt und somit über den Zeitraum der Analyse mehr Substanz zur Verfügung steht. Zum anderen kann so leichter auf zeitraubende und kostspielige sowie kontaminationsanfällige Aufreinigungsschritte verzichtet werden. Weiterhin wurde festgestellt, dass in den Proben, in denen die Spezies Nukleinsäure und/oder Protein neben zellulären Substanzen, wie sie insbesondere in einem Zelllysat vorliegen, gegebenenfalls zusätzlich stabilisiert werden können.

In einer bevorzugten Ausführungsform des erfindungsgemäßen Stabilisierungsverfahrens ist die Probe eine biologische Probe, die vor dem In-Kontakt-Bringen mit der Zusammensetzung zur Erzeugung der flüssigen Probenzubereitung einer Lyse und gegebenenfalls weiteren Aufreinigungsschritten unterzogen wird. Die Lyse kann hier mit herkömmlichen Lyseverfahren wie oben beschrieben erfolgen und bei den weiteren Aufreinigungsschritten kann es sich beispielsweise um ein Präparationsverfahren oder Immobilisierungsverfahren einer Nukleinsäure- oder Protein-Präparation handeln.

In einer weiteren bevorzugten Ausführungsform des erfindungsgemäßen Verfahrens ist die Probe eine biologische Probe, in der die zumindest eine Spezies einer Nukleinsäure und/oder zumindest eines Proteins in einer Hülle, insbesondere einer biologischen Hülle, enthalten ist und wobei die Probe mit der flüssigen oder festen Zusammensetzung in Kontakt gebracht wird, um ein Lysat zu erzeugen. Die Probe kann also Organismen enthalten. Das Lysat wird hier vorzugsweise gemäß dem oben beschriebenen erfindungsgemäßen Verfahren zur Lyse einer biologischen Probe erzeugt.

In einer weiteren Ausführungsform dieses Verfahrens enthält die Probe zumindest zwei Spezies aus der Gruppe bestehend aus Nukleinsäuren und Proteinen. Die Probenzubereitung wird derart erzeugt bzw. die Lyse wird derart durchgeführt, dass die zwei oder mehrere bestimmte Spezies Nukleinsäure und/oder Proteine in der erzeugten Probenzubereitung bzw. dem erzeugten Lysat gelöst und/oder suspendiert bzw. auf einem Trägermaterial immobilisiert werden. Beispielsweise können in dem Verfahren DNA und RNA, insbesondere gDNA und RNA in der Probenzubereitung bzw. dem Lysat gelöst oder suspendiert werden. Alternativ können selektiv Proteine in Lösung gehalten werden. Wie oben beschrieben sind bei der Wahl der bei der Erzeugung der Probenzubereitung bzw. der Lyse anzuwendenden Bedingungen die Art der Probe sowie die verschiedenen in der zur Erzeugung der Probenzubereitung verwendeten Zusammensetzung enthaltenen Komponenten bzw. in der zur Lyse verwendeten Zusammensetzung ggf. enthaltenen Lysereagenzien zu berücksichtigen, und insbesondere die Menge der eingesetzten stickstoffhaltigen Verbindung.

In einer weiteren bevorzugten Ausführungsform des erfindungsgemäßen Stabilisierungsverfahrens enthält die Probe zumindest zwei Spezies aus der Gruppe bestehend aus Nukleinsäuren und/oder Proteinen und die Probenzubereitung bzw. die Lyse wird derart durchgeführt, dass die in der Probe enthaltenen Spezies Nukleinsäuren und/oder Proteine mehrheitlich in der erzeugten Probenzubereitung bzw. dem Lysat im Wesentlichen gelöst und/oder suspendiert oder immobilisiert werden. Vorzugsweise werden im Wesentlichen alle Spezies gelöst und/oder suspendiert bzw. immobilisiert. Auch hier können entweder alle in der Probe enthaltenen Spezies Nukleinsäuren oder alle in der Probe enthaltenen Spezies Proteine gelöst und/oder suspendiert werden. Dies bedeutet, dass während der Probenzubereitung bzw. Lyse, im Wesentlichen keine Präzipitation, wie sie z.B. zur Entfernung von Nukleinsäuren oder Proteinen aus Lysaten eingesetzt werden, erfolgt. Enthält die Probe mehrere Spezies Nukleinsäuren und/oder Proteine und sollen zwei, mehrerer oder alle dieser Spezies nach dem In-Konakt-Bringen mit der stickstoffhaltigen Verbindung in der Probenzubereitung bzw. dem Lysat gelöst und/oder suspendiert bleiben, ist es besonders bevorzugt, dass sich die relative Konzentration dieser verschiedenen Spezies Nukleinsäuren und/oder Proteine zueinander durch die Zugabe der die stickstoffhaltige Verbindung enthaltende Zusammensetzung bzw. durch die Lyse nicht verändert. D.h., dass die relative Konzentration dieser Spezies in der Probenzubereitung bzw. dem Lysat gegenüber der relativen Konzentration dieser Spezies in der unbehandelten Probe im Wesentlichen unverändert bleibt. Erneut gilt, dass, wie oben bereits ausgeführt, die Art der Probe sowie die verschiedenen ggf. verwendeten Lysereagenzien und die Art der verwendeten stickstoffhaltigen Verbindung bei der Erzeugung der Probenzubereitung bzw. der Durchführung der Lyse zu berücksichtigen sind und gegebenenfalls geeignete Routinetests durchzuführen sind, um geeignete Bedingungen für eine vorgegebene Probenart zu ermitteln.

In einer weiteren bevorzugten Ausführungsform des erfindungsgemäßen Stabilisierungsverfahrens werden in der Probenzubereitung bzw. dem Lysat DNA und/oder RNA gelöst und/oder suspendiert bzw. immobilisiert. Zusätzlich oder alternativ dazu kann zumindest eine Spezies Protein in der Probenzubereitung bzw. dem Lysat gelöst und/oder suspendiert bzw. immobilisiert werden.

Wie bereits zuvor im Zusammenhang mit dem erfindungsgemäßen Verfahren zur Lyse einer biologischen Probe beschrieben, kann die zur Lyse verwendete Zusammensetzung als weitere Lysereagenzien zumindest ein Reagenz aus der Gruppe der Komplexbildner, Detergentien, Substanzen zur Volumeneinengung, und/oder Lösemittel enthalten. Auch kann die Lyse unter mechanischer Einwirkung und/oder enzymatisch erfolgen, oder vor der Lyse die Probe mit einem hypotonen Waschpuffer gewaschen werden. Die Lyse kann dabei unterstützt werden durch die oben genannten Verfahren zur physikalischen, chemischen oder enzymatischen Lyse. Insbesondere haben sich Lyse-Verfahren mit physikalischer Unterstützung durch Erhitzen oder Homogenisierung des Lysats, mit chemischer Unterstützung durch Verwendung von Detergentien oder enzymatische Unterstützung durch Verwendung von Proteasen wie z.B. Proteinase K (wenn nicht Protein nachgewiesen werden soll), Lysozym oder auch Nucleasen (z.B. DNase; in diesem Fall DNA nicht nachgewiesen werden) als tauglich erwiesen. Hier gelten in gleicher Weise die oben im Zusammenhang mit dem erfindungsgemäßen Lyseverfahren gemachten Ausführungen.

In dem erfindungsgemäßen Stabilisierungsverfahren ist die Menge der stickstoffhaltigen Verbindung, die der Probe zugegeben wird, vorzugsweise so gewählt, dass die die Konzentration der stickstoffhaltigen Verbindung in der erzeugten Probenzubereitung bzw. dem Lysat zwischen 0,001 mM bis 1 M, vorzugsweise zwischen 0,001 bis 100mM, insbesondere vorzugweise zwischen 0,001 bis 30 mM und speziell 0,001 bis 19 mM oder 0,001 bis 15 mM beträgt. Werden Polyamine (a) verwendet, so beträgt die Konzentration vorzugsweise zwischen 0,001 mM und 15 mM, vorzugsweise zwischen 0,001 bis 1 mM. Überraschenderweise wird bereits bei diesen sehr geringen Konzentrationen an Polyaminen, insbesondere Spermidin, eine wesentliche Stabilisierung z.B. von RNA festgestellt. Bei Verwendung von Aminosäuren (b) kann die Konzentration vorzugsweise im Bereich von 0,001 bis 20 mM, insbesondere im Bereich von 1 bis 15 mM liegen. Bei Verwendung von stickstoffhaltigen Heterocyclen (c) kann die Konzentration vorzugsweise zwischen 0,001 bis 20 mM, insbesondere vorzugsweise zwischen 0,001 bis 15 mM liegen. Bei Verwendung von Carbonsäureamiden (e) kann die Konzentration vorzugsweise zwischen 0,001 bis 15 mM liegen. Werden anorganische Ammoniumverbindungen verwendet, kann die Konzentration vorzugsweise 0,001 bis 100 mM betragen, insbesondere vorzugsweise von 0.001 bis 15 mM. Bei Verwendung von Ammoniumgruppen enthaltenden inneren Salzverbindungen beträgt die Konzentration vorzugsweise 0.001 bis 300 mM, insbesondere bevorzugt 0.001 bis 200 mM.

Bezüglich der Art der stickstoffhaltigen Verbindungen, die vorzugsweise in dem erfindungsgemäßen Stabilisierungsverfahren eingesetzt werden können, gelten in gleicher Weise die oben mit Bezug auf das erfindungsgemäße Lyseverfahren gemachten Ausführungen.

In einem weiteren Aspekt betrifft die vorliegende Erfindung ein Analyseverfahren zum Nachweis zumindest einer Spezies von Nukleinsäuren und/oder Proteinen in einer Probe, das Verfahren umfassend die Schritte:
a) Bereitstellen einer Probenzubereitung oder eines Lysats, das zumindest eine Spezies einer Nukleinsäure und/oder zumindest eines Proteins enthält, wobei die zumindest eine Spezies Nukleinsäure und/oder Protein mit dem erfindungsgemäßen Stabilisierungsverfahren stabilisiert wurde, und
b) Verwendung der Probenzubereitung in einer zum Nachweis der zumindest einen Nukleinsäure oder des zumindest einen Proteins geeigneten Reaktion oder Reaktionssequenz. Vorzugsweise wird die Probenzubereitung direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkender Stoffe aus der Probenzubereitung durchgeführt werden.

Durch dieses Verfahren kann aufgrund der Stabilisierung der nachzuweisenden Spezies Nukleinsäure und/oder der Spezies Protein ein verbesserter Nachweis erreicht werden, wobei vorzugsweise auf eine der Nachweisreaktion vorgelagerte Probenaufbereitung verzichtet werden kann. Da die nachzuweisende Spezies Nukleinsäure bzw. Protein stabilisiert ist, kann der Nachweis mit geringeren Probenmengen geführt werden.

Bezüglich der Art der zum Nachweis der entsprechenden Spezies Nukleinsäure und/oder Protein verwendeten Reaktion oder Reaktionssequenz gelten die oben bereits mit Bezug auf die im Zusammenhang mit der erfindungsgemäßen Lyse gemachten Ausführungen.

Neben der Stabilisierung kann die Verwendung der stickstoffhaltigen Verbindung bei der Erzeugung der Probenzubereitung bzw. des Lysats auch zu einer Maskierung bestimmter weiterer in der Probe enthaltenen Spezies Nukleinsäure führen. Wird die Probe einer Lyse unterzogen, kann auch eine Verbesserung der Lyse, insbesondere eine erhöhte Konzentration der gewünschten Spezies Nukleinsäuren und/oder Proteine in dem Lysat, erreicht werden. Auch kann neben der Stabilisierung ggf. eine Maskierung bestimmter Spezies Nukleinsäuren in der Probe erreicht werden. Auch können ggf. durch die Verwendung der stickstoffhaltigen Verbindung inhibitorische Effekte in der erzeugten Probenzubereitung bzw. dem Lysat vermindert werden.

In einem weiteren Aspekt betrifft die vorliegende Erfindung ein Verfahren zur Verminderung inhibitorischer Effekte in einer Probe, die zumindest eine Spezies Nukleinsäure und/oder eine Spezies Protein und zumindest eine inhibierende Substanz enthält. Dieses Verfahren umfasst die Schritte:
A) Bereitstellen einer Probe, die zumindest eine Spezies einer Nukleinsäure und/oder eines Proteins sowie zumindest eine inhibierende Substanz umfasst,
B) In-Kontakt-Bringen der Probe mit einer flüssigen oder festen Zusammensetzung, um eine flüssige Probenzubereitung zu erzeugen, wobei die Zusammensetzung zumindest eine stickstoffhaltige Verbindung enthält, die ausgesucht ist aus der Gruppe bestehend aus a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen Verbindungen umfassen, d) Aminen vom Typ R1R2NR3, wobei R1, R2 und R3 unabhängig voneinander ausgewählt sind aus der Gruppe bestehend aus H, C1-C5-Alkylgruppen und Arylgruppen, wobei R1, R2 und R3 nicht gleichzeitig H sind, e) Carbonsäureamiden, f) anorganischen Ammoniumsalzen, g) Ammoniumgruppen enthaltenden inneren Salzverbindungen, h) Nukleinsäure bindenden Antibiotika, i) Verbindungen, die in der kleinen Grube der DNA binden, j) stickstoffhaltige Verbindungen ausgewählt aus den unter a-i beschriebenen Gruppen mit einer zusätzliche Derivatisierung, sowie Mischungen aus zwei oder mehreren dieser Verbindungen, wobei die Menge der stickstoffhaltigen Verbindung, die der Probe zugegeben wird so gewählt ist, dass in der flüssigen Probenzubereitung der inhibierende Effekt der inhibierenden Substanz verringert wird.

Unter dem Begriff inhibierender Substanz wird hier jede Substanz verstanden, die in einem nachfolgenden Analyse- bzw. Nachweisverfahren, die mit der Probenzubereitung oder einer durch weitere Aufarbeitungsschritte erhaltenen Probe durchgeführt wird, inhibierend einwirkt, d.h. nachteilig auf die Nachweisreaktion einwirkt. Liegen in der Probe Nukleinsäuren vor, die nachgewiesen werden sollen, können z.B. Proteine solche inhibierende Substanzen darstellen. Sollen Proteine in der Probe nachgewiesen werden, können z.B. Nukleinsäuren solche inhibierenden Substanzen darstellen. Die wechselseitige inhibierende Wirkung von Proteinen und Nukleinsäuren kann beispielsweise auf der Ausbildung von Nukleoprotein-Komplexen beruhen. In bevorzugten Ausführungsformen der vorliegenden Erfindung handelt es sich bei der inhibierenden Substanz um ein Protein, insbesondere ein nukleinsäurebindendes Protein, oder um eine proteinbindende Nukleinsäure.
Unter inhibierendem Effekt wird hier verstanden, dass eine Wechselwirkung zwischen der Spezies Nukleinsäure und/oder der Spezies Protein und der inhibierenden Substanz stattfindet, die sich nachteilig auf eine mit der Probenzubereitung durchgeführte Analyse- bzw. Nachweisreaktion auswirkt. Durch die Gegenwart der inhibierenden Substanz wird das Ergebnis dieser Reaktion gegenüber einer identischen Probe, welche die inhibierende Substanz nicht enthält, somit nachteilig beeinflusst. Eine Verringerung des inhibierenden Effekts kann dadurch festgestellt werden, dass z.B. eine nachfolgende Analyse- oder Nachweisreaktion einer bestimmten Spezies Nukleinsäure oder Protein, die mit einer Probe durchgeführt wird, der eine der erfindungsgemäß verwendeten stickstoffhaltigen Verbindungen zugegeben wurde, im Vergleich mit der gleichen Analyse- oder Nachweisreaktion, die mit einer gleichen Probe, der keine stickstoffhaltige Verbindung zugegeben wurde, verbessert wird.

Unter einer Probe wird hier neben Organismen, die zum Zwecke des Aufschlusses einer Lyse unterzogen werden, jedes Stoffgemisch verstanden, das zumindest eine Spezies einer Nukleinsäure und/oder eines Proteins aufweist. Dabei kann es sich um ein Stoffgemisch handeln oder auch nur um die reine Nukleinsäure bzw. das Protein. Solche Stoffgemische können z.B. durch Nukleinsäure- oder Proteinsäure-Präparationen erhalten werden. Die Probe kann dabei als Feststoff oder Lösung vorliegen bzw. es kann sich um reine Organismen bzw. um Suspensionen von Organismen in einem flüssigen Medium, wie z.B. Zellkulturen. In Abhängigkeit von der Art der Probe kann die Zusammensetzung der Probe als Flüssigkeit, z.B. als Pufferlösung oder als Feststoff zugegeben werden.

In einer bevorzugten Ausführungsform der vorliegenden Erfindung ist die Probe eine biologische Probe, die vor dem In-Kontakt-Bringen mit der Zusammensetzung zur Erzeugung der flüssigen Probenzubereitung einer Lyse und gegebenenfalls weiteren Aufreinigungsschritten unterzogen wird. Die Lyse kann hier mit herkömmlichen Lyseverfahren erfolgen und bei den weiteren Aufreinigungsschritten kann es sich beispielsweise um ein Präparationsverfahren einer Nukleinsäure- oder Protein-Präparation handeln. Die Lyse kann dabei unterstützt werden durch die oben genannten Verfahren zur physikalischen, chemischen oder enzymatischen Lyse. Insbesondere haben sich Lyse-Verfahren mit physikalischer Unterstützung durch Erhitzen oder Homogenisierung des Lysats, mit chemischer Unterstützung durch Verwendung von Detergentien oder enzymatische Unterstützung durch Verwendung von Proteasen wie z.B. Protease K (wenn nicht Protein nachgewiesen werden soll), Lysozym oder auch Nucleasen (z.B. DNase; in diesem Fall DNA nicht nachgewiosen werden) als tauglich erwiesen.

In einer Ausführungsform dieses Verfahrens wird die Stabilisierung von Nukleinsäuren durch eine der ausgewählten Substanzen durchgeführt nachfolgend nach einer enzymatischen, physikalischen oder chemischen Lyse, ganz besonders bevorzugt unter Verwendung von Detergenzien und Protease wie. z.B Proteinase K. So werden z.B. Detergenzien in einer Konzentartion von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.

In einer weiteren Ausführungsform dieses Verfahrens wird die Stabilisierung von RNA durch eine oder mehrere der beanspruchten Substanzen durchgeführt nachfolgend nach einem enzymatischen Abbau durch eine Endonuklease wie z.B. DNase. So kann die DNase I in einer Konzentration von 0,003 U/µl bis 0,5 U /µl in der stablisierten Probe enthalten sein.

In einer weiteren Ausführungsform dieses Verfahrens wird die Stabilisierung von Protein durch eine oder mehrere der beanspruchten Substanzen durchgeführt nachfolgend nach einem enzymatischen Abbau durch von Nukleinsäuren durch Nukleasen wie z.B. RNase, DNasen, Benzonasen etc.

In einer weiteren Ausführungsform dieses Verfahrens wird die Stabilisierung von Biomolekülen in der Probe z.B. von Nukleinsäure oder Protein durchgeführt durch eine oder mehrere der beanspruchten Substanzenausgewählt aus z.B Arginin, Imidazol, Prolin, Lysin, Spermin, Spermidin, Glutaminsäure, Indazol, Thymin, Thymidin, Guanin, Guanosin, Adenin, Histidin, Tryptophan, Ammoniumsulfat oder durch eine Mischung dieser.

In einer weiteren bevorzugten Ausführungsform dieses Aspekts der vorliegenden Erfindung wird die zumindest eine Spezies Nukleinsäure oder Protein vor oder während der Zugabe der stickstoffhaltigen Verbindung auf einem Trägermaterial immobilisiert. Dabei können erneut die bereits im Zusammenhang mit den anderen Aspekten der vorliegenden Erfindung beschriebenen Trägermaterialien verwendet werden.

In einer weiteren Ausfühurngsform dieses erfindungsgemäßen Verfahrens wird die Probenzubereitung derart durchgeführt, dass die zumindest eine Spezies der Nukleinsäure und/oder des Proteins in der flüssigen Probenzubereitung gelöst und/oder suspendiert wird. Dies kann insbesonders durch die Zugabe geeigneter Mengen der stickstoffhaltigen Verbindung zu der Probe bewirkt werden. Wird durch die Zugabe der Zusammensetzung eine Lyse einer biologischen Probe bewirkt, so kann dementsprechend die Lyse derart durchgeführt werden, dass die die zumindest eine Spezies der Nukleinsäure und/oder des Proteins in der flüssigen Probenzubereitung gelöst und/oder suspendiert wird.

In einer weiteren bevorzugten Ausführungsform des erfindungsgemäßen Verfahrens ist die Probe eine biologische Probe, in der die zumindest eine Spezies einer Nukleinsäure und/oder zumindest eines Proteins in einer Hülle, z.B. einer biologischen Hülle, enthalten ist, und wobei die Probe mit der flüssigen oder festen Zusammensetzung in Kontakt gebracht wird, um ein Lysat zu erzeugen. Die Probe kann also Organismen enthalten. Das Lysat wird hier vorzugsweise gemäß dem oben beschriebenen erfindungsgemäßen Verfahren zur Lyse einer biologischen Probe erzeugt.

Wie auch in dem oben bereits beschriebenen, erfindunggemäßen Stabilisierungsverfahren enthält die Probe in einer bevorzugten Ausführungsform des erfindungsgemäßen Verfahrens zur Verminderung inhibierender Effekte zumindest zwei Spezies aus der Gruppe bestehend aus Nukleinsäuren und/oder Proteinen und die Erzeugung der Probe bzw. des Lysats wird so durchgeführt, dass eine Mehrheit der in der Probe enthaltenen Spezies Nukleinsäure und/oder Proteine in der Probenzubereitung bzw. im Lysat gelöst und/oder suspendiert oder immobilisiert werden. In einer Variante des Verfahrens werden im Wesentlichen alle dieser Spezies gelöst und/oder suspendiert oder immobilisiert. Dabei können entweder alle in der Probe enthaltenen Spezies Nukleinsäuren oder alle in der Lösung enthaltenen Spezies Proteine gelöst und/oder suspendiert bzw. immobilisiert werden. Hier gelten in gleicher Weise die oben mit Bezug auf die analoge Ausführungsform des Stabilisierungsverfahrens gemachten Ausführungen.

Dies bedeutet, dass während der Erzeugung der Probenzubereitung bzw. der Lyse, im Wesentlichen keine Präzipitation, wie sie z.B. zur Entfernung von Nukleinsäuren oder Proteinen aus Lysaten eingesetzt werden, erfolgt. Enthält die Probe mehrere Spezies Nukleinsäuren und/oder Proteine und sollen zwei, mehrere oder alle dieser Spezies nach der Lyse in dem Lysat gelöst und/oder suspendiert bleiben, ist es besonders bevorzugt, dass sich die relative Konzentration dieser verschiedenen Spezies Nukleinsäuren und/oder Proteine zueinander durch die Lyse nicht verändert, die relative Konzentration dieser Spezies im Lysat gegenüber der relativen Konzentration dieser Spezies in der Probe im Wesentlichen unverändert bleibt. Erneut gilt, dass, wie oben bereits ausgeführt, die Art der Probe sowie die verschiedenen weiteren ggf. in der zur Erzeugung der Probenzubereitung zugegebenen Zusammensetzung vorliegenden Komponenten bzw. die zur Erzeugung des Lysats ggf. verwendeten Lysereagenzien und die Art der verwendeten stickstoffhaltigen Verbindung bei der Durchführung der Lyse zu berücksichtigen sind. Geeignete Routinetests sind gegebenenfalls durchzuführen, um geeignete Bedingungen für eine vorgegebene Probenart zu ermitteln.

Auch in dem erfindungsgemäßen Verfahren zur Verminderung inhibitorischer Effekte sieht eine bevorzugte Ausführungsform vor, dass DNA und/oder RNA in der Probenzubereitung oder dem Lysat enthalten, insbesondere gelöst und/oder suspendiert wird. Alternativ kann auch zumindest eine Spezies Protein in dem Lysat enthalten, insbesondere gelöst oder suspendiert werden.

Wie bereits zuvor im Zusammenhang mit dem erfindungsgemäßen Verfahren zur Lyse einer biologischen Probe beschrieben, kann die zur Lyse verwendete Zusammensetzung als weitere Lysereagenzien zumindest ein Reagenz aus der Gruppe der Komplexbildner, Detergentien, Substanzen zur Volumeneinengung, und/oder Lösemittel enthalten. Auch kann die Lyse unter mechanischer Einwirkung und/oder enzymatisch erfolgen, oder vor der Lyse die Probe mit einem hydrotonen Waschpuffer gewaschen werden. Hier gelten in gleicher Weise die oben im Zusammenhang mit dem erfindungsgemäßen Lyseverfahren gemachten Ausführungen.

In dem erfindungsgemäßen Stabilisierungsverfahren ist die Menge der stickstoffhaltigen Verbindung, die der Probe zugegeben wird, vorzugsweise so gewählt, dass die die Konzentration der stickstoffhaltigen Verbindung in der erzeugten Probenzubereitung bzw. dem Lysat zwischen 0.001 mM bis 1 M, vorzugsweise zwischen 0,001 bis 100 mM, insbesondere vorzugweise zwischen 0,001 bis 50 mM oder 0,001 bis 30 mM und speziell 0,001 bis 19 mM oder 0,01 bis 15 mM beträgt. Werden Polyamine (a) verwendet, so beträgt die Konzentration vorzugsweise zwischen 0,001 mM und 15 mM, vorzugsweise zwischen 0,001 bis 1 mM. Bei Verwendung von Aminosäuren (b) kann die Konzentration vorzugsweise im Bereich von 0,001 bis 20 mM, insbesondere im Bereich von 1 bis 15 mM liegen. Bei Verwendung von stickstoffhaltigen Heterocyclen (c) kann die Konzentration vorzugsweise zwischen 0,001 bis 50 mM, insbesondere vorzugsweise zwischen 0,001 bis 30 mM liegen. Bei Verwendung von Carbonsäureamiden (e) kann die Konzentration vorzugsweise zwischen 0,001 bis 20 mM liegen. Werden anorganische Ammoniumverbindungen verwendet, kann die Konzentration vorzugsweise 0.001 bis 100 mM betragen, insbesondere vorzugsweise von 0.001 bis 50 mM. Bei Verwendung von Nukleinsäure bindenden Antibiotika kann die Konzentration vorzugsweise 0.001 bis 15 mM, insbesondere bevorzugt 0.001 bis 1 mM betragen.

In einer besonders bevorzugten Ausführungsform des erfindungsgemäßen Verfahrens zur Verminderung inhibitorischer Effekte ist die zumindest eine Nukleinsäurespezies eine DNA-Spezies, insbesondere gDNA und/oder eine RNA-Spezies.

Bezüglich der Art der stickstoffhaltigen Verbindungen, die vorzugsweise in dem erfindungsgemäßen Verfahren zur Verminderung inhibitorischer Effekte eingesetzt werden können, gelten in gleicher Weise die oben mit Bezug auf das erfindungsgemäße Lyseverfahren oder das erfindungsgemäße Stabilisierungsverfahren gemachten Ausführungen.

In einem weiteren Aspekt betrifft die vorliegende Erfindung ein Analyseverfahren zum Nachweis zumindest einer Spezies Nukleinsäure und/oder Protein in einer Probe, wobei das Verfahren die folgenden Schritte umfasst: a) Bereitstellen einer erfindungsgemäßen Probenzubereitung oder eines Lysats, das zumindest eine Spezies einer Nukleinsäure und/oder zumindest eines Proteins enthält, wobei die Probenzubereitung bzw. das Lysat mindestens eine inhibitorische Effekte mit dem erfindungsgemäßen Verfahren zur Verminderung inhibitorischer Effekte vermindert wurden; b) Verwendung der Probenzubereitung in einer zum Nachweis der zumindest einen Nukleinsäure oder des zumindest einen Proteins geeigneten Reaktion oder Reaktionssequenz.

Durch dieses Verfahren kann aufgrund der Verminderung der inhibierenden Effekte in der Probenzubereitung bzw. dem Lysat ein verbesserter Nachweis der zu analysierenden Spezies erreicht werden, wobei vorzugsweise auf eine der Nachweisreaktion vorausgehende Probenaufbereitung zur Abtrennung der inhibierenden Substanz verzichtet werden kann.

Dementsprechend wird in einer bevorzugten Ausführungsform dieses erfindunggemäßen Analyseverfahrens die Probenzubereitung bzw. das Lysat in Schritt b) direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen durchgeführt werden, oder dass weitere Schritte zur Entfernung von Stoffen, die den Abbau von Nukleinsäuren und/oder Proteinen bewirken, aus der Probenzubereitung durchgeführt werden müssen, bzw. dass solche Stoffe inaktiviert werden müssen. Vorzugsweise wird dabei die Probenzubereitung bzw. das Lysat ohne jeden weiteren Aufarbeitungsschritt direkt zur Analyse bzw. zur Herstellung einer zur Analyse geeigneten Reaktionslösung verwendet.

In einer weiteren Ausführungsform kann die Lyse unterstützt werden durch die oben genannten Verfahren zur physikalischen, chemischen oder enzymatischen Lyse. Insbesondere haben sich Lyse-Verfahren mit physikalischer Unterstützung durch Erhitzen oder Homogenisierung des Lysats, mit chemischer Unterstützung durch Verwendung von Detergentien oder enzymatische Unterstützung durch Verwendung von Proteasen wie z.B. Protease K (wenn nicht Protein nachgewiesen werden soll), Lysozym oder auch Nucleasen (z.B. DNase; in diesem Fall DNA nicht nachgewiesen werden) als tauglich erwiesen

In einer Ausführungsform dieses Verfahrens wird die Stabilisierung von Nukleinsäuren durch eine der ausgewählten Substanzen durchgeführt nachfolgend nach einer enzymatischen, physikalischen oder chemischen Lyse, ganz besonders bevorzugt unter Verwendung von Detergenzien und Protease wie. z.B Proteinase K. So werden z.B. Detergenzien in einer Konzentration von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.

In einer weiteren Ausführungsform dieses Verfahrens wird die Verminderung des inhibitorischen Effekts durch eine oder mehrere der beanspruchten Substanzen durchgeführt. Insbesondere kann z.B. ein Nukleinsäure-Abbau durch eine der Susbstanzen verbessert werden. So kann die DNase I in einer Konzentration von 0,003 U/µl bis 0,5 U µl in der Probe DNA abbauen.

In einer weiteren Ausführungsform dieses Verfahrens wird die Stabilisierung von Biomolekülen in der Probe z.B. von Nukleinsäure oder Protein durchgeführt durch eine oder mehrere der beanspruchten Substanzen ausgewählt aus z.B Arginin, Imidazol, Prolin, Lysin, Spermin, Spermidin, Glutaminsäure, Indazol, Thymin, Thymidin, Guanin, Guanosin, Adenin, Histidin, Tryptophan, Ammoniumsulfat oder durch eine Mischung dieser.

Bezüglich der Art der zum Nachweis der entsprechenden Spezies Nukleinsäure und/oder Protein verwendeten Reaktion oder Reaktionssequenz gelten die oben bereits mit Bezug auf die im Zusammenhang mit der erfindungsgemäßen Lyse gemachten Ausführungen.

In einer besonders bevorzugten Ausführungsform dieses erfindungsgemäßen Analyseverfahrens enthält die Probenzubereitung oder das Lysat RNA neben gDNA und die Reaktionssequenz in Schritt b) umfasst optional die Reaktion von Nukleinsäuren mit Enzymen. Solche Reaktionen können insbesondere sein: b1) optionaler enzymatischer Abbau von gDNA und nachfolgend b2) RT-PCR, vorzugsweise Real-time-RT-PCR zum Nachweis von RNA. Alternativ kann das das Lysat RNA neben gDNA enthalten und die Reaktion bzw. Reaktionssequenz umfasst in Schritt b) die Schritte: b1) PCR, vorzugsweise Real-time-PCR zum Nachweis von gDNA oder b2) RT-PCR, vorzugsweise Real-time-RT-PCR zum Nachweis von RNA. Die PCR oder RT-PCR kann dabei wahlweise als Endpunkt-, als Multiplex- und/oder als Real-time-Reaktion ablaufen.

In einer Ausführungsform des erfindungsgemäßen Verfahrens wird mit dem Analyseverfahren ein erstes Umwandlungsprodukt einer ausgewählten Nukleinsäure bereitgestellt, z.B. Erststrang-cDNA aus RNA.

Auch bezüglich des oben erläuterten Verfahrens zur Verminderung inhibitorischer Effekte in einer Probe kann die in der Probenzubereitung bzw. dem Lysat enthaltene stickstoffhaltige Verbindung zu einer zusätzlich verbesserten Lyse, einer Stabilisierung bestimmter Spezies Nukleinsäure und/oder Proteinen sowie zu einer Maskierung bestimmter Nukleinsäuren führen.

In einem weiteren Aspekt betrifft die vorliegende Erfindung ein Verfahren zur selektiven Maskierung zumindest einer Spezies einer Nukleinsäure in einer Probe, die zumindest eine erste Spezies einer Nukleinsäure und eine von der ersten Spezies unterschiedliche zweite Spezies enthält. Dieses Verfahren umfasst die Schritte:
A) Bereitstellen einer Probe, die zumindest zwei unterschiedliche Spezies Nukleinsäure enthält,
B) In-Kontakt-Bringen der Probe mit einer flüssigen oder festen Zusammensetzung, um eine flüssige Probenzubereitung zu erzeugen, wobei die Zusammensetzung zumindest eine stickstoffhaltige Verbindung enthält, die ausgesucht ist aus der Gruppe bestehend aus:
   a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindenden Antibiotika, i) Verbindungen, die in der kleinen Grube der DNA binden, , j) stickstoffhaltige Verbindungen ausgewählt aus den unter a-i beschriebenen Gruppen mit einer zusätzliche Derivatisierung, sowie Mischungen aus zwei oder mehreren dieser Verbindungen, wobei die Erzeugung der Probenzubereitung derart durchgeführt wird, dass sowohl eine erste und eine zweite Spezies Nukleinsäure in der flüssigen Probenzubereitung gelöst und/oder suspendiert wird, und wobei die Menge der stickstoffhaltigen Verbindung so gewählt ist, dass die zweite Spezies Nukleinsäure derart maskiert wird, dass die erste Spezies Nukleinsäure in einem Nachweisverfahren für Nukleinsäuren verbessert nachgewiesen werden kann, verglichen mit dem gleichen Nachweisverfahren, bei dem der Probe die stickstoffhaltige Verbindung nicht zugegeben wurde.
Durch dieses Verfahren wird eine einfache differentielle Analyse der zweiten Spezies Nukleinsäure in der Probe ermöglicht. In diesem Verfahren kann die erste Spezies Nukleinsäure, die in der differentiellen Analyse nicht analysiert werden kann, die aber potentiell die Nachweisreaktion der zweiten Spezies stören oder verfälschen könnte, gezielt in der Probe maskiert werden. Auf eine Abtrennung der ersten oder zweiten Spezies Nukleinsäure aus der Probe kann somit verzichtet werden.
Anderes ausgedrückt kommt es z.B. bei der differentiellen Analyse von DNA und RNA zu einer differentiellen Maskierung von DNA oder RNA, so dass die maskierte Nukleinsäure innerhalb der Analyse nicht oder mit geringerer Effizienz nachgewiesen werden kann bzw. das Ergebnis der Analyse der zweiten Spezies nich nachteilig beeinflusst.

In bevorzugten Ausführungsformen des erfindungsgemäßen Maskierungungsverfahren ist die die Probe eine biologische Probe, die vor dem In-Kontakt-Bringen mit der Zusammensetzung zur Erzeugung der flüssigen Probenzubereitung einer Lyse und gegebenenfalls weiteren Aufreinigungsschritten unterzogen wird. In einer weiteren bevorzugten Ausführungsform ist die Probe eine biologische Probe, in der die Spezies Nukleinsäuren in einer Hülle, vorzugsweise einer biologischen Hülle, enthalten sind und wobei die Probe mit der flüssigen oder festen Zusammensetzung in Kontakt gebracht wird, um ein Lysat zu erzeugen. In einer weiteren bevorzugten Ausführungsform enthält die Probe zumindest zwei Spezies Nukleinsäuren und die Erzeugung der Probenzubereitung bzw. die Lyse wird derart durchgeführt wird, dass mehrere bestimmte Spezies Nukleinsäure und/oder Proteine in dem erzeugten Lysat gelöst oder suspendiert werden. Weiterhin ist es bevorzugt, dass die Probe zumindest zwei Spezies Nukleinsäure enthält und die Erzeugung der Probenzubereitung bzw. der Lyse derart durchgeführt wird, dass im Wesentlichen alle oder zumindest eine Mehrheit in der Probe enthaltenen Spezies Nukleinsäuren in der Probenzubereitung bzw. im Lysat gelöst und/oder suspendiert werden. Weiterhin ist es bevorzugt, dass die Zusammensetzung, welche die stickstoffhaltige Verbindung enthält, als weitere Lysereagenzien zumindest ein Reagenz aus der Gruppe der Komplexbildner, Detergentien, Substanzen zur Volumeneinengung, und/oder Lösemittel enthält. Auch kann die Lyse unter mechanischer Einwirkung und/oder enzymatisch erfolgen, oder vor der Lyse die Probe mit einem hypotonen Waschpuffer gewaschen werden. Bezüglich all dieser bevorzugten Ausführungsformen gelten in gleicher Weise die oben im Zusammenhang mit dem erfindungsgemäßen Lyseverfahren und dem erfindungsgemäßen Stabilisierungsverfahren bzw. dem erfindungsgemäßen Verfahren zur Verminderung inhibitorischer Effekte gemachten Ausführungen.

In weiteren bevorzugten Ausführungsformen des erfindungsgemäßen Verfahrens zur selektiven Maskierung ist
a) die erste Spezies Nukleinsäure eine Spezies DNA und die zweite Spezies Nukleinsäure eine Spezies RNA oder PNA; oder
b) die erste Spezies Nukleinsäure eine Spezies RNA und die zweite Spezies Nukleinsäure eine Spezies DNA oder PNA; oder
c) die erste Spezies Nukleinsäure eine Spezies PNA ist und die zweite Spezies eine Spezies DNA oder RNA,
c) sowohl die erste Spezies Nukleinsäure als auch die zweite Spezies Nukleinsäure DNA-Spezies, oder
d) sowohl die erste Spezies Nukleinsäure als auch die zweite Spezies Nukleinsäure RNA-Spezies, oder
e) sowohl die erste Spezies Nukleinsäure als auch die zweite Spezies Nukleinsäure PNA.

In bevorzugten Ausführungsformen des erfindungsgemäßen Verfahrens zur selektiven Maskierung wird die Menge der stickstoffhaltigen Verbindung in der Erzeugung der Probenzubereitung bzw. des Lysats verwendeten Zusammensetzung so gewählt, dass die Konzentration der stickstoffhaltigen Verbindung in der erzeugten Probenzubereitung bzw. dem Lysat zwischen 0,001 mM bis 1 M, vorzugsweise 0,001 mM bis 100 mM, insbesondere vorzugsweise 0,001 bis 30 mM, speziell 0,001 bis 19 mM beträgt oder 0,001 bis 15 mM beträgt. Bei der Verwendung von Polyaminen (a), insbesondere Spermidin zur selektiven Maskierung von DNA, z.B. gDNA, vorzugsweise gegenüber RNA, beträgt die Konzentration vorzugsweise 0,001 bis 10 mM, vorzugsweise 0,001 bis 1mM. Bei der Verwendung von Aminosäuren (b) zur selektiven Maskierung von DNA, z.B. gDNA, vorzugsweise gegenüber RNA kann die Konzentration vorzugsweise 0,001 bis 50 mM, insbesondere bevorzugt von 0,001 bis 30 mM betragen. Bei der Verwendung von stickstoffhaltigen heterocyclischen Verbindungen zur Maskierung von DNA, z.B. gDNA, vorzugsweise gegenüber RNA kann die Konzentration vorzugsweise von 0,001 bis 30 mM, vorzugsweise von 0,001 bis 20 mM betragen. Bei der Verwendung von anorganischen Ammoniumverbindungen zur Maskierung von DNA, z.B. gDNA, vorzugsweise gegenüber RNA kann die Konzentration vorzugsweise von 0,001 bis 50 mM, vorzugsweise von 0,001 bis 30 mM betragen. Bei der Verwendung von Carbonsäureamiden zur Maskierung von DNA, z.B. gDNA, vorzugsweise gegenüber RNA kann die Konzentration vorzugsweise von 0,001 bis 20 mM, vorzugsweise von 0,001 bis 10 mM betragen. Die zu wählende Konzentration kann durch die Art der Probe beeinflusst werden und ist ggf. mit zu berücksichtigen.

Bezüglich der Art der stickstoffhaltigen Verbindungen, die vorzugsweise in dem erfindungsgemäßen Verfahren zur Verminderung inhibitorischer Effekte eingesetzt werden können, gelten in gleicher Weise die oben mit Bezug auf das erfindungsgemäße Lyseverfahren gemachten Ausführungen.

In einem weiteren Aspekt betrifft die vorliegende Erfindung ein differentielles Analyseverfahren zum Nachweis einer oder mehrerer Spezies Nukleinsäuren in einer Probe, wobei das Verfahren die folgenden Schritte umfasst:
a) Bereitstellen einer Probenzubereitung, die zumindest eine erste Spezies Nukleinsäure und eine zweite Spezies Nukleinsäure enthält und wobei die erste Spezies Nukleinsäure von der zweiten Spezies Nukleinsäure unterschiedlich ist, wobei die erste Spezies Nukleinsäure mit dem erfindungsgemäßen Verfahren zur selektiven Maskierung maskiert wurde und
b) Verwendung der Probenzubereitung bzw. des Lysates in einer zum Nachweis der zweiten Nukleinsäure geeigneten Reaktion oder Reaktionssequenz.

Durch die selektive Maskierung kann das erfindungsgemäße Analyseverfahren ohne eine vorhergehenden Abtrennung der ggf. die Analyse einer zweiten Spezies Nukleinsäure nachteilig beinflussenden ersten Spezies Nukleinsäure durchgeführt werden. Dadurch wird das Analyseverfahren verbessert und vereinfacht, aufwendige vor der Analyse durchzuführende Abtrennungsschritte können weggelassen werden.

In einer bevorzugten Ausführungsform des erfindungsgemäßen Analyseverfahrens wird Schritt b) ohne weitere Verfahrensschritte zur Reduzierung der Anzahl der in der Probenzubereitung enthaltenen Spezies von Nukleinsäuren und/oder ohne weitere Verfahrensschritte zur Entfernung von Stoffen aus der Probenzubereitung, die den Abbau von Nukleinsäuren bewirken, durchgeführt bzw. ohne weitere Verfahrensschritte zur Inaktivierung solcher Stoffe in der Probenzubereitung Vorzugsweise wird die Probenzubereitung bzw. das Lysat direkt ohne weiteren Aufarbeitungsschritt in der Reaktion bzw. Reaktionssequenz zum Nachweis der zweiten Nukleinsäurespezies bzw. zur Herstellung einer zur Analyse geeigneten Reaktionslösung verwendet. Dadurch wird die Probenvorbereitung für den Nachweis einer bestimmten Spezies Nukleinsäure wesentlich vereinfacht, wodurch der Zeitaufwand sowie die Kosten und Fehleranfälligkeit des Analyseverfahrens verringert werden kann.

Bezüglich der Art der zum Nachweis der entsprechenden Spezies Nukleinsäuren verwendeten Reaktion oder Reaktionssequenz gelten die oben bereits mit Bezug auf die im Zusammenhang mit den zuvor beschriebenen Aspekte der vorliegenden Erfindung, z.B. der erfindungsgemäßen Lyse, gemachten Ausführungen.

In einer besonders bevorzugten Ausführungsform des differentiellen Analyseverfahrens ist die in Schritt b) zum Nachweis der zweiten Spezies Nukleinsäure eingesetzte Reaktion bzw. Reaktionssequenz eine PCR oder RT-PCR, vorzugsweise eine Real-time-PCR oder Real-time-RT-PCR. Vorzugsweise ist die in der Probenzubereitung bzw. in dem Lysat die erste Spezies Nukleinsäure gDNA und die zweite Spezies Nukleinsäure RNA, und in Schritt b) die Reaktion bzw. Reaktionssequenz eine RT-PCR, vorzugsweise eine Real-Time-RT-PCR. Dabei werden vorzugsweise die oben mit Bezug auf das erfindungsgemäße Lyseverfahren beschriebenen stickstoffhaltigen Verbindung ausgesucht aus der Gruppe der Polyamine, Heterocyclen, Aminosäuren, Carbonsäureamide und Ammoniumverbindungen verwendet, vorzugsweise Verbindungen ausgesucht aus der Gruppe bestehend aus Spermidin, Ammoniumsulfat, Ammoniumhydrogenphosphat, Glycin, 2,3-Dimethylpyrazin, Benzimidazol, Imidazol, Arginin, Histidin, Harnstoff und Distamycine, insbesondere Distamycin D.

In einer weiteren bevorzugten Ausführungsform des erfindungsgemäßen differentiellen Analyseverfahrens ist in der Probenzubereitung bzw. in dem Lysat die erste Spezies Nukleinsäure RNA und die zweite Spezies Nukleinsäure DANN, vorzugsweise gDNA. In Schritt b) ist die Reaktion bzw. Reaktionssequenz vorzugsweise eine PCR, beispielsweise eine Real-time-PCR. Vorzugsweise werden hierbei oben im Zusammenhang mit dem erfindungsgemäßen Lyseverfahren beschriebenen Verbindung ausgesucht aus der Gruppe der Heterocyclen, Aminosäuren und Ammoniumverbindungen verwendet, die insbesondere vorzugsweise ausgesucht sind aus der Gruppe bestehend aus Prolin, Indazol und Ammoniumsulfat.

Die vorliegende Erfindung wird nun im Folgenden anhand ausgesuchter Beispiele und den dazugehörigen Figuren näher erläutert. Dabei dienen die Beispiele dem Zweck der Verdeutlichung der vorliegenden Erfindung und sind nicht als einschränkend anzusehen.

In einem weiteren Aspekt betrifft die vorliegende Erfindung ein Analyseverfahren zum Nachweis zumindest einer Spezies von RNA in einer Probe, das Verfahren umfassend die Schritte:
a) Bereitstellen einer Probenzubereitung oder eines Lysats, das zumindest eine Spezies einer RNA enthält, wobei die zumindest eine Spezies RNA mit zuminest einer der erfindungsgemäßen Susbstanzen in einer Probenzubereitung behandelt wurde, und
b) Verwendung der Probenzubereitung in einer zum Nachweis der zumindest einen Spezies einer RNA geeigneten Reaktion oder Reaktionssequenz. Vorzugsweise wird die Probenzubereitung direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkender Stoffe aus der Probenzubereitung durchgeführt werden.

In einer Ausführungsform dieses Verfahrens wird die Behandlung der mindestens einen Spezies von RNA durch eine der ausgewählten Substanzen durchgeführt, wenn die RNA in Organismen enthalten ist, besonders bevorzugt in einzelnen Zellen, Zellverbänden, Geweben oder ganze Tieren oder Pflanzen, kultivierten Zellen, oder Absonderungsprodukten oder Sekreten wie z.B. stabilisiertes und nicht stabilisiertes Blut, Plasma, Serum, Gewebsflüssigkeiten, Sperma, Abstrichen, Sputum, Saliva, Tränenflüssigkeit, Urin, Kot, Haaren, Hautschuppen, Bakterien, Viren etc.

In einer Ausführungsform des erfindungsgemäßen Verfahrens können die oben genannten Organismen vor der Behandlung mit den erfindungsgemäßen Substanzen mit einer Waschlösung vorbehandelt werden. Besonders bevorzugt wird hier eine Waschlösung verwendet, die Kontaminationen der RNA-enthaltenden Probe entfernt und/oder auf andere Weise die RNA-enthaltende Probe für die Behandlung mit den erfindungsgemäßen Substanzen vorbereitet.

Durch dieses Verfahren kann aufgrund der Behandlung der nachzuweisenden Spezies der RNA ein verbesserter Nachweis und/oder eine Reaktion erreicht werden.

Bezüglich der Art der zum Nachweis der entsprechenden Spezies RNA verwendeten Reaktion oder Reaktionssequenz gelten die oben bereits mit Bezug auf die im Zusammenhang mit der erfindungsgemäßen Lyse gemachten Ausführungen.

In einer weiteren Ausführungsform des erfindungsgemäßen Verfahrens werden die Organismen mit dem Lysepuffer inkubiert, vorzugsweise zwischen 1 bis 120 Minuten. In einer weiteren Ausführungsform des erfindungsgemäßen Verfahrens wird die Organsismen mit derm lysepuffer bei gegenüber der Raumtemperatur erhöhter Temperatur inkubiert, vorzugsweise bei 30 bis 90°C, besonders bevorzugt bei 50 bis 85°C.

In einer Ausführungsform dieses Verfahrens wird die Behandlung der mindestens einen Spezies von RNA durch eine der ausgewählten Substanzen durchgeführt vor, während oder nachfolgend einer enzymatischen, physikalischen oder chemischen Lyse, ganz besonders bevorzugt unter Verwendung von Detergenzien und Protease wie. z.B Proteinase K. So werden z.B. Detergenzien in einer Konzentartion von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.

In einer weiteren Ausführungsform dieses Verfahrens wird die Behandlung der mindestens einen Spezies RNA durch eine oder mehrere der beanspruchten Substanzen durchgeführt vor, während oder nachfolgend nach einem enzymatischen Abbau von DNA durch eine Endonuklease wie z.B. DNase. So kann die DNase I in einer Konzentration von 0,003 U/µl bis 0,5 U /µl in der stablisierten Probe enthalten sein.

In einer weiteren Ausführungsform des erfindungsgemäßen Verfahrens werden nachfolgend einer enzymatischen Behandlung die Enzyme deaktiviert, beispielsweise durch Erhöhung der Temperatur, beispielsweise auf 60 bis 95°C.

In einer weiteren Ausführungsform dieses Verfahrens wird die Behandlung der mindestens einen Spezies RNA durch eine oder mehrere der beanspruchten Substanzen und einem physikalischen Behandlungsschritt durchgeführt, besonders bevorzugt durch Scherkräfte, Druckunterschiede oder Temperatureinwirkung.

In einer weiteren Ausführungsform dieses Verfahrens wird die Behandlung der mindestens einen Spezies RNA in der Probe durchgeführt durch eine oder mehrere der beanspruchten Substanzen ausgewählt aus z.B Arginin, Imidazol, Prolin, Lysin, Spermin, Spermidin, Glutaminsäure, Indazol, Thymin, Thymidin, Guanin, Guanosin, Adenin, Histidin, Tryptophan, Ammoniumsulfat oder durch eine Mischung dieser.

In einer weiteren Ausführungsform dieses Verfahrens wird die Behandlung der mindestens einen Spezies RNA in einer Lösung durchgeführt, die mindestens eine der beschriebenen Substanzen enthält, wobei der die Lösung einen pH zwischen 7,1 und 14, bevorzugt einen pH zwischen 7,1 und 12 und ganz besonders bevorzugt einen pH zwischen 7,4 und 10 aufweist.

Dabei kann die zum Nachweis der zumindest einen Spezies RNA geeignete Reaktion oder Reaktionssequenz ausgesucht sein z.B. aus der Gruppe der Nukleinsäure-Bindereaktionen, insbesondere der Nukleinsäure-Hybridisierungen, insbesondere Polymerisationen von Nukleinsäuren und insbesondere der Gruppe der Amplifikationsreaktionen. In einer besonderen Ausführungsform dieses Verfahrens ist das Nachweisverfahren der mindestens einen Spezies von RNA eine PCR (Polymerase-Kettenreaktion), Real-Time-PCR (als Sonden basierendes Verfahren wie auch als Verfahren, dass abhängig ist von einem sequenzunspezifisch bindenden Detektormolekül wie. z.B. SybrGreen), RT-PCR (Reverse Transkription Polymerase-Kettenreaktion), Real-time-RT-PCR (als Sonden basierendes Verfahren wie auch als Verfahren, dass abhängig ist von einem sequenzunspezifisch bindenden Detektormolekül wie. z.B. SybrGreen), Multiplex PCR, Multiplex RT-PCR, die sondenbasierenden Verfahren der Real-Time Multiplex PCR und Real-Time Multiplex RT-PCR. In dem beschriebenen Zusammenhang ist der Begriff RT-PCR wie folgt zu verstehen: Die Reaktion der RT-PCR besteht aus einer Reversen Transkription und einer PCR, wobei die beiden Reaktionen unabhängig voneinenader in zwei getrennten Gefäßen oder zusammen in einem Gefäß ablaufen kann. Beide Reaktionen können durch ein oder mehrere Enzyme durchgeführt werden. Neben der RT-PCR können auch anderer Reaktionen mit der mindestens einen Spezies von RNA druchgeführt warden wie z.B. NASBA (Nucleic Acid Sequence Based Amplification), 3SR (Sequence Sustained Self Replication), 2SR, TMA (Transcription Mediated Amplification), MDA (Multiple Displacement Amplification) Rolling Circle Amplification, Whole-Transcriptome-Amplification, Whole-Genome-Amplification und Rolling Transcription Amplification oder Loop-mediated isothermal amplification (LAMP).

In einem weiteren Aspekt betrifft die vorliegende Erfindung ein Analyseverfahren zum Nachweis zumindest einer Spezies von DNA in einer Probe, das Verfahren umfassend die Schritte:
a) Bereitstellen einer Probenzubereitung oder eines Lysats, das zumindest eine Spezies einer DNA enthält, wobei die zumindest eine Spezies DNA mit zuminest einer der erfindungsgemäßen Substanzen in einer Probenzubereitung behandelt wurde, und
b) Verwendung der Probenzubereitung in einer zum Nachweis der zumindest einen Spezies einer DNA geeigneten Reaktion oder Reaktionssequenz. Vorzugsweise wird die Probenzubereitung direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkender Stoffe aus der Probenzubereitung durchgeführt werden.

In einer Ausführungsform dieses Verfahrens wird die Behandlung der mindestens einen Spezies von DNA durch eine der ausgewählten Substanzen durchgeführt, wenn die DNA in Organismen enthalten ist, besonders bevorzugt in einzelnen Zellen, Zellverbänden, Geweben oder ganze Tieren oder Pflanzen, kultivierten Zellen, oder Absonderungsprodukten oder Sekreten wie z.B. stabilisiertes und nicht stabilisiertes Blut, Plasma, Serum, Gewebsflüssigkeiten, Sperma, Abstrichen, Sputum, Saliva, Tränenflüssigkeit, Urin, Kot, Haaren, Hautschuppen, Bakterien, Viren etc.

In einer Ausführungsform des erfindungsgemäßen Verfahrens können die oben genannten Organismen vor der Behandlung mit den erfindungsgemäßen Substanzen mit einer Waschlösung vorbehandelt werden. Ganz besonders bevorzugt wird hier eine Waschlösung verwendet, die Kontaminationen der DNA-enthaltenden Probe entfernt und/oder auf andere Weise die DNA-enthaltende Probe für die Behandlung mit den erfindungsgemäßen Substanzen vorbereitet.

Durch dieses Verfahren kann aufgrund der Behandlung der nachzuweisenden Spezies der DNA ein verbesserter Nachweis und/oder eine Reaktion erreicht werden.

Bezüglich der Art der zum Nachweis der entsprechenden Spezies DNA verwendeten Reaktion oder Reaktionssequenz gelten die oben bereits mit Bezug auf die im Zusammenhang mit der erfindungsgemäßen Lyse gemachten Ausführungen.

In einer weiteren Ausführungsform des erfindungsgemäßen Verfahrens werden die Organismen mit dem Lysepuffer inkubiert, vorzugsweise zwischen 1 bis 120 Minuten. In einer weiteren Ausführungsform des erfindungsgemäßen Verfahrens wird die Organsismen mit derm lysepuffer bei gegenüber der Raumtemperatur erhöhter Temperatur inkubiert, vorzugsweise bei 30 bis 100°C, besonders bevorzugt bei 50 bis 85°C.

In einer Ausführungsform dieses Verfahrens wird die Behandlung der mindestens einen Spezies von DNA durch eine der ausgewählten Substanzen durchgeführt vor, während oder nachfolgend einer enzymatischen, physikalischen oder chemischen Lyse, ganz besonders bevorzugt unter Verwendung von Detergenzien und Protease wie. z.B Proteinase K. So werden z.B. Detergenzien in einer Konzentartion von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.
In einer weiteren Ausführungsform dieses Verfahrens wird die Behandlung der mindestens einen Spezies DNA durch eine der ausgewählten Substanzen durchgeführt nachfolgend nach einem enzymatischen Abbau von RNA durch eine Endonuklease wie z.B. RNase.

In einer weiteren Ausführungsform des erfindungsgemäßen Verfahrens werden nachfolgend einer enzymatischen Behandlung die Enzyme deaktiviert, beispielsweise durch Erhöhung der Temperatur, beispielsweise auf 60 bis 100°C.

In einer weiteren Ausführungsform dieses Verfahrens wird die Behandlung der mindestens einen Spezies DNA in der Probe durchgeführt durch eine Substanz ausgewählt aus z.B Tryptophan, Prolin, Histidin oder Guanosin oder durch eine Mischung dieser.

In einer weiteren Ausführungsform dieses Verfahrens wird die Behandlung der mindestens einen Spezies DNA in einer Lösung durchgeführt, die mindestens eine der beschriebenen Substanzen enthält, wobei die Lösung einen pH zwischen 7,1 und 14, bevorzugt einen pH zwischen 7,1 und 12 und ganz besonders bevorzugt einen pH zwischen 7,4 und 10 aufweist.

In einer weiteren Ausführungsform dieses Verfahrens wird die Behandlung der mindestens einen Spezies DNA durch eine der ausgewählten Substanzen und einem physikalischen Behandlungsschritt durchgeführt, ganz besonders bevorzugt durch Temperatureinwirkung.

Dabei kann die zum Nachweis der zumindest einen Spezies DNA geeignete Reaktion oder Reaktionssequenz ausgesucht sein z.B. aus der Gruppe der Nukleinsäure-Bindereaktionen, insbesondere der Nukleinsäure-Hybridisierungen, insbesondere Polymerisationen von Nukleinsäuren und insbesondere der Gruppe der Amplifikationsreaktionen. In einer besonderen Ausführungsform dieses Verfahrens ist das Nachweisverfahren der mindestens einen Spezies von RNA eine PCR (Polymerase-Kettenreaktion), Real-Time-PCR (als Sonden basierendes Verfahren wie auch als Verfahren, dass abhängig ist von einem sequenzunspezifisch bindenden Detektormolekül wie. z.B. SybrGreen), Multiplex PCR, die sondenbasierenden Verfahren der Real-Time Multiplex PCR. Neben der PCR können auch anderer Reaktionen mit der mindestens einen Spezies von DNA durchgeführt werden wie z.B. MDA (Multiple Displacement Amplification), Rolling Circle Amplification, Whole-Genome-Amplification und Rolling Transcription Amplification oder Loop-mediated isothermal amplification (LAMP).

In einem weiteren Aspekt betrifft die vorliegende Erfindung ein Analyseverfahren zum Nachweis zumindest zweier Spezies Biomoleküle, beispielsweise mindestens einer Spezies einer Nukleinsäure und mindestens einer Spezies eines Proteins in einer Probe, das Verfahren umfassend die Schritte:
a) Bereitstellen einer Probenzubereitung oder eines Lysats, das zumindest zwei Spezies von Biomolekülen, beispielsweise zumindest eine Spezies einer Nukleinsäure und mindestens eine Spezies eines Proteins enthält, wobei die zumindest eine Spezies Nukleinsäure und eine Spezies Protein mit zumindest einer der erfindungsgemäßen Substanzen in einer Probenzubereitung behandelt wurde, und
b) Verwendung der Probenzubereitung in einer oder mehreren zum Nachweis zumindest zweier Spezies von Biomolekülen, beispielsweise zumindest eine Spezies einer Nukleinsäure und einer Spezies Protein, geeigneten Reaktionen oder Reaktionssequenzen. Vorzugsweise wird die Probenzubereitung direkt verwendet, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkender Stoffe aus der Probenzubereitung durchgeführt werden.

In einer Ausführungsform dieses Verfahrens wird die Behandlung von Nukleinsäuren und/oder Proteinen durch eine der ausgewählten Substanzen durchgeführt, wenn die Nukleinsäuren und/oder Protein in Organismen enthalten ist, besonders bevorzugt in einzelnen Zellen, Zellverbänden, Geweben oder ganze Tieren oder Pflanzen, kultivierten Zellen, oder Absonderungsprodukten oder Sekreten wie z.B. stabilisiertes und nicht stabilisiertes Blut, Plasma, Serum, Gewebsflüssigkeiten, Sperma, Abstrichen, Sputum, Saliva, Tränenflüssigkeit, Urin, Kot, Haaren, Hautschuppen, Bakterien, Viren etc.

In einer Ausführungsform des erfindungsgemäßen Verfahrens können die oben genannten Organismen vor der Behandlung mit den erfindungsgemäßen Substanzen mit einer Waschlösung vorbehandelt werden. Ganz besonders bevorzugt wird hier eine Waschlösung verwendet, die Kontaminationen entfernen und/oder auf andere Weise die Probe für die Behandlung mit den erfindungsgemäßen Substanzen vorbereitet.

Durch dieses Verfahren können aufgrund der Behandlung in einer behandelten Probe Nukleinsäuren und/oder Proteine gleichermaßen in einer Reaktion nachgewiesen werden. Durch dieses Verfahren können aufgrund der Behandlung in einer behandelten Probe RNA und DNA oder RNA und Proteine oder DNA und Proteine nachgewiesen werden.
Bezüglich der Art der zum Nachweis der entsprechenden Spezies Nukleinsäure und/oder Protein verwendeten Reaktion oder Reaktionssequenz gelten die oben bereits mit Bezug auf die im Zusammenhang mit der erfindungsgemäßen Lyse gemachten Ausführungen.

In einer Ausführungsform dieses Verfahrens für den Nachweis von RNA und DNA in einer behandelten Probe wird die Behandlung der Nukleinsäuren eine der ausgewählten Substanzen durchgeführt vor, während oder nachfolgend einer enzymatischen, physikalischen oder chemischen Lyse, ganz besonders bevorzugt unter Verwendung von Detergenzien und Protease wie. z.B Proteinase K. So werden z.B. Detergenzien in einer Konzentartion von 0,005 bis 10 % eingesetzt. Proteasen wie z.B. die Proteinase K können in einer Konzentration von 0,5 x 10⁻⁴ mAU/µl bis 50 x 10⁻⁴ mAU/µl in der stablisierten Probe enthalten sein.

In einer Ausführungsform dieses Verfahrens für den Nachweis von RNA und Protein in einer behandelten Probe wird die Behandlung der Nukleinsäuren eine der ausgewählten Substanzen durchgeführt vor, während oder nachfolgend einer enzymatischen, physikalischen oder chemischen Lyse, ganz besonders bevorzugt unter Verwendung von Detergenzien und/oder DNA spezifischen Nuklease. So kann z.B eine DNase I in einer Konzentration von 0,003 U/µl bis 0,5 U /µl in der behandelten Probe enthalten sein.

In einer Ausführungsform dieses Verfahrens für den Nachweis von DNA und Protein in einer behandelten Probe wird die Behandlung der Nukleinsäuren eine der ausgewählten Substanzen durchgeführt vor, während oder nachfolgend einer enzymatischen, physikalischen oder chemischen Lyse, ganz besonders bevorzugt unter Verwendung von Detergenzien und/oder RNA spezifischen Nuklease.

In einer weiteren Ausführungsform des erfindungsgemäßen Verfahrens werden die Organismen mit dem Lysepuffer inkubiert, vorzugsweise zwischen 1 bis 120 Minuten. In einer weiteren Ausführungsform des erfindungsgemäßen Verfahrens wird die Organsismen mit derm lysepuffer bei gegenüber der Raumtemperatur erhöhter Temperatur inkubiert, vorzugsweise bei 30 bis 85°C, besonders bevorzugt bei 50 bis 80°C.

In einer weiteren Ausführungsform dieses Verfahrens wird die Behandlung der mindestens zwei Spezies Biomolekül in der Probe durchgeführt durch eine Substanz ausgewählt aus z.B Arginin, Prolin, oder Imidazol oder durch eine Mischung dieser.

In einer weiteren Ausführungsform dieses Verfahrens wird die Behandlung der Probe in einer Lösung durchgeführt, die mindestens eine der beschriebenen Substanzen enthält, wobei die einen pH zwischen 7,1 und 14, bevorzugt einen pH zwischen 7,1 und 12 und ganz besonders bevorzugt einen pH zwischen 7,4 und 10.

Dabei kann die zum Nachweis der zumindest einen Spezies Nukleinsäure geeignete Reaktion oder Reaktionssequenz ausgesucht sein z.B. aus der Gruppe der Nukleinsäure-Bindereaktionen, insbesondere der Nukleinsäure-Hybridisierungen, insbesondere Polymerisationen von Nukleinsäuren und insbesondere der Gruppe der Amplifikationsreaktionen. In einer besonderen Ausführungsform dieses Verfahrens ist das Nachweisverfahren der mindestens einen Spezies von RNA eine PCR (Polymerase-Kettenreaktion), Real-Time-PCR (als Sonden basierendes Verfahren wie auch als Verfahren, dass abhängig ist von einem sequenzunspezifisch bindenden Detektormolekül wie. z.B. SybrGreen), RT-PCR (Reverse Transkription Polymerase-Kettenreaktion), Real-time-RT-PCR (als Sonden basierendes Verfahren wie auch als Verfahren, dass abhängig ist von einem sequenzunspezifisch bindenden Detektormolekül wie. z.B. SybrGreen), Multiplex PCR, Multiplex RT-PCR, die sondenbasierenden Verfahren der Real-Time Multiplex PCR und Real-Time Multiplex RT-PCR. In dem beschriebenen Zusammenhang ist der Begriff RT-PCR wie folgt zu verstehen: Die Reaktion der RT-PCR besteht aus einer Reversen Transkription und einer PCR, wobei die beiden Reaktionen unabhängig voneinenader in zwei getrennten Gefäßen oder zusammen in einem Gefäß ablaufen kann. Beide Reaktionen können durch ein oder mehrere Enzyme durchgeführt werden. Neben der RT-PCR können auch anderer Reaktionen mit der mindestens einen Spezies von RNA druchgeführt warden wie z.B. NASBA (Nucleic Acid Sequence Based Amplification), 3SR (Sequence Sustained Self Replication), 2SR, TMA (Transcription Mediated Amplification), MDA (Multiple Displacement Amplification) Rolling Circle Amplification, Whole-Transcriptome-Amplification, Whole-Genome- Amplification und Rolling Transcription Amplification oder Loop-mediated isothermal amplification (LAMP).
Die zum Nachweis des zumindest einen Proteins geeignete Reaktion oder Reaktionssequenz kann ausgesucht sein aus der Gruppe der Protein-Bindereaktionen, insbesondere Protein-Erkennung, insbesondere durch andere Proteine, Reaktionen beruhend auf der enzymatischen Aktivität des Proteins, Antikörper, Aptamere, Liganden, Nukleinsäuren, insbesondere Western-Blotting, oder andere Substanzen wie Glutathion und NAD, oder ausgesucht ist aus der Gruppe der Proteinmodifikation oder -prozessierung, insbesondere (De)Phosphorylierung, (De)Glycosylierung, und Spaltung durch Proteasen.
Alle der oben genannten Verfahren können entweder in Lösung, Suspension oder Festphase durchgeführt werden.

Die Erfindung betrifft ein Verfahren zur Probenvorbereitung für einen nachfolgendes Präparations-, Prozessierungs- oder Analyseverfahren einer zumindest eine Spezies Nukleinsäure und/oder eine Spezies Protein enthaltenden Probe, wobei das Verfahren die folgenden Schritte umfasst: A) Bereitstellen einer Probe, die zumindest eine Spezies einer Nukleinsäure und/oder eines Proteins umfasst, B) In-Kontakt-Bringen der Probe mit einer flüssigen oder festen Zusammensetzung, um eine flüssige Probenzubereitung zu erzeugen, wobei die flüssige Probenzubereitung einen pH zwischen 7,1 und 14, bevorzugt einen pH zwischen 7,1 und 12 und ganz besonders bevorzugt einen pH zwischen 7,4 und 10 aufweist, und wobei die Zusammensetzung zumindest eine stickstoffhaltige Verbindung enthält, die ausgesucht ist aus der Gruppe bestehend aus a) Polyaminen, b) Aminosäuren, sowie Oligo- und Polypeptide, c) stickstoffhaltigen heterocyclischen Verbindungen, einschließlich Homo- oder Heteropolymeren, die diese stickstoffhaltigen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, sowie Mischungen aus zwei oder mehreren dieser Verbindungen.

Die Erfindung betrifft weiterhin ein Verfahren Verfahren zur Lyse einer biologischen Probe, die zumindest eine Spezies einer Nukleinsäure und/oder zumindest eine Spezies eines Proteins in einer Hülle enthält, wobei die Probe zur Erzeugung eines Lysats mit einer flüssigen oder festen Zusammensetzung in Kontakt gebracht wird, die zumindest eine stickstoffhaltige Verbindung enthält, die ausgesucht ist aus der Gruppe bestehend aus: a) Polyaminen, b) Aminosäuren, sowie Oligo- und Polypeptide, c) stickstoffhaltigen heterocyclischen Verbindungen, einschließlich Homo- oder Heteropolymeren, die diese stickstoffhaltigen Verbindungen umfassen, Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden f) anorganischen Ammoniumsalzen, g) Ammoniumgruppen enthaltenden inneren Salzverbindungen, h) Nukleinsäure bindenden Antibiotika, i) Verbindungen, die in der kleinen Grube der DNA binden, sowie Mischungen aus zwei oder mehreren dieser Verbindungen, wobei die so erzeugte flüssige Probenzubereitung einen pH zwischen 7,1 und 14, bevorzugt einen pH zwischen 7,1 und 12 und ganz besonders bevorzugt einen pH zwischen 7,4 und 10 aufweist.

Bevorzugt ist hierbei, dass die Lyse derart durchgeführt wird, dass die mindestens eine Spezies der Nukleinsäure und/oder Proteins in dem erzeugten Lysat gelöst oder suspendiert wird.
Weiterhin bevorzugt ist hierbei, dass die Probe zumindest zwei Spezies aus der Gruppe bestehend aus Nukleinsäuren und Proteinen enthält und die Lyse derart durchgeführt wird, dass mehrere bestimmte Spezies Nukleinsäure und/oder Proteine in dem erzeugten Lysat gelöst oder suspendiert werden.

Weiterhin bevorzugt ist hierbei, dass die Probe zumindest zwei Spezies aus der Gruppe bestehend aus Nukleinsäuren und Proteinen enthält und die Lyse derart durchgeführt wird, dass im Wesentlichen alle in der biologischen Probe enthaltenen Spezies Nukleinsäuren und/oder Proteine im erzeugten Lysat gelöst und/oder suspendiert werden.

Weiterhin bevorzugt ist hierbei, dass der Probe während der Lyse ein Trägermaterial zur Immobilisierung der zumindest einen Spezies der Nukleinsäure oder des Proteins zugegeben wird.

Weiterhin bevorzugt ist hierbei, dass zumindest eine Spezies Nukleinsäuren, insbesondere DNA und/oder RNA, in dem Lysat enthalten ist.

Weiterhin bevorzugt ist hierbei, dass eine oder mehrere Spezies Proteine in dem Lysat enthalten ist.

Weiterhin bevorzugt ist hierbei, dass die Zusammensetzung als weitere Lysereagenzien zumindest ein Reagenz aus der Gruppe der Komplexbildner, Detergentien, Substanzen zur Volumeneinengung, und/oder Lösemittel enthält.

Weiterhin bevorzugt ist hierbei, dass die Lyse unter mechanischer Einwirkung und/oder enzymatisch erfolgt.

Weiterhin bevorzugt ist hierbei, dass die Probe vor der Lyse mit einem Waschpuffer, insbesondere einem hypotonen Waschpuffer, gewaschen wird.

Weiterhin bevorzugt ist hierbei, dass die Konzentration der stickstoffhaltigen Verbindung in dem erzeugten Lysat zwischen 0,001 mM bis 1M beträgt, vorzugsweise zwischen 0,001 bis 100mM, insbesondere bevorzugt 0,001 bis 30 mM, und speziell 0.001 bis 19 mM und 0.001 bis 15mM.

Weiterhin bevorzugt ist hierbei, dass die zumindest eine Nukleinsäurespezies eine DNA-Spezies oder eine RNA-Spezies ist.

Weiterhin bevorzugt ist hierbei, dass a) die Polyamine ausgesucht sind aus der Gruppe umfassend offenkettige und cyclische Polyamine mit 2 bis 6 Aminogruppen, die Aminosäuren ausgesucht sind aus der Gruppe bestehend aus alpha-Aminosäuren, insbesondere den proteinogenen apolaren und polaren alpha-Aminosäuren, insbesondere aus der Gruppe bestehend aus Lysin, Arginin, Histidin, Prolin, Glycin, Alanin, Glutamin, Glutaminsäure und Tryptophan; die heterocyclischen Verbindungen ausgesucht sind aus der Gruppe der fünf- oder sechsgliedrigen Ringe bzw. der sechsgliedrigen Ringe mit anelliertem fünfgliedrigen Ring, wobei der fünfgliedrige Ring, der sechsgliedrige Ring und/oder der anellierte fünfgliedrige Ring 1 bis 3 Stickstoffatome aufweist und die einzelnen Ringglieder Substituenten ausgesucht aus der Gruppe bestehend aus C₁-C₅-Alkylgruppen, C(O)OH, - C(O)NH₂, =O, -OH, =S, -SH, =NH, -NH₂, Alkyl-O-, Alkyl-S-, Alkylamino- und Dialkylamino-, wobei die Alkylgruppen C₁-C₅-Alkylgruppen, vorzugsweise C₁-C₃-Alkylgruppen sind, sowie F-, Cl-, Br- oder J aufweisen können, und die heterocyclischen Verbindungen in den jeweiligen fünf- oder sechsgliedrigen Ringgruppen als weiteres Heteroatom ein oder mehrere O- oder S- Atome aufweisen können, und die heterocyclischen Verbindungen vorzugsweise ausgesucht sind aus der Gruppe bestehend aus Imidazol, Adenin, Guanin, Thymin, Cytosin, Pyrimidin, Pyridin, 2,3-Dimethylpyrazin, Thiazol, 2-Aminothiazol, Indazol, und Benzimidazol; d) in den Aminen vom Typ R¹R²NR³ die Reste R¹, R² und R³ unabhängig voneinander ausgewählt sind aus der Gruppe bestehend aus H und C₁-C₃-Alkylgruppen; e) die Carbonsäureamide die Struktur X-C(=O)NH₂ aufweisen, wobei X ausgesucht ist aus der Gruppe bestehend aus: -NH₂, C₁-C₅-Alkyl, C₂-C₅-Alkenyl, C₂-C₅-Alkinyl oder Aryl, vorzugsweise Phenyl oder ein aminosubstituiertes Aryl, H₂NC(=O)-Y-, wobei Y eine Alkylengruppe von Typ -(CH₂)ₙ- sein kann und wobei n eine ganze Zahl aus dem Bereich 0 bis 10, vorzugsweise 0 bis 5 ist, oder Y eine C₁-C₁₀-Alkenylengruppe, vorzugsweise eine C₁-C₆-Alkenylengruppe, oder eine Arylgruppe ist, und wobei X vorzugsweise -NH₂ oder 2-Aminophenyl ist; f) das anorganische Ammoniumsalz ausgesucht ist aus der Gruppe bestehend aus Ammoniumsulfat, Ammoniumhydrogenphoshat und Ammoniumcarbonat, g) die Ammoniumgruppen enthaltenden inneren Salzverbindungen ausgesucht sind aus Betain, Ectoin und Trimethylaminoxid; h) die Nukleinsäure bindenden Antibiotika ausgesucht sind aus der Gruppe bestehend aus Distamycinen, insbesondere Distamycin D, Mitomycinen, Norfloxacin, Streptozocin, Duocarmycinen, Actinomycinen, und Aminoglycisiden; und i) die Verbindungen, die in der kleinen Grube der DNA binden, ausgesucht sind aus der Gruppe bestehen aus Thiazotropsin, Tri-Imidazol und Chromomycinen.

Weiterhin bevorzugt ist hierbei, dass die stickstoffhaltige Verbindung ausgesucht ist aus der Gruppe a) der Polyamine, wobei die Polyamine vorzugsweise ausgesucht sind aus der Gruppe umfassend unverzweigte Polyamine mit 2 bis 6 Aminogruppen, insbesondere Spermidin, b) der polaren, basischen und sauren Aminosäuren, insbesondere Lysin, Arginin, Histidin, Glutaminsäure, oder c) der heterocyclischen Verbindungen, die ausgesucht sind aus der Gruppe Imidazol und 2,3-Dimethylpyrazin, Pyrimidin und Guanin.

Ein weiterer Gegenstand der Erfindung ist ein Analyseverfahren zum Nachweis zumindest einer Spezies Nukleinsäure und/oder zumindest einer Spezies Protein in einer Probe, umfassend die Schritte: A) Erzeugung eines Lysats gemäß dem Verfahren zur Lyse den oben beschriebenen bevorzugten Ausführungsformen, B) Verwendung des Lysats in einer zum Nachweis der zumindest einen Spezies Nukleinsäure und/oder der zumindest einen Spezies Protein geeigneten Reaktion oder Reaktionssequenz.

Bevorzugt ist hierbei, dass das Lysat direkt verwendet wird, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkenden Stoffe aus der Probenzubereitung durchgeführt werden.

Weiterhin bevorzugt ist hierbei, dass die zum Nachweis der zumindest einen Nukleinsäure geeignete Reaktion oder Reaktionssequenz ausgesucht ist aus der Gruppe der Nukleinsäure-Bindereaktionen, insbesondere der Nukleinsäure-Hybridisierungen, insbesondere Northern-, Southern-Blotting oder auch Hybridisierung von Oligonukleotiden und Sonden, der Gruppe der enzymatischen Modifikationen bzw. Polymerisationen von Nukleinsäuren, insbesondere Sequenzierungsreaktionen, in-vitro Transkription, Restriktionsendonuklease-spaltungen, der Gruppe der Amplifikationsreaktionen, insbesondere PCR (Polymerase-Kettenreaktion), real-time-PCR, RT-PCR (Reverse Transkription Polymerase-Kettenreaktion), Real-time-RT-PCR, NASBA (Nucleic Acid Sequence Based Amplification), 3SR (Sequence Sustained Self Replication), 2 SR, TMA (Transcription Mediated Amplification), MDA (Multiple Displacement Amplification) Rolling Circle Amplification und Rolling Transcription Amplification.

Weiterhin bevorzugt ist hierbei, dass die zum Nachweis des zumindest einen Proteins geeignete Reaktion oder Reaktionssequenz ausgesucht ist aus der Gruppe der Protein-Bindereaktionen, insbesondere Protein-Erkennung, insbesondere durch andere Proteine, Reaktionen beruhend auf der enzymatischen Aktivität des Proteins, Antikörper, Aptamere, Liganden, Nukleinsäuren, insbesondere Western-Blotting, oder andere Substanzen wie Glutathion und NAD, oder ausgesucht ist aus der Gruppe der Proteinmodifikation oder - prozessierung, insbesondere (De)Phosphorylierung, (De)Glycolysierung, und Spaltung durch Proteasen.

Ein weiterer Gegenstand der Erfindung ist ein Verfahren zur Stabilisierung von Nukleinsäuren und/oder Proteinen umfassend: A) Bereitstellen einer Probe, die zumindest eine Spezies einer Nukleinsäure und/oder eines Proteins umfasst, B) In-Kontakt-Bringen der Probe mit einer flüssigen oder festen Zusammensetzung, um eine flüssige Probenzubereitung zu erzeugen, wobei die flüssige Probenzubereitung einen pH zwischen 7,1 und 14, bevorzugt einen pH zwischen 7,1 und 12 und ganz besonders bevorzugt einen pH zwischen 7,4 und 10 aufweist, und wobei die Zusammensetzung zumindest eine stickstoffhaltige Verbindung enthält, die ausgesucht ist aus der Gruppe bestehend aus a) Polyaminen, b) Aminosäuren, sowie Oligo- und Polypeptide, c) stickstoffhaltigen heterocyclischen Verbindungen, einschließlich Homo- oder Heteropolymeren, die diese stickstoffhaltigen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, sowie Mischungen aus zwei oder mehreren dieser Verbindungen.

Bevorzugt ist hierbei, dass die zumindest eine Spezies Nukleinsäure oder Protein vor, während oder nach der Zugabe der stickstoffhaltigen Verbindung auf einem Trägermaterial immobilisiert wird.

Weiterhin bevorzugt ist hierbei, dass die Erzeugung der Probenzubereitung derart erfolgt, dass die zumindest eine Spezies der Nukleinsäure und/oder des Proteins in der flüssigen Probenzubereitung gelöst und/oder suspendiert wird.

Weiterhin bevorzugt ist hierbei, dass die Probe eine biologische Probe ist, die vor dem In-Kontakt-Bringen mit der Zusammensetzung zur Erzeugung der flüssigen Probenzubereitung einer Lyse und gegebenenfalls weiteren Aufreinigungsschritten unterzogen wird.

Weiterhin bevorzugt ist hierbei, dass die Probe eine biologische Probe ist, in der die zumindest eine Spezies einer Nukleinsäure und/oder zumindest eines Proteins in einer Hülle enthalten ist und wobei die Probe mit der flüssigen oder festen Zusammensetzung in Kontakt gebracht wird, um ein Lysat zu erzeugen.

Weiterhin bevorzugt ist hierbei, dass die Probe zumindest zwei Spezies aus der Gruppe bestehend aus Nukleinsäuren und Proteinen enthält und die Erzeugung der Probenzubereitung bzw. die Lyse derart durchgeführt wird, dass mehrere bestimmte Spezies Nukleinsäure und/oder Proteine in dem erzeugten Lysat gelöst und/oder suspendiert oder immobilisiert werden.

Weiterhin bevorzugt ist hierbei, dass die Probe zumindest zwei Spezies aus der Gruppe bestehend aus Nukleinsäuren und Proteinen enthält und die Erzeugung der Probenzubereitung bzw. der Lyse derart durchgeführt wird, dass im Wesentlichen alle in der Probe enthaltenen Spezies Nukleinsäuren und/oder Proteine in der Probenzubereitung bzw. im Lysat gelöst und/oder suspendiert oder immobilisiert werden.

Weiterhin bevorzugt ist hierbei, dass DNA und/oder RNA in der Probenzubereitung bzw. dem Lysat gelöst und/oder suspendiert oder immobilisiert wird.

Weiterhin bevorzugt ist hierbei, dass zumindest eine Spezies Protein in der Probenzubereitung bzw. dem Lysat gelöst und/oder suspendiert oder immobilisiert wird.

Weiterhin bevorzugt ist hierbei, dass die Zusammensetzung als weitere Lysereagenzien zumindest ein Reagenz aus der Gruppe der Komplexbildner, Detergentien, Substanzen zur Volumeneinengung und/oder Lösemittel enthält.

Weiterhin bevorzugt ist hierbei, dass die Lyse unter mechanischer Einwirkung und/oder enzymatisch erfolgt.

Weiterhin bevorzugt ist hierbei, dass die Probe vor der Lyse mit einem Waschpuffer, vorzugsweise mit einem hypotonen Waschpuffer, gewaschen wird.

Weiterhin bevorzugt ist hierbei, dass die Konzentration der stickstoffhaltigen Verbindung in der erzeugten Probenzubereitung bzw dem Lysat zwischen 0,001 mM bis 1M beträgt, vorzugsweise zwischen 0,001 bis 100mM, insbesondere vorzugweise zwischen 0,001 bis 30 mM und speziell 0,001 bis 19 mM oder 0,001 bis 15 mM.

Weiterhin bevorzugt ist hierbei, dass a) die Polyamine ausgesucht sind aus der Gruppe umfassend offenkettige und cyclische Polyamine mit 2 bis 6 Aminogruppen, b) die Aminosäuren ausgesucht sind aus der Gruppe bestehend aus alpha-Aminosäuren, insbesondere den proteinogenen apolaren und polaren alpha-Aminosäuren, insbesondere aus der Gruppe bestehend aus Lysin, Arginin, Histidin, Prolin, Glycin, Alanin, Glutamin, Glutaminsäure und Tryptophan; c) die heterocyclischen Verbindungen ausgesucht sind aus der Gruppe der fünf- oder sechsgliedrigen Ringe bzw. der sechsgliedrigen Ringe mit anelliertem fünfgliedrigen Ring, wobei der fünfgliedrige Ring, der sechsgliedrige Ring und/oder der anellierte fünfgliedrige Ring 1 bis 3 Stickstoffatome aufweist und die einzelnen Ringglieder Substituenten ausgesucht aus der Gruppe bestehend aus C₁-C₅-Alkylgruppen, C(O)OH, -C(O)NH₂, =O, -OH, =S, -SH, =NH, -NH₂, C₁-C₆-Alkyl-O-, C₁-C₆-Alkyl-S-, Alkylamino- und Dialkylamino-, wobei die Alkylgruppen C₁-C₅-Alkylgruppen, vorzugsweise C₁-C₃-Alkylgruppen sind, sowie F-, Cl-, Br- oder J aufweisen können, und die heterocyclischen Verbindungen in den jeweiligen fünf- oder sechsgliedrigen Ringgruppen als weiteres Heteroatom ein oder mehrere O- oder S- Atome aufweisen können, und die heterocyclischen Verbindungen vorzugsweise ausgesucht sind aus der Gruppe bestehend aus Imidazol, Adenin, Guanin, Thymin, Cytosin, Pyrimidin, Pyridin, 2,3-Dimethylpyrazin, Thiazol, 2-Aminothiazol, Indazol, und Benzimidazol; d) in den Aminen vom Typ R¹R²NR³ die Reste R¹, R² und R³ unabhängig voneinander ausgewählt sind aus der Gruppe bestehend aus H und C₁-C₃-Alkylgruppen; e) die Carbonsäureamide die Struktur X-C(=O)NH₂ aufweisen, wobei X ausgesucht ist aus der Gruppe bestehend aus: -NH₂, C₁-C₅-Alkyl, C₂-C₅-Alkenyl, C₂-C₅-Alkinyl oder Aryl, vorzugsweise Phenyl oder ein aminosubstituiertes Aryl, H₂NC(=O)-Y-, wobei Y eine Alkylengruppe von Typ -(CH₂)ₙ- sein kann und wobei n eine ganze Zahl aus dem Bereich 0 bis 10, vorzugsweise 0 bis 5 ist, oder Y eine C₁-C₁₀-Alkenylengruppe, vorzugsweise eine C₁-C₆-Alkenylengruppe, oder eine Arylgruppe ist, und wobei X vorzugsweise -NH₂ oder 2-Aminophenyl ist; f) das anorganische Ammoniumsalz ausgesucht ist aus der Gruppe bestehend aus Ammoniumsulfat, Ammoniumhydrogenphoshat und Ammoniumcarbonat, g) die Ammoniumgruppen enthaltenden inneren Salzverbindungen ausgesucht sind aus Betain, Ectoin und Trimethylaminoxid; h) die Nukleinsäure bindenden Antibiotika ausgesucht sind aus der Gruppe bestehend aus Distamycinen, insbesondere Distamycin D, Mitomycinen, Norfloxacin, Streptozocin, Duocarmycinen, Actinomycinen, und Aminoglycisiden; und i) die Verbindungen, die in der kleinen Grube der DNA binden, ausgesucht sind aus der Gruppe bestehen aus Thiazotropsin, Tri-Imidazol und Chromomycinen.

Weiterhin bevorzugt ist hierbei, dass die stickstoffhaltige Verbindung ausgesucht ist aus der Gruppe a) der Polyamine, wobei die Polyamine vorzugsweise ausgesucht sind aus der Gruppe umfassend unverzweigte Polyamine mit 2 bis 6 Aminogruppen, insbesondere Spermidin, b) der polaren, basischen und sauren Aminosäuren, insbesondere Lysin, Arginin, Histidin, Glutaminsäure, c) der heterocyclischen Verbindungen, die ausgesucht sind aus der Gruppe Imidazol und 2,3-Dimethylpyrazin, Pyrimidin und Guanin.

Ein weiterer Gegenstand der Erfindung ist ein Analyseverfahren zum Nachweis zumindest einer Spezies von Nukleinsäuren und/oder Proteinen in einer Probe, umfassend die Schritte: a) Bereitstellen einer Probenzubereitung oder eines Lysats, das zumindest eine Spezies einer Nukleinsäure und/oder zumindest eines Proteins enthält, wobei die zumindest eine Spezies Nukleinsäure und/oder Protein mit dem Verfahren gemäß einem der oben beschriebenen Ausführungsformen stabilisiert wurde und b) Verwendung der Probenzubereitung oder des Lysats in einer zum Nachweis der zumindest einen Nukleinsäure oder des zumindest einen Proteins geeigneten Reaktion oder Reaktionssequenz.

Bevorzugt ist hierbei, dass die Probenzubereitung bzw. das Lysat direkt verwendet wird, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkenden Stoffe aus der Probenzubereitung durchgeführt werden.

Weiterhin bevorzugt ist hierbei, dass die zum Nachweis der zumindest einen Nukleinsäure geeignete Reaktion oder Reaktionssequenz ausgesucht ist aus der Gruppe der Nukleinsäure-Bindereaktionen, insbesondere der Nukleinsäure-Hybridisierungen, insbesondere Northern-, Southern-Blotting oder auch Hybridisierung von Oligonukleotiden und Sonden, der Gruppe der enzymatischen Modifikationen bzw. Polymerisationen von Nukleinsäuren, insbesondere Sequenzierungsreaktionen, in-vitro Transkription, Restriktionsendonuklease-spaltungen, der Gruppe der Amplifikationsreaktionen, insbesondere PCR (Polymerase-Kettenreaktion), Real-time-PCR, RT-PCR (Reverse Transkription Polymerase-Kettenreaktion), Real-time-RT-PCR, NASBA (Nucleic Acid Sequence Based Amplification), 3SR (Sequence Sustained Self Replication), 2 SR, TMA (Transcription Mediated Amplification), MDA (Multiple Displacement Amplification) Rolling Circle Amplification und Rolling Transcription Amplification.

Weiterhin bevorzugt ist hierbei, dass die in der Probenzubereitung bzw. dem Lysat enthaltende Nukleinsäurespezies RNA ist und in Schritt b) die Reaktion bzw. Reaktionssequenz RT-PCR ist, vorzugsweise Real-Time-RT-PCR.

Weiterhin bevorzugt ist hierbei, dass die zum Nachweis des zumindest einen Proteins geeignete Reaktion oder Reaktionssequenz ausgesucht ist aus der Gruppe der Protein-Bindereaktionen, insbesondere Protein-Erkennung, insbesondere durch andere Proteine, Reaktionen beruhend auf der enzymatischen Aktivität des Proteins, Antikörper, Aptamere, Liganden, Nukleinsäuren, insbesondere Western-Blotting, oder andere Substanzen wie Glutathion und NAD, oder ausgesucht ist aus der Gruppe der Proteinmodifikation oder - prozessierung, insbesondere (De)Phosphorylierung, (De)Glycolysierung, und Spaltung durch Proteasen.

Weiterhin bevorzugt ist hierbei, dass die Proteinerkennung durch Antikörper und/oder Aptamere erfolgt.

Ein weiterer Gegenstand der Erfindung ist ein Verfahren zur Verminderung inhibitorischer Effekte in einer Probe enthaltend zumindest eine Spezies Nukleinsäure und/oder eine Spezies Protein sowie umfassend zumindest eine inhibierende Substanz, wobei das Verfahren die folgenden Schritte umfasst: A) Bereitstellen einer Probe, die zumindest eine Spezies einer Nukleinsäure und/oder eines Proteins sowie zumindest eine inhibierende Substanz umfasst, B) In-Kontakt-Bringen der Probe mit einer flüssigen oder festen Zusammensetzung, um eine flüssige Probenzubereitung zu erzeugen, wobei die flüssige Probenzubereitung einen pH zwischen 7,1 und 14, bevorzugt einen pH zwischen 7,1 und 12 und ganz besonders bevorzugt einen pH zwischen 7,4 und 10 aufweist, und wobei die Zusammensetzung zumindest eine stickstoffhaltige Verbindung enthält, die ausgesucht ist aus der Gruppe bestehend aus a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen Verbindungen umfassen, Aminen vom Typ R¹R²NR³, wobei R¹, R² und R³ unabhängig voneinander ausgewählt sind aus der Gruppe bestehend aus H, C₁-C₅-Alkylgruppen und Arylgruppe, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, sowie Mischungen aus zwei oder mehreren dieser Verbindungen, wobei die Menge der stickstoffhaltigen Verbindung, die der Probe zugegeben wird so gewählt ist, dass in der flüssigen Probenzubereitung der inhibierende Effekt der inhibierenden Substanz verringert wird.

Bevorzugt ist hierbei, dass die Probe eine biologische Probe ist, die vor dem In-Kontakt-Bringen mit der Zusammensetzung zur Erzeugung der flüssigen Probenzubereitung einer Lyse und gegebenenfalls weiteren Aufreinigungsschritten unterzogen wird.

Weiterhin bevorzugt ist hierbei, dass die zumindest eine Spezies Nukleinsäure oder Protein vor oder während der Zugabe der stickstoffhaltigen Verbindung auf einem Trägermaterial immobilisiert wird.

Weiterhin bevorzugt ist hierbei, dass die Erzeugung der Probenzubereitung derart erfolgt, dass die zumindest eine Spezies der Nukleinsäure und/oder des Proteins in der flüssigen Probenzubereitung gelöst und/oder suspendiert wird.

Weiterhin bevorzugt ist hierbei, dass die Probe eine biologische Probe ist, in der die zumindest eine Spezies der Nukleinsäure und/oder die zumindest eine Spezies des Proteins in einer Hülle enthalten ist und wobei die Probe zur Erzeugung eines Lysats mit der flüssigen oder festen Zusammensetzung in Kontakt gebracht wird.

Weiterhin bevorzugt ist hierbei, dass die Probe zumindest zwei Spezies aus der Gruppe bestehend aus Nukleinsäuren und Proteinen enthält und die Erzeugung der Probenzubereitung bzw. die Lyse derart durchgeführt wird, dass mehrere bestimmte Spezies Nukleinsäure und/oder Proteine in dem erzeugten Lysat gelöst und/oder suspendiert oder immobilisiert werden.

Weiterhin bevorzugt ist hierbei, dass die Probe zumindest zwei Spezies aus der Gruppe bestehend aus Nukleinsäuren und Proteinen enthält und die Erzeugung der Probenzubereitung bzw. der Lyse derart durchgeführt wird, dass im Wesentlichen alle in der Probe enthaltenen Spezies Nukleinsäuren und/oder Proteine in der Probenzubereitung bzw. im Lysat gelöst und/oder suspendiert oder immobilisiert werden.

Weiterhin bevorzugt ist hierbei, dass zumindest eine Spezies DNA und/oder RNA in der Probenzubereitung bzw. dem Lysat gelöst und/oder suspendiert oder immobilisiert wird.

Weiterhin bevorzugt ist hierbei, dass die zumindest eine Spezies Protein in der Probenzubereitung bzw. dem Lysat gelöst und/oder suspendiert oder immobilisiert wird.

Weiterhin bevorzugt ist hierbei, dass die Zusammensetzung als weitere Lysereagenzien zumindest ein Reagenz aus der Gruppe der Komplexbildner, Detergentien, Substanzen zur Volumeneinengung und/oder Lösemittel enthält.

Weiterhin bevorzugt ist hierbei, dass die Lyse unter mechanischer Einwirkung und/oder enzymatisch erfolgt.

Weiterhin bevorzugt ist hierbei, dass die Probe vor der Lyse mit einem Waschpuffer, vorzugsweise mit einem hypotonen Waschpuffer, gewaschen wird.

Weiterhin bevorzugt ist hierbei, dass die Konzentration der stickstoffhaltigen Verbindung in der erzeugten Probenzubereitung bzw. dem Lysat zwischen 0,001 mM bis 1 M, vorzugsweise zwischen 0,001 bis 100mM, insbesondere zwischen 0,001 bis 30mM beträgt, und speziell zwischen 0,001 bis 19 mM oder 0,001 bis 15 mM beträgt.

Weiterhin bevorzugt ist hierbei, dass die zumindest eine Nukleinsäurespezies eine DNA-Spezies, insbesondere gDNA und/oder eine RNA-Spezies ist.

Weiterhin bevorzugt ist hierbei, dass a) die Polyamine ausgesucht sind aus der Gruppe umfassend offenkettige und cyclische Polyamine mit 2 bis 6 Aminogruppen, b) die Aminosäuren ausgesucht sind aus der Gruppe bestehend aus alpha-Aminosäuren, insbesondere den proteinogenen apolaren und polaren alpha-Aminosäuren, insbesondere aus der Gruppe bestehend aus Lysin, Arginin, Histidin, Prolin, Glycin, Alanin, Glutamin, Glutaminsäure und Tryptophan; c) die heterocyclischen Verbindungen ausgesucht sind aus der Gruppe der fünf- oder sechsgliedrigen Ringe bzw. der sechsgliedrigen Ringe mit anelliertem fünfgliedrigen Ring, wobei der fünfgliedrige Ring, der sechsgliedrige Ring und/oder der anellierte fünfgliedrige Ring 1 bis 3 Stickstoffatome aufweist und die einzelnen Ringglieder Substituenten ausgesucht aus der Gruppe bestehend aus C₁-C₅-Alkylgruppen, C(O)OH, -C(O)NH₂, =O, -OH, =S, -SH, =NH, -NH₂, C₁-C₆-Alkyl-O-, C₁-C₆-Alkyl-S-, Alkylamino- und Dialkylamino-, wobei die Alkylgruppen C₁-C₅-Alkylgruppen, vorzugsweise C₁-C₃-Alkylgruppen sind, sowie F-, Cl-, Br- oder J aufweisen können, und die heterocyclischen Verbindungen in den jeweiligen fünf- oder sechsgliedrigen Ringgruppen als weiteres Heteroatom ein oder mehrere O- oder S- Atome aufweisen können, und die heterocyclischen Verbindungen vorzugsweise ausgesucht sind aus der Gruppe bestehend aus Imidazol, Adenin, Guanin, Thymin, Cytosin, Pyrimidin, Pyridin, 2,3-Dimethylpyrazin, Thiazol, 2-Aminothiazol, Indazol, und Benzimidazol; d) in den Aminen vom Typ R¹R²NR³ die Reste R¹, R² und R³ unabhängig voneinander ausgewählt sind aus der Gruppe bestehend aus H und C₁-C₃-Alkylgruppen; e) die Carbonsäureamide die Struktur X-C(=O)NH₂ aufweisen, wobei X ausgesucht ist aus der Gruppe bestehend aus: -NH₂, C₁-C₅-Alkyl, C₂-C₅-Alkenyl, C₂-C₅-Alkinyl oder Aryl, vorzugsweise Phenyl oder ein aminosubstituiertes Aryl, H₂NC(=O)-Y-, wobei Y eine Alkylengruppe von Typ -(CH₂)ₙ- sein kann und wobei n eine ganze Zahl aus dem Bereich 0 bis 10, vorzugsweise 0 bis 5 ist, oder Y eine C₁-C₁₀-Alkenylengruppe, vorzugsweise eine C₁-C₆-Alkenylengruppe, oder eine Arylgruppe ist, und wobei X vorzugsweise -NH₂ oder 2-Aminophenyl ist; f) das anorganische Ammoniumsalz ausgesucht ist aus der Gruppe bestehend aus Ammoniumsulfat, Ammoniumhydrogenphoshat und Ammoniumcarbonat, g) die Ammoniumgruppen enthaltenden inneren Salzverbindungen ausgesucht sind aus Betain, Ectoin und Trimethylaminoxid; h) die Nukleinsäure bindenden Antibiotika ausgesucht sind aus der Gruppe bestehend aus Distamycinen, insbesondere Distamycin D, Mitomycinen, Norfloxacin, Streptozocin, Duocarmycinen, Actinomycinen, und Aminoglycisiden; und i) die Verbindungen, die in der kleinen Grube der DNA binden, ausgesucht sind aus der Gruppe bestehen aus Thiazotropsin, Tri-Imidazol und Chromomycinen.

Weiterhin bevorzugt ist hierbei, dass die stickstoffhaltige Verbindung ausgesucht ist aus der Gruppe a) der Polyamine, wobei die Polyamine vorzugsweise ausgesucht sind aus der Gruppe umfassend unverzweigte Polyamine mit 2 bis 6 Aminogruppen, insbesondere Spermidin, b) der polaren, basischen und sauren Aminosäuren, insbesondere Lysin, Arginin, Histidin, Glutaminsäure, oder c) der heterocyclischen Verbindungen, die ausgesucht sind aus der Gruppe Imidazol und 2,3-Dimethylpyrazin, Pyrimidin und Guanin.

Ein weiterer Gegenstand der Erfindung ist ein Analyseverfahren zum Nachweis zumindest einer Spezies von Nukleinsäuren und/oder Proteinen in einer Probe, umfassend die Schritte: a) Bereitstellen einer Probenzubereitung oder eines Lysats, das zumindest eine Spezies einer Nukleinsäure und/oder zumindest eines Proteins enthält, wobei in der Probenzubereitung bzw. dem Lysat inhibitorische Effekte mit dem Verfahren gemäß den obigen Ausführungsformen vermindert wurden; b) Verwendung der Probenzubereitung in einer zum Nachweis der zumindest einen Nukleinsäure oder des zumindest einen Proteins geeigneten Reaktion oder Reaktionssequenz.

Bevorzugt ist hierbei, dass die Probenzubereitung bzw. das Lysat in Schritt b) direkt verwendet wird, ohne dass vor der Nachweisreaktion weitere Verfahrensschritte zur Reduzierung der Anzahl der in der Probenzubereitung enthaltenen Spezies von Nukleinsäuren und/oder Proteinen und/oder zur Entfernung bzw. Inaktivierung von den Abbau von Nukleinsäuren und/oder Proteinen bewirkenden Stoffen aus der Probenzubereitung durchgeführt werden.

Weiterhin bevorzugt ist hierbei, dass die zum Nachweis der zumindest einen Nukleinsäure oder des zumindest einen Proteins geeignete Reaktion oder Reaktionssequenz ausgesucht ist aus der Gruppe der Nukleinsäure-Bindereaktionen, insbesondere der Nukleinsäure-Hybridisierungen, insbesondere Northern-, Southern-Blotting oder auch Hybridisierung von Oligonukleotiden und Sonden, der Gruppe der enzymatischen Modifikationen bzw. Polymerisationen von Nukleinsäuren, insbesondere Sequenzierungsreaktionen, in-vitro Transkription, Restriktionsendonuklease-spaltungen, der Gruppe der Amplifikationsreaktionen, insbesondere PCR (Polymerase-Kettenreaktion), Real-time-PCR, RT-PCR (Reverse Transkription Polymerase-Kettenreaktion), Real-time-RT-PCR, NASBA (Nucleic Acid Sequence Based Amplification), 3SR (Sequence Sustained Self Replication), 2 SR, TMA (Transcription Mediated Amplification), MDA (Multiple Displacement Amplification) Rolling Circle Amplification und Rolling Transcription Amplification.

Weiterhin bevorzugt ist hierbei, dass die zum Nachweis des zumindest einen Proteins geeignete Reaktion oder Reaktionssequenz ausgesucht ist aus der Gruppe der der Protein-Bindereaktionen, insbesondere Protein-Erkennung, insbesondere durch andere Proteine, Reaktionen beruhend auf der enzymatischen Aktivität des Proteins, Antikörper, Aptamere, Liganden, Nukleinsäuren, insbesondere Western-Blotting, oder andere Substanzen wie Glutathion und NAD, oder ausgesucht ist aus der Gruppe der Proteinmodifikation oder - prozessierung, insbesondere (De)Phosphorylierung, (De)Glycolysierung, und Spaltung durch Proteasen.

Weiterhin bevorzugt ist hierbei, dass die Probenzubereitung oder das Lysat RNA neben gDNA enthält und in Schritt b) die Reaktionssequenz die Reaktion von Nukleinsäure mit Enzymen umfasst.

Weiterhin bevorzugt ist hierbei, dass die Reaktionssequenz in Schritt b) die Schritte b1) optional enzymatischer Abbau von gDNA und nachfolgend b2) Reverse Transkription oder RT-PCR, vorzugsweise Real-Time-RT-PCR zum Nachweis von RNA umfasst.

Weiterhin bevorzugt ist hierbei, dass die Probenzubereitung oder das Lysat mindenstens eine Spezies Protein enthält und die Reaktion bzw. Reaktionssequenz in Schritt b) den Schritt b1) Bindung durch mindestens einen Antikörper oder ein Aptamer umfasst.

Ein weiterer Gegenstand der Erfindung ist ein Verfahren zur selektiven Maskierung zumindest einer Spezies einer Nukleinsäure in einer Probe, die zumindest eine erste Spezies einer Nukleinsäure und eine von der ersten Spezies unterschiedliche zweite Spezies Nukleinsäure enthält, das Verfahren umfassend die Schritte: A) Bereitstellen einer Probe, die zumindest zwei unterschiedliche Spezies Nukleinsäure enthält, B) In-Kontakt-Bringen der Probe mit einer flüssigen oder festen Zusammensetzung, um eine flüssige Probenzubereitung zu erzeugen, wobei die flüssige Probenzubereitung einen pH zwischen 7,1 und 14, bevorzugt einen pH zwischen 7,1 und 12 und ganz besonders bevorzugt einen pH zwischen 7,4 und 10 aufweist, und wobei die Zusammensetzung zumindest eine stickstoffhaltige Verbindung enthält, die ausgesucht ist aus der Gruppe bestehend aus: a) Polyaminen, b) Aminosäuren, sowie Oligo- und Polypeptiden, c) stickstoffhaltigen heterocyclischen Verbindungen, einschließlich Homo- oder Heteropolymeren, die diese stickstoffhaltigen Verbindungen umfassen, d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind, e) Carbonsäureamiden, f) anorganischen Ammoniumsalzen, g) Ammoniumgruppen enthaltenden inneren Salzverbindungen, h) Nukleinsäure bindende Antibiotika, i) Verbindungen, die in der kleinen Grube der DNA binden, sowie Mischungen aus zwei oder mehreren dieser Verbindungen, wobei die Erzeugung der Probenzubereitung derart durchgeführt wird, dass sowohl die erste und die zweite Spezies Nukleinsäure in der flüssigen Probenzubereitung gelöst und/oder suspendiert wird, und wobei die Menge der stickstoffhaltigen Verbindung so gewählt ist, dass die zweite Spezies Nukleinsäure derart maskiert wird, dass die erste Spezies Nukleinsäure in einem Nachweisverfahren für Nukleinsäuren verbessert nachgewiesen werden kann, verglichen mit dem gleichen Nachweisverfahren, bei dem der Probe die stickstoffhaltige Verbindung nicht zugegeben wurde.

Bevorzugt ist hierbei, dass die Probe eine biologische Probe ist, die vor dem In-Kontakt-Bringen mit der Zusammensetzung zur Erzeugung der flüssigen Probenzubereitung einer Lyse und gegebenenfalls weiteren Aufreinigungsschritten unterzogen wird.

Weiterhin bevorzugt ist hierbei, dass die Probe eine biologische Probe ist, in der die Spezies Nukleinsäuren in einer Hülle enthalten sind und wobei die Probe mit der flüssigen oder festen Zusammensetzung in Kontakt gebracht wird, um ein Lysat zu erzeugen.

Weiterhin bevorzugt ist hierbei, dass die Probe zumindest zwei Spezies Nukleinsäuren enthält und die Erzeugung der Probenzubereitung bzw. die Lyse derart durchgeführt wird, dass mehrere bestimmte Spezies Nukleinsäure und/oder Proteine in dem erzeugten Lysat gelöst oder suspendiert werden.

Weiterhin bevorzugt ist hierbei, dass die Probe zumindest zwei Spezies Nukleinsäure enthält und die Erzeugung der Probenzubereitung bzw. der Lyse derart durchgeführt wird, dass im Wesentlichen alle in der Probe enthaltenen Spezies Nukleinsäuren in der Probenzubereitung bzw. im Lysat gelöst und/oder suspendiert werden.

Weiterhin bevorzugt ist hierbei, dass a) die erste Spezies Nukleinsäure eine Spezies DNA und die zweite Spezies Nukleinsäure eine Spezies RNA oder PNA ist; oder b) die erste Spezies Nukleinsäure eine Spezies RNA und die zweite Spezies Nukleinsäure eine Spezies DNA oder PNA ist; oder c) die erste Spezies Nukleinsäure eine Spezies PNA ist und die zweite Spezies eine Spezies DNA oder RNA ist, d) sowohl die erste Spezies Nukleinsäure als auch die zweite Spezies Nukleinsäure DNA-Spezies sind, oder e) sowohl die erste Spezies Nukleinsäure als auch die zweite Spezies Nukleinsäure RNA-Spezies sind, oder f) sowohl die erste Spezies Nukleinsäure als auch die zweite Spezies Nukleinsäure PNA sind.

Weiterhin bevorzugt ist hierbei, dass die Zusammensetzung als weitere Lysereagenzien zumindest ein Reagenz aus der Gruppe der Komplexbildner, Detergentien, Substanzen zur Volumeneinengung, und/oder Lösemittel enthält.

Weiterhin bevorzugt ist hierbei, dass die Lyse unter mechanischer Einwirkung und/oder enzymatisch erfolgt.

Weiterhin bevorzugt ist hierbei, dass die Probe vor der Lyse mit einem Waschpuffer, vorzugsweise mit einem hypotonen Waschpuffer, gewaschen wird.

Weiterhin bevorzugt ist hierbei, dass die Konzentration der stickstoffhaltigen Verbindung in der erzeugten Probenzubereitung bzw. dem Lysat zwischen 0,001 mM bis 1M, vorzugsweise 0,001 mM bis 100 mM, insbesondere vorzugeweise 0,001 bis 30 mM, speziell 0,001 bis 19 mM beträgt oder 0,001 bis 15 mM beträgt.

Weiterhin bevorzugt ist hierbei, dass a) die Polyamine ausgesucht sind aus der Gruppe umfassend offenkettige und cyclische Polyamine mit 2 bis 6 Aminogruppen, b) die Aminosäuren ausgesucht sind aus der Gruppe bestehend aus alpha-Aminosäuren, insbesondere den proteinogenen apolaren und polaren alpha-Aminosäuren, insbesondere aus der Gruppe bestehend aus Lysin, Arginin, Histidin, Prolin, Glycin, Alanin, Glutamin, Glutaminsäure und Tryptophan; c) die heterocyclischen Verbindungen ausgesucht sind aus der Gruppe der fünf- oder sechsgliedrigen Ringe bzw. der sechsgliedrigen Ringe mit anelliertem fünfgliedrigen Ring, wobei der fünfgliedrige Ring, der sechsgliedrige Ring und/oder der anellierte fünfgliedrige Ring 1 bis 3 Stickstoffatome aufweist und die einzelnen Ringglieder Substituenten ausgesucht aus der Gruppe bestehend aus C₁-C₅-Alkylgruppen, C(O)OH, -C(O)NH₂, =O, -OH, =S, -SH, =NH, -NH₂, C₁-C₆-Alkyl-O-, C₁-C₆-Alkyl-S-, Alkylamino- und Dialkylamino-, wobei die Alkylgruppen C₁-C₅-Alkylgruppen, vorzugsweise C₁-C₃-Alkylgruppen sind, sowie F-, Cl-, Br- oder J aufweisen können, und die heterocyclischen Verbindungen in den jeweiligen fünf- oder sechsgliedrigen Ringgruppen als weiteres Heteroatom ein oder mehrere O- oder S- Atome aufweisen kann, und die heterocyclischen Verbindungen vorzugsweise ausgesucht sind aus der Gruppe bestehend aus Imidazol, Adenin, Guanin, Thymin, Cytosin, Pyrimidin, Pyridin, 2,3-Dimethylpyrazin, Thiazol, 2-Aminothiazol, Indazol, und Benzimidazol; d) in den Aminen vom Typ R¹R²NR³ die Reste R¹, R² und R³ unabhängig voneinander ausgewählt sind aus der Gruppe bestehend aus H und C₁-C₃-Alkylgruppen; e) die Carbonsäureamide die Struktur X-C(=O)NH₂ aufweisen, wobei X ausgesucht ist aus der Gruppe bestehend aus: -NH₂, C₁-C₅-Alkyl, C₂-C₅-Alkenyl, C₂-C₅-Alkinyl oder Aryl, vorzugsweise Phenyl oder ein aminosubstituiertes Aryl, H2NC(=O)-Y-, wobei Y eine Alkylengruppe von Typ -(CH₂)ₙ- sein kann und wobei n eine ganze Zahl aus dem Bereich 0 bis 10, vorzugsweise 0 bis 5 ist, oder Y eine C₁-C₁₀-Alkenylengruppe, vorzugsweise eine C₁-C₆-Alkenylengruppe, oder eine Arylgruppe ist, und wobei X vorzugsweise -NH₂ oder 2-Aminophenyl ist; f) das anorganische Ammoniumsalz ausgesucht ist aus der Gruppe bestehend aus Ammoniumsulfat, Ammoniumhydrogenphoshat und Ammoniumcarbonat, g) die Ammoniumgruppen enthaltenden inneren Salzverbindungen ausgesucht sind aus Betain, Ectoin und Trimethylaminoxid; h) die Nukleinsäure bindenden Antibiotika ausgesucht sind aus der Gruppe bestehend aus Distamycinen, insbesondere Distamycin D, Mitomycinen, Norfloxacin, Streptozocin, Duocarmycinen, Actinomycinen, und Aminoglycisiden; und i) die Verbindungen, die in der kleinen Grube der DNA binden, ausgesucht sind aus der Gruppe bestehen aus Thiazotropsin, Tri-Imidazol und Chromomycinen.

Weiterhin bevorzugt ist hierbei, dass die stickstoffhaltige Verbindung ausgesucht ist aus der Gruppe a) der Polyamine, wobei die Polyamine vorzugsweise ausgesucht sind aus der Gruppe umfassend unverzweigte Polyamine mit 2 bis 6 Aminogruppen, insbesondere Spermidin, b) der polaren, basischen und sauren Aminosäuren, insbesondere Lysin, Arginin, Histidin, Glutaminsäure, oder c) der heterocyclischen Verbindungen, die ausgesucht sind aus der Gruppe Imidazol und 2,3-Dimethylpyrazin, Pyrimidin und Guanin.

Ein weiterer Gegenstand der Erfindung ist ein differentielles Analyseverfahren zum Nachweis einer oder mehrerer Spezies Nukleinsäuren in einer Probe, umfassend die Schritte: a) Bereitstellen einer Probenzubereitung, die zumindest eine erste Spezies Nukleinsäure und eine zweite Spezies Nukleinsäure enthält und wobei die erste Spezies Nukleinsäure von der zweiten Spezies Nukleinsäure unterschiedlich ist, wobei die erste Spezies Nukleinsäure mit dem Verfahren gemäß einem der obigen Ausführungsformen maskiert wurde und b) Verwendung der Probenzubereitung bzw. des Lysates in einer zum Nachweis der zweiten Nukleinsäure geeigneten Reaktion oder Reaktionssequenz.

Bevorzugt ist hierbei, dass Schritt b) ohne weitere Verfahrensschritte zur Reduzierung der Anzahl der in der Probenzubereitung enthaltenen Spezies von Nukleinsäuren und/oder zur Entfernung bzw. Inaktivierung von den Abbau von Nukleinsäuren bewirkenden Stoffen aus der Probenzubereitung durchgeführt werden.

Weiterhin bevorzugt ist hierbei, dass die Probenzubereitung bzw. das Lysat direkt ohne weiteren Aufarbeitungsschritt in der Reaktion bzw. Reaktionssequenz zum Nachweis der zweiten Nukleinsäurespezies verwendet wird.

Weiterhin bevorzugt ist hierbei, dass die zum Nachweis der zumindest zweiten Nukleinsäure geeignete Reaktion oder Reaktionssequenz ausgesucht ist aus der Gruppe der Nukleinsäure-Bindereaktionen, insbesondere der Nukleinsäure-Hybridisierungen, insbesondere Northern-, Southern-Blotting oder auch Hybridisierung von Oligonukleotiden und Sonden, der Gruppe der enzymatischen Modifikationen bzw. Polymerisationen von Nukleinsäuren, insbesondere Sequenzierungsreaktionen, in-vitro Transkription, Restriktionsendonuklease-spaltungen" der Gruppe der Amplifikationsreaktionen, insbesondere PCR (Polymerase-Kettenreaktion), Real-time-PCR, RT-PCR (Reverse Transkription Polymerase-Kettenreaktion), Real-time-RT-PCR, NASBA (Nucleic Acid Sequence Based Amplification), 3SR (Sequence Sustained Self Replication), 2 SR, TMA (Transcription Mediated Amplification), MDA (Multiple Displacement Amplification), Rolling Circle Amplification und Rolling Transcription Amplification.

Weiterhin bevorzugt ist hierbei, dass die in Schritt b) zum Nachweis der zweiten Spezies Nukleinsäure eingesetzte Reaktion bzw. Reaktionssequenz eine PCR oder RT-PCR ist, vorzugsweise eine Real-time-PCR oder Real-time-RT-PCR.

Weiterhin bevorzugt ist hierbei, dass in der Probenzubereitung bzw. in dem Lysat die erste Spezies Nukleinsäure gDNA und die zweite Spezies Nukleinsäure RNA ist, und in Schritt b) die Reaktion bzw. Reaktionssequenz RT-PCR ist, vorzugsweise Real-Time-RT-PCR.

Weiterhin bevorzugt ist hierbei, dass die stickstoffhaltige Verbindung ausgesucht ist aus der Gruppe der Polyamine, Heterocyclen, Aminosäuren, Carbonsäureamide und Ammoniumverbindungen, wie in den obigen Ausführungsformen definiert, und vorzugsweise ausgesucht ist aus der Gruppe bestehend aus Spermidin, Ammoniumsulfat, Ammoniumhydrogenphosphat, Glycin, 2,3-Dimethylpyrazin, Benzimidazol, Imidazol, Arginin, Histidin, und Harnstoff.

Weiterhin bevorzugt ist hierbei, dass in der Probenzubereitung bzw. in dem Lysat die erste Spezies Nukleinsäure RNA und die zweite Spezies Nukleinsäure DNA vorzugsweise gDNA, ist, und in Schritt b) die Reaktion bzw. Reaktionssequenz PCR ist, vorzugsweise Real-Time-PCR.

Weiterhin bevorzugt ist hierbei, dass die stickstoffhaltige Verbindung ausgesucht ist aus der Gruppe der Heterocyclen, Aminosäuren und Ammoniumverbindungen, wie in den den obigen Ausführungsformen definiert, und vorzugsweise ausgesucht ist aus der Gruppe bestehend aus Prolin, Indazol und Ammoniumsulfat.

In den Figuren zeigen:
- Figur 1:: Ein Diagramm, in dem die Delta-Ct-Werte aus verschiedenen Proben gegenübergestellt sind, die mit und ohne stickstoffhaltige Verbindung (Additive) lysiert wurden.
- Figur 2:: Ein Diagramm zur Verdeutlichung der Steigerung der RNA-Ausbeute, die mit einer Ausführungsform des erfindungsgemäßen Lyseverfahrens ermöglicht wird.
- Figur 3:: Ebenfalls ein Diagramm zur Verdeutlichung der Steigerung der RNA- Ausbeute, die mit einer Ausführungsform des erfindungsgemäßen Lyseverfahren ermöglicht wird.
- Figur 4:: Ein Diagramm, das die stabilisierende Wirkung verschiedener Additve einer Ausführungsform des erfindungsgemäßen Stabilisierungsverfahrens aufzeigt.
- Figur 5:: Ein Diagramm, das die stabilisierende Wirkung verschiedener Additive bei Verwendung unterschiedlicher Konzentrationen in unterschiedlichen Zellkulturen in einer Ausführungsform des erfindungsgemäßen Stabilisierungsverfahrens zeigt.
- Figur 6:: Das Ergebnis einer Gel-Analyse verschiedener Zelllysate.
- Figur 7:: Ein Diagramm, in dem die Abhängigkeit der Delta-Ct-Werte von der Inkubationszeit gezeigt ist.
- Figur 8:: Ein Diagramm, in dem die Abhängigkeit der Delta-Ct-Werte von der Zellzahl gezeigt ist.
- Figur 9:: Ein Diagramm, dass die verbesserte Zugänglichkeit von gDNA durch Verwendung verschiedener Additive in Lysaten die gemäß einer Ausfühungsform des erfindungsgemäßen Verfahrens zur Verminderung inhibierender Effekte erhalten werden, aufzeigt.
- Figur 10:: Ein Diagramm, dass die verbesserte Zugänglichkeit von gDNA für DNase I durch Verwendung verschiedener Additive in Lysaten die gemäß einer Ausführungsform des erfindungsgemäßen Verfahrens zur Verminderung inhibierender Effekte erhalten werden, aufzeigt.

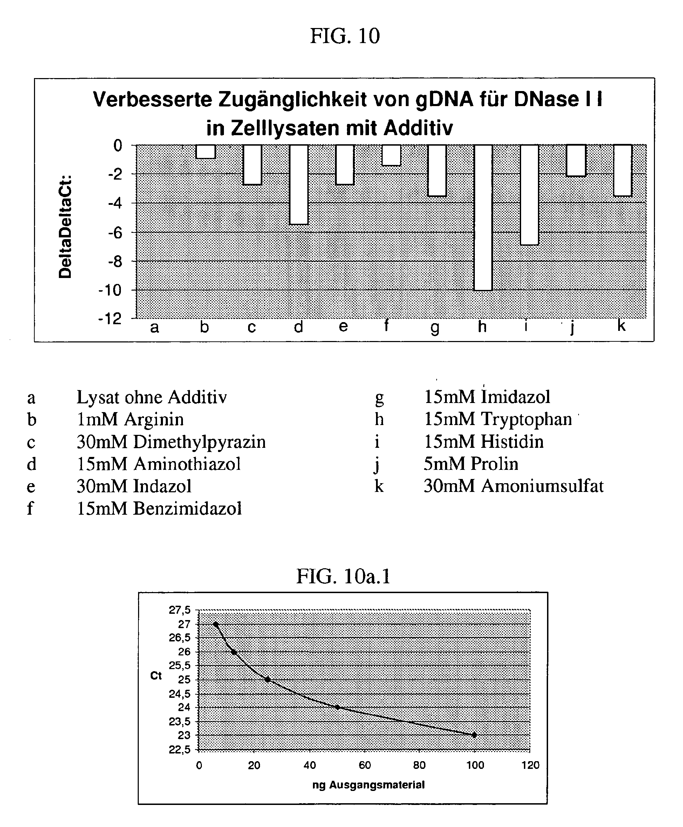

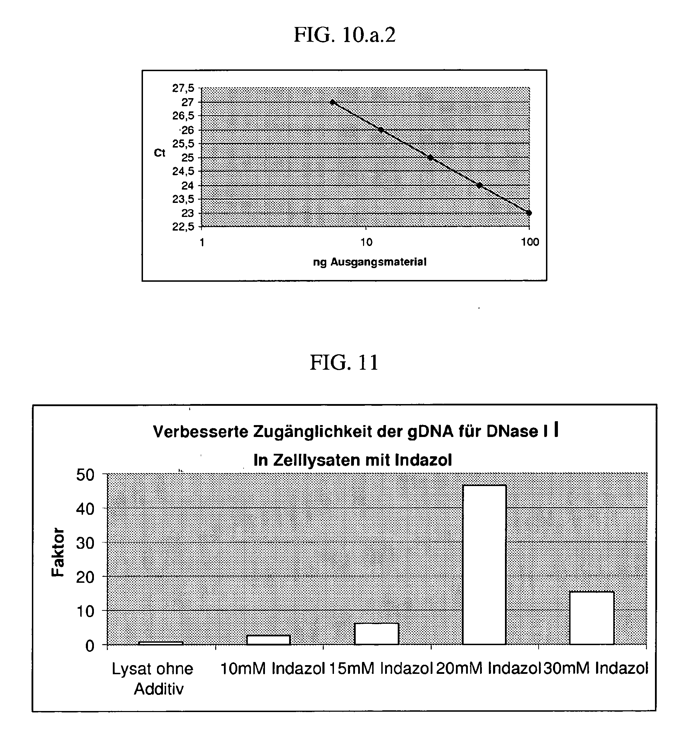

- Figur 10.a.1: und
- Figur 10.a.2:: Den Zusammenhang zwischen Ct-Wert und Faktor anhand idealisierter, hypothetischer Werte.
- Figur 11:: Ein Diagramm, das die Konzentrationsabhängigkeit des gemessenen Delta-Ct- Wertes zeigt.

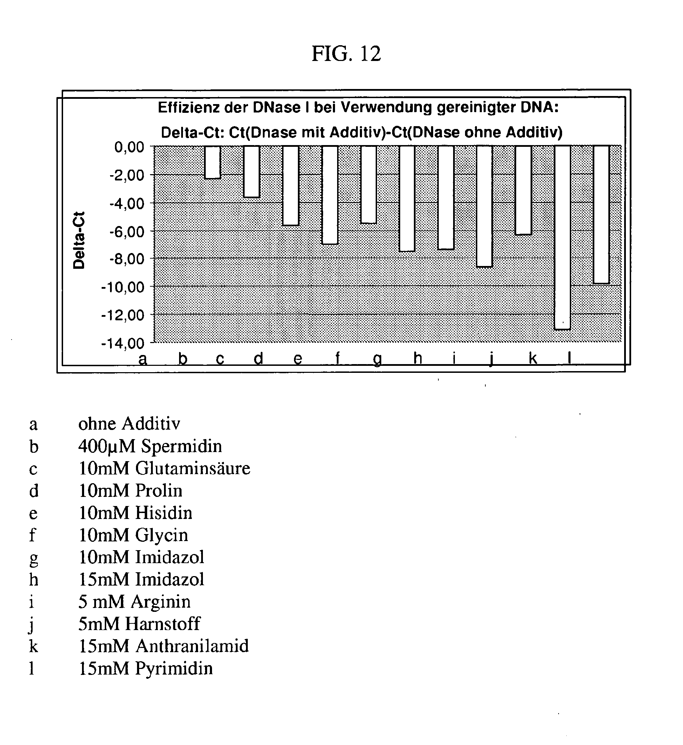

- Figur 12:: Ein Diagramm, das die gemessenen Delta-Ct-Werte bei Verwendung von gereinigter DNA in einer Ausführungsform des erfindungsgemäßen Verfahrens zur Verminderung inhibitorischer Effekte zeigt.

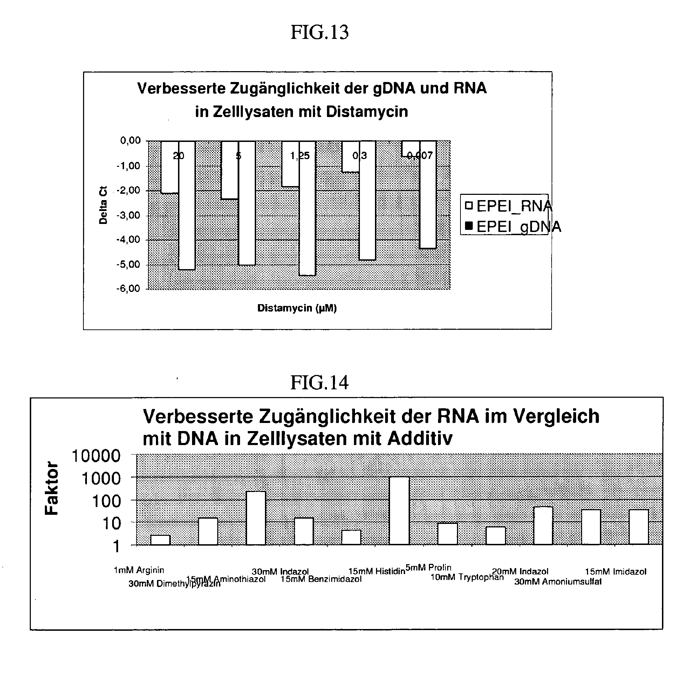

- Figur 13:: Ein Diagramm, das die erfindungsgemäß verbesserte Zugänglichkeit von gDNA und RNA in Lysaten in Gegenwart von Distamycin D zeigt.
- Figur 14:: Ein Diagramm, das die erfindungsgemäß verbesserte Zugänglichkeit von RNA im Vergleich zu DNA in Zelllysaten mit unterschiedlichen Additiven zeigt.

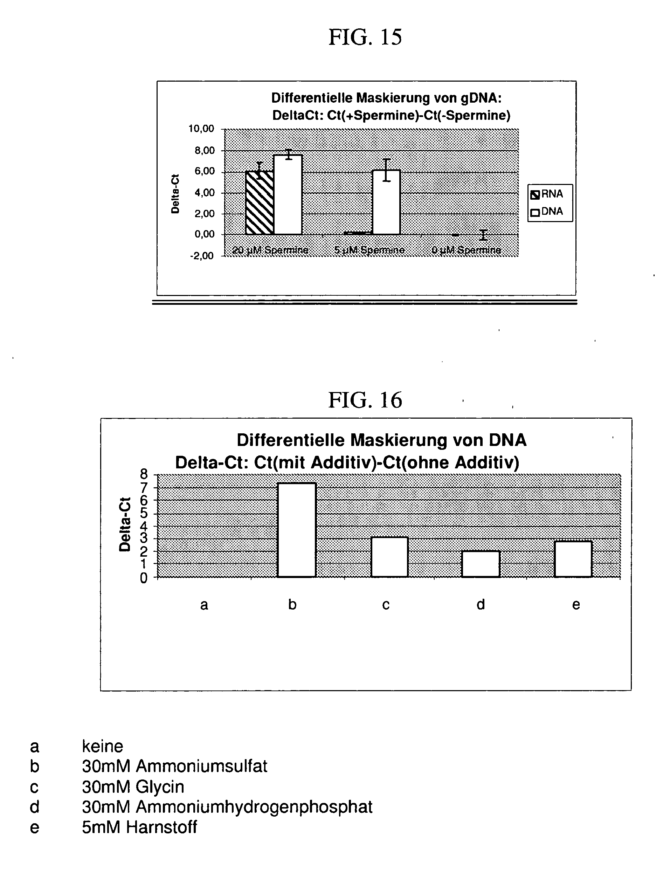

- Figur 15:: Ein Diagramm, das den Effekt der erfindungsgemäßen differentiellen Maskierung von gDNA gegenüber RNA in Zelllysaten mit verschiedenen Konzentrationen Spermin anhand der gemesssen Delta-Ct-Werte zeigt.
- Figur 16:: Ein Diagramm, das den Effekt der erfindungsgemäßen differentiellen Maskierung in gDNA in Zelllysaten unter Verwendung verschiedener Additive zeigt.

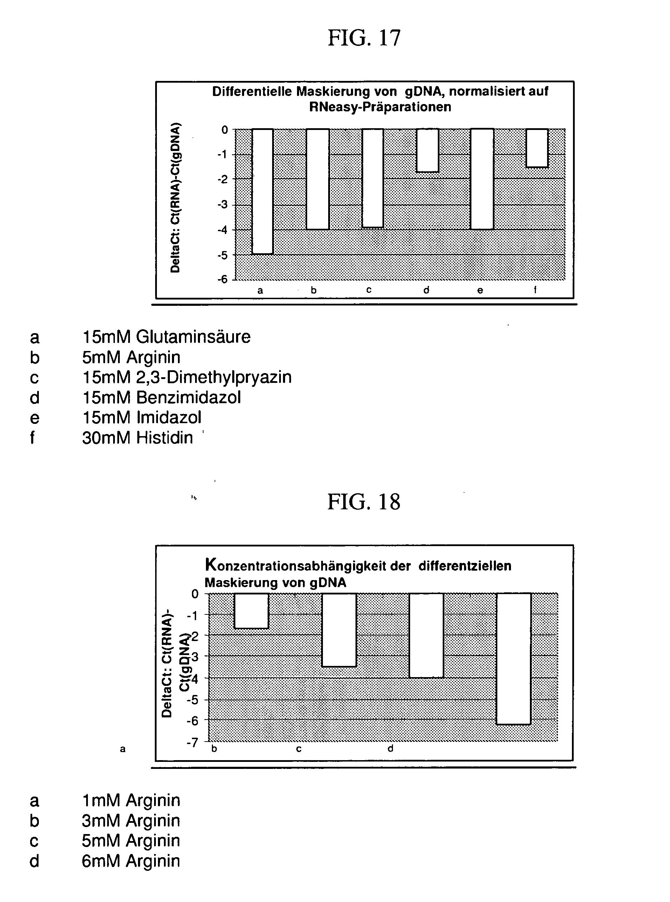

- Figur 17:: Ein Diagramm, das den Effekt der erfindungsgemäßen differentiellen Maskierung von gDNA, normalisiert auf RNeasy-Präparationen, zeigt.
- Figur 18:: Ein Diagramm, das die Konzentrationsabhängigkeit des Effekts der erfindungsgemäßen Maskierung von gDNA in Zelllysaten zeigt.

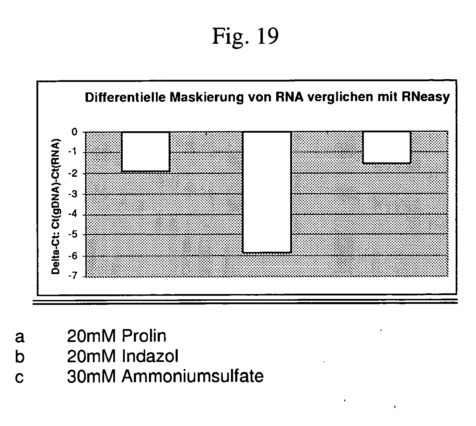

- Figur 19:: Ein Diagramm, das den Effekt der erfindungsgemäßen Maskierung von RNA zeigt.

### Beispiele

In den folgenden Beispielen werden die stickstoffhaltigen Verbindungen, wie sie in der vorliegenden Erfindung in den Ansprüchen definiert sind, als Additiv bezeichnet. Insoweit nicht im Folgenden anders beschrieben, wurden in den Beispielen die folgenden Materialien verwendet:

### Stammlösungen:

(1) 500 mM EGTA, pH 8
(2) 500 mM EDTA, pH 8
(3) TE-Buffer, 10 mM Tris-HCl, 1 mM EDTA, pH 7,5
(4) EPE: 0,06 Vol-% Nonidet P40, 1 mM EDTA, 1 mM EGTA, 0,1 Vol-% Polyethylenglycol MG 6000 (PEG)

### Geräte:

(1) ABI Prism 7700 (ABI, Foster City, CA, USA) (Real-time-RT-PCR)
(2) PCR-ThermoCycler (Biometra GmbH, Göttingen, Deutschland)
(3) Spektrophotometer zur Bestimmung der Konzentration von DNA oder RNA (Beckman DU 7400, Beckman Coulter Inc., Fullerton, CA, USA)
(4) Agilent Bioanalyzer (Agilent, USA) (denaturierende Gelanalyse)

### Zellkulturen:

(1) HeLa Zellen
(2) 293 Zellen
(3) HUH 7 Zellen
(4) NIN3T3 Zellen
(5) HepG2 Zellen
(6) MCF7 Zellen

### Medien:

(1) D-MEM (BRL; USA) teilweise mit den Additiven 10% FCS, 1% Pen/Strept und/oder 1% nicht essentielle Aminosäuren für die Zellen Hela, HUH7, 293, NIH3T3
(2) RPMI 1640 (BRL; USA) teilweise mit den Additiven 10% FCS, 1% Glutamin und/oder 1% PEN/Strept für HepG2 Zellen
(3) RPMI 1640 (BRL; USA) teilweise mit den Additiven 10% FCS, 1% Sodium Pyruvat, 1% Glutamin, 1% Pen/Strept, 1% Non essential AS und/oder 0,25% Bovines Insulin für MCF7 Zellen

### Kits und Enzyme:

(1) RNeasy®: RNA Präparationsmethode der QIAGEN GmbH, Hilden, Deutschland
(2) RNeasy 96®: RNA Präparationsmethode der QIAGEN GmbH, Hilden, Deutschland
(3) Omniscript® RT-Kit: Kit für die Reverse-Transkriptase Reaktion von RNA (QIAGEN GmbH, Hilden, Deutschland)
(4) Sensiscript® RT-Kit: Kit für die Reverse-Transkriptase Reaktion von RNA (QIAGEN GmbH, Hilden, Deutschland)
(5) QuantiTect® SybrGreen PCR Kit: Kit zur Real-time PCR mit SybrGreen (QIAGEN GmbH, Hilden, Deutschland)
(6) QuantiTect® Probe PCR Kit: Kit zur Real-time PCR mit markierten Sonden (QIAGEN GmbH, Hilden, Deutschland)
(7) DNase I: (QIAGEN GmbH, Hilden, Deutschland)

### Transkripte und Genom-Loci, die in Real-time RT-PCR bzw. PCR getestet wurden:

(1) ß-Aktin: als Transkript und Genomlocus
(2) GAPDH als Transkript und Genomlocus
(3) ß-Tubulin als Transkript

### DNase I Verdau (Beispiel 10 und 11):

In den Beispielen 10 und 11 wurde der DNase I Verdau wie folgt durchgeführt: Der DNase I Verdau wurde der Lysate wurde vor der Real-time PCR durchgeführt. Dazu wurden 2 µl der Lysate in einem 20 µl Volumen in einem geeigneten Puffer, der 150 µM CaCl₂, 4 mM MgCl₂ und 50 mM Tris pH 8,4 enthielt, mit 2 Einheiten (Units) DNase I verdaut. Die Reaktion wurde abgestoppt durch Zugabe einer EDTA/EGTA-Lösung. Daraufhin wurden 2 µl der Reaktionslösung in die Real-time PCR gegeben, um die Menge der verbliebenen gDNA zu messen.

### I. Lyse

### Beispiel 1

### Lyse von Zellen in Gegenwart verschiedener Additive

Mit diesem Versuch wird demonstriert, dass durch die Zugabe eines Additivs die Lyse von Zellen effizienter gestaltet werden kann. Dazu wurden in einem ersten Versuch Zellen in einem Lysepuffer A, der als Additiv Imidazol in einer Konzentration von 15 mM in H₂O enthielt, lysiert. In einem zweiten Versuch wurden Zellen in einem Lysepuffer B lysiert, der neben dem Additiv Imidazol (15 mM), Nonidet-P40, Polyethylenglycol, EGTA und EDTA enthält. Als Vergleichsbeispiel wurden Zellen in einem Lysepuffer C lysiert, der Nonidet-P40, Polyethylenglycol, EGTA und EDTA enthielt. Zur Bestimmung der Lyseeffizienz wurde in einer vergleichenden Real-time RT-PCR der CT-Wert der jeweiligen zellulären Transkripte bestimmt. Der Ct-Wert ist der PCR-Zyklus, bei dem das PCR-Signal zum ersten Mal detektiert wird, d.h. sichtbar wird. Der Delta-Ct-Wert berechnet sich in diesem Versuch aus der Differenz des Ct-Wertes nach Lyse ohne Additiv und dem Ct-Wert bei Lyse unter Verwendung des Additivs Imidazol. Ein negativer Delta-Ct Wert weist auf eine verbesserte Lyse gegenüber dem Vergleichssystem ohne Additiv hin. Ein positiver Delta-Ct-Wert weist auf eine geringere Effizienz bei der Lyse gegenüber dem Vergleichssystem ohne Additiv hin. *Durchführung:* 40000 293-Zellen wurden in einem geeigneten Medium für 3 Tage inkubiert. In einem ersten Versuch wurden die Zellen in einem Lysepuffer, der Nonidet-P40, Polyethylenglycol, EGTA und EDTA aber kein Additiv enthält, lysiert. In einem zweiten Versuch wurden die Zellen in einem Lysepuffer lysiert, der Nonidet-P40, Polyethylenglycol, EGTA, EDTA sowie 15 mM Imidazol als Additiv enthält. In einem dritten Versuch wurden die Zellen in einem Lysepuffer lysiert, der Imidazol in H₂O gelöst enthielt. 2 µl der jeweils so gewonnenen RNA wurde mit Omniscript®, erhältlich von der Firma QIAGEN, Hilden, Deutschland, in einer Standard Reversen Transkriptase-Reaktion umgesetzt. Nach Abschluss dieser Reaktion wurden 2 µl der RT-Reaktionslösung in Real-time PCRs zum Nachweis eines bestimmten Transkriptes überführt.

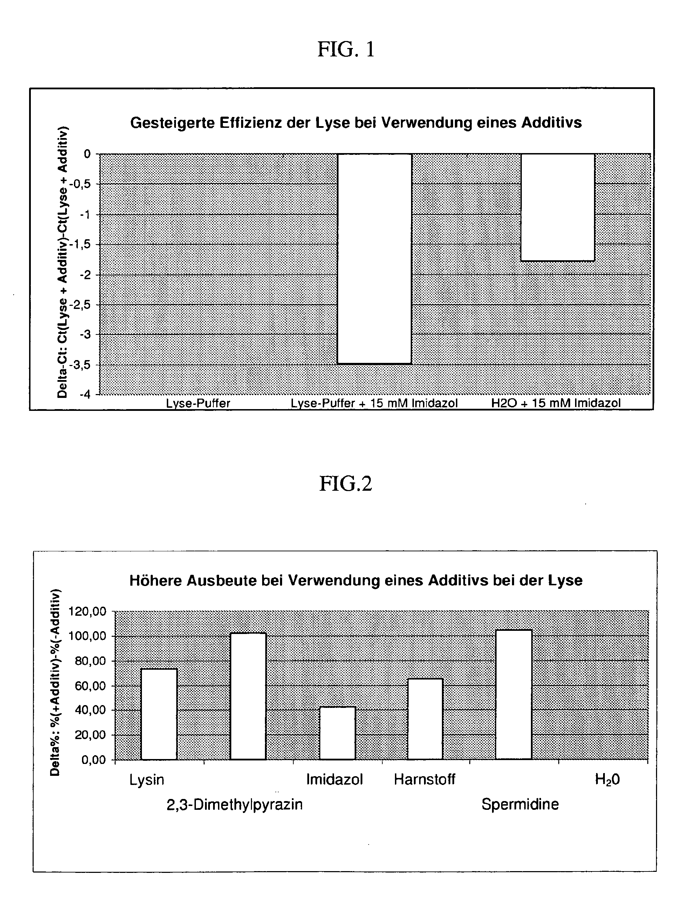

*Ergebnis:* Figur 1 zeigt ein Diagramm, in dem die jeweils ermittelten Delta-Ct-Werte der einzelnen Versuche gegenübergestellt sind. Diese Ergebnisse zeigen, dass zelluläre RNA in Gegenwart der jeweiligen Lysepuffer, die Imidazol als Additiv enthielten Delta-Ct-Werte zeigten, die ca. 1,5 bis 3,5 Cts niedriger (d.h. größere negative Werte) waren als bei der Lyse unter Verwendung des herkömmlichen Lysepuffers ohne Imidazol. Dies zeigt einen deutlichen Verbesserungsgrad bei der Lyse auf.

### Beispiel 2

Lyse von Zellen in H₂O unter Verwendung verschiedener stickstoffhaltiger Verbindungen als Additive

In diesem Beispiel wird die RNA-Ausbeute bei der Lyse in H₂O in Gegenwart und Abwesenheit verschiedener Additive verglichen [Lysin (1 mM), 2,3-Dimethylpyrazin (15 mM), Imidazol (15 mM), Harnstoff (5 mM), Spermidin (1 mM)]. Eine erhöhte Ausbeute an RNA weist auf eine effizientere Lyse hin. Um eventuelle sekundäre Effekte auszuschließen, wurden die RNAs der jeweiligen Lysate im Anschluß an die Lyse über RNeasy96®, erhältlich von der Firma QIAGEN, Hilden, Deutschland, gereinigt und durch densitometrische Messung der Konzentration bei 260 nm quantifiziert.

*Durchführung:* 20000 MCF7-Zellen wurden in einem geeigneten Medium in einer 96well Multititer Kulturschale inkubiert. Die Zellen werden in H₂O oder alternativ in H₂O, das unterschiedliche Additive enthält, lysiert. Das Lysat wird über RNeasy 96 (erhältlich von QIAGEN GmbH, Hilden, Germany) gereinigt und densitometrisch bei einer Wellenlänge von 260 nm vermessen. Die Ausbeute an RNA bei der Lyse mit H₂O ohne Additiv, wurde auf einen Wert von 100% normalisiert.

*Ergebnis:* Figur 2 zeigt ein Diagramm, in dem die RNA-Ausbeuten unter Verwendung verschiedener Additive verglichen werden. Aus diesen Ergebnissen geht hervor, dass die durch die Verwendung des jeweiligen Additivs bessere RNA-Ausbeuten erzielt werden, was auf eine effizientere Lyse bei Verwendung der Additve hinweist.

### Beispiel 3

### Lyse von Zellen unter Verwendung von Lysepuffern und verschiedenen Additiven

In diesem Beispiel wird die RNA-Ausbeute bei der Lyse unter Verwendung von Lysepuffern in Gegenwart und Abwesenheit verschiedener Additive verglichen. Eine erhöhte Ausbeute an RNA weist auf eine effizientere Lyse hin. Um eventuelle sekundäre Effekte auszuschließen, wurden die RNAs der jeweiligen Lysate im Anschluss an die Lyse über RNeasy96 erhältlich von der Firma QIAGEN, Hilden, Deutschland, gereinigt und durch densitometrische Messung der Konzentration bei 260 nm quantifiziert.

*Durchführung:* 20000 MCF7-Zellen werden in einem geeigneten Medium in einer 96well Multititer Kulturschale inkubiert. Die Zellen werden entweder in einem Lysepuffer, der Nonidet-P40, Polyethylenglycol, EGTA und EDTA enthält, oder alternativ in einem Lysepuffer, der neben den genannten Reagenzien zusätzlich ein Additiv [Arginin (5 mM), Lysin (1mM), 2,3-Dimethylpyrazin (15 mM), Imidazol (15 mM), Spermidin (1 mM), Pyrimidin (15 mM), Guanin (15 mM)] enthält, lysiert. Das Lysat wird über RNeasy 96 (QIAGEN GmbH, Hilden, Deutschland) gereinigt und anschließend bei einer Wellenlänge von 260 nm densitometrisch vermessen. Die RNA-Ausbeute bei der Lyse mit dem Lysepuffer ohne Additiv wurde auf einen Wert von 100% normalisiert.

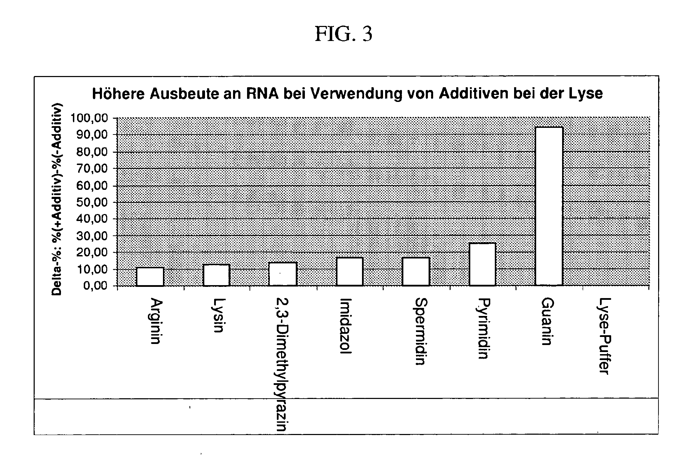

*Ergebnis:* Figur 3 zeigt ein Diagramm, in dem die RNA-Ausbeuten der verschiedenen Versuche gegenübergestellt sind. Aus diesem Diagramm geht hervor, dass bei allen verwendeten Additiven eine erhöhte Ausbeute erreicht werden konnte. Eine besonders hohe Ausbeutesteigerung (ca. 94 %) konnte hier bei Verwendung von Guanin als Additiv verwendet werden.

### II. Stabilisierung

### Beispiel 4

### Stabilisierung von zellulärer RNA in Zelllysaten durch verschiedene Additive

Zellen wurden in einem Lysepuffer mit bzw. ohne Additiv lysiert. Um den Grad der RNA-Stabilisierung zu bestimmen, wurde in einer vergleichenden Real-time RT-PCR der Ct-Wert eines zellulären Transkriptes bestimmt. Der Ct-Wert gibt den PCR-Zyklus an, bei dem das PCR-Signal zum ersten Mal detektierbar ist, also sichtbar wird. Der Delta-Ct-Wert berechnet sich in diesem Experiment aus der Differenz des Ct-Wertes der Proben der Lyse mit Additiv und des Ct-Wertes der Probe Lyse ohne Additiv. Je niedriger, d.h. je höher der Betrag des negativen Delta-Ct Wertes ist, desto größer ist der Stabilisierungseffekt der durch die Zugabe des Additivs zu dem Lysat bewirkt wird.

*Durchführung:* 40000 HepG2-Zellen wurden in einem geeigneten Medium für 3 Tage inkubiert. In einem ersten Versuch wurden die Zellen in einem Lysepuffer, der Nonidet-P40, Polyethylenglycol, EGTA und EDTA enthält, lysiert. In weiteren Versuchen wurden die Zellen in einem Lysepuffer, der neben den oben genannten Lysereagenzien als Additiv Imidazol (15 mM), Proline (15 mM), Glutaminsäure (15 mM), Histidine (15 mM), Arginine (5 mM), Tyrptophan (15 mM), Glycin (15 mM), Pyrimidin (15 mM), Guanin (15 mM), Cytosin (30 mM), Betain (100 mM), Ectoin (200 mM) 2,3-Dimethylpyrazin (30 mM), 2-Aminothiazol (15 mM), Indazol (15 mM), Benzimidazol (15 mM), Harnstoff (5 mM) oder Ammoniumsulfat (30 mM) enthält. 2 µl des jeweils so gewonnenen Lysats wurden anschließend mit Omniscript® (QIAGEN GmbH, Hilden, Deutschland) in einer Standard Reversen Transkriptase-Reaktion umgesetzt. Nach Abschluss der Reversen Transkriptase Reaktion wurde 2 µl der RT- Reaktionslösung in eine Real-time PCRs überführt.

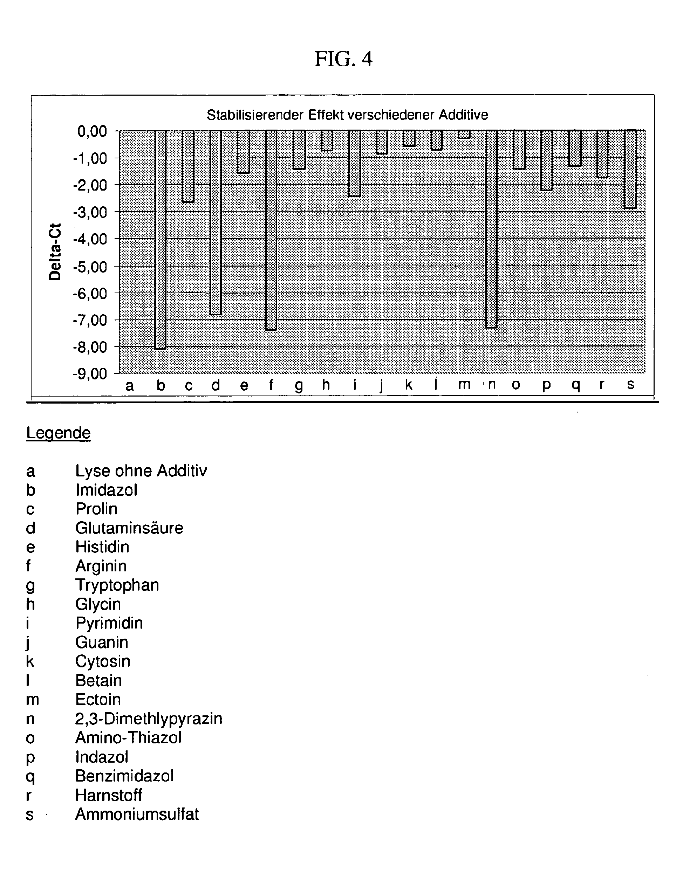

*Ergebnis:* Figur 4 zeigt ein Diagramm, in dem die Stabilisierung von zellulärer RNA durch die verschiedenen Additive anhand des Delta-Ct-Wertes verdeutlicht wird. Aus dem Diagramm geht hervor, dass in Gegenwart verschiedener Konzentrationen des Additives Delta-Ct Werte im Bereich von 0,5-8 Cts erreicht werden. Dies verdeutlicht, dass im Vergleich zur Verwendung von Lysepuffern, denen kein Additiv zugesetzt wurde, die Stabilität der RNA in einem Lysepuffer mit Additiv deutlich größer ist.

### Beispiel 5

### Stabilisierung zellulärer RNA in Zelllysaten in Gegenwart von Additiven

Zellen wurden in einem Lysepuffer mit bzw. ohne Additiv lysiert. Zur Bestimmung des Grades der RNA-Stabilisierung wurde in einer vergleichenden Real-time RT-PCR der Ct-Wert eines zellulären Transkriptes bestimmt. Der Ct-Wert gibt den PCR-Zyklus an, bei dem das PCR-Signal zum ersten Mal detektierbar ist, d.h. sichtbar wird. Der Delta-Ct-Wert berechnet sich in diesem Experiment aus der Differenz des Ct-Wertes nach Lyse ohne Additiv und des Ct-Wertes nach Lyse mit Additiv. Je niedriger, d.h. je höher der Betrag des negativen Delta-Ct-Wertes ist, umso größer ist der Stabilisierung der RNA in Gegenwart des jeweiligen Additivs.

*Durchführung:* 40000 HeLa-, HUH7 und 293-Zellen wurden in einem geeigneten Medium über einen Zeitraum von 3 Tagen inkubiert. In einem ersten Versuch wurden die Zellen in einem Lysepuffer, der Nonidet-P40, Polyethylenglycol, EGTA und EDTA enthält, lysiert. In weiteren Versuchen wurden die Zellen in einem Lysepuffer, der neben den oben genannten Lysereagenzien auch noch die Additive Imidazol, Lysin oder Spermin enthielt. 2 µl der jeweils so gewonnenen Lylats wurde mit Omniscript® (QIAGEN GmbH, Hilden, Deutschland) in einer Standard Reverse Transkriptase-Reaktion umgesetzt. Nach Abschluss der Reaktion wurden 2 µl der RT-Reaktionslösung in Real-time PCRs überführt.

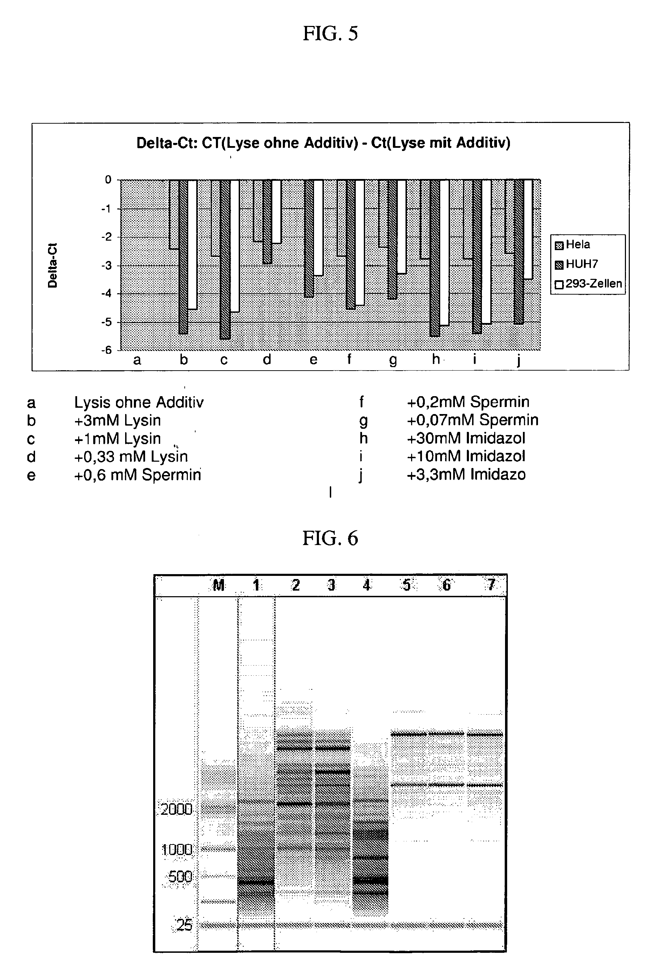

*Ergebnis:* Figur 5 zeigt ein Diagramm, in der die Delta-Ct-Werte der verschiedenen Proben verglichen wurden. Es zeigt sich, dass die Proben der zellulären RNA der verschiedenen Zellkulturen in Gegenwart verschiedener Konzentrationen der Additive in Gegensatz der Proben, die unter Verwendung des Standard-Lysepuffers ohne Additive erhalten wurden, negative Ct-Werte im Bereich von ca. -2 bis -5,5 aufwiesen. Dies deutet auf eine deutlich verbesserte Stabilität der RNA in den Additive enthaltenden Proben hin.

### Beispiel 6

### Stabilisierung zellulärer RNA in Zelllysaten in Gegenwart von Additiven

Zellen wurden in einem Lysepuffer mit bzw. ohne potentiell stabilisierendes Additiv lysiert. Zur Bestimmung der RNA-Stabilisierung wurde die RNA auf einem denaturierenden Gel analysiert. Da die RNA aber komplexiert ist mit Zellulärer Substanz, muss diese zunächst über Rneasy® (QIAGEN GmbH, Hilden, Deutschland) gereinigt werden, damit eine Analyse auf einem denaturierenden Gel möglich wird. Die Integrität der RNA kann anhand der 18S und 28S rRNA Banden bestimmt werden.

*Durchführung:* 40000 HepG2-Zellen wurden in einem geeigneten Medium über einen Zeitraum von 3 Tagen inkubiert. Die Zellen wurden jeweils in den folgenden Lysepuffern lysiert:
1: Spur 1: Ein Lysepuffer, der Nonidet-P40, Polyethylenglycol (PEG), EGTA und EDTA enthält.
2: Spur 2: Wie 1, zusätzlich enthaltend weitere 2mM EGTA
3: Spur 3: Wie 1, zusätzlich enthaltend weitere 2mM EDTA
4: Spur 4: Ein Lysepuffer enthaltend Nonidet-P40, EGTA und EDTA
5: Spur 5: Wie 1, zusätzlich 1mM L-Lysin,
6: Spur 6: Wie 1, zusätzlich 0,2mM Spermin und
7. Spur 7: Wie 1, zusätzlich 10mM Imdiazol.

Nach Lyse in den genannten Lysepuffern, wurde die im Lysat enthaltene RNA über RNeasy gereinigt und die Degradierung auf einem denaturierenden Gel (Agilent BioAnalyzer) überprüft.

*Ergebnis:* Figur 6 zeigt das Ergebnis der Gel-Analyse. Deutlich erkennbar ist die Degradierung zellulärer RNA im einfachen Lysepuffer mit oder ohne PEG (Spuren 1 und 4). Im Gegensatz dazu zeigte die RNA in Gegenwart der Additive Lysin, Spermin, oder Imidazol (Spuren 5, 6 und 7) keine Degradierungsanzeichen. Eine verbesserte, aber nicht ausreichende Stabilisierung wurde in Gegenwart erhöhter Konzentrationen der Nukleaseinhibitoren EGTA bzw. EDTA gefunden (Spuren 2 und 3).

### Beispiel 7

### Stabilisierung zellulärer RNA aus Zelllysaten durch Additive bei Raumtemperatur

Zellen wurden in einem mit einem Lysepuffer, der Imidazol enthielt, lysiert. Zu Vergleichszwecken wurde zelluläre RNA über RNeasy gereinigt. Die Lysate wurden keinem zusätzlichen Aufreinigungschritt unterzogen und enthielten daher noch sämtliche RNase. Durch die mittels des Additivs Imidazol und gegebenenfalls weiterer in der Probe enthaltener zellulärer Substanzen bewirkte Stabilisierung der RNA sollte die Degradierung der RNA in den Lysaten vermindern oder unterbinden, selbst bei Inkubation bei Raumtemperatur.
Zur Bestimmung der RNA-Stabilisierung wurde in einer vergleichende Real-time RT-PCR der Ct-Wert eines zellulären Transkriptes bestimmt. Der Ct-Wert ist der PCR-Zyklus, bei dem das PCR-Signal zum ersten Mal detektierbar, d.h. sichtbar wird. Der Delta-Ct-Wert berechnet sich in den vorliegenden Beispiel aus der Differenz Ct-Wert (t=0), wobei "t" Zeit bedeutet, und dem Ct-Wert (t=x). Bleibt der gemessene Delta-Ct-Wert über den gemessenen Zeitraum bei einem Wert um 0, so deutet dies auf einen hohen Grad der Stabilisierung hin, da dies darauf hinweist, dass über den vermessenen Zeitraum keine signifikante Degradierung der RNA eintritt.

*Durchführung:* 16000 293-Zellen werden in einem geeigneten Medium über einen Zeitraum von 3 Tagen inkubiert. Zum einen wurden die Zellen in einem Lysepuffer, der Nonidet-P40, Polyethylenglycol, EGTA und EDTA sowie Imidazol enthält, lysiert, zum anderen wurde die RNA über RNeasy® (QIAGEN, Hilden, Deutschland) präpariert. Die Lysate bzw. Eluate wurden bei Raumtemperatur für maximal 2 h inkubiert. Zu unterschiedlichen Zeitpunkten wurden jeweils 2 µl der inkubierten RNAs mit Omniscript® (QIAGEN, Hilden, Deutschland) in einer Standard Reverse Transkriptase-Reaktion umgesetzt. Nach Abschluss der RT-Reaktion wurden 2 µl der Reaktionslösung in eine Real-time PCR überführt.

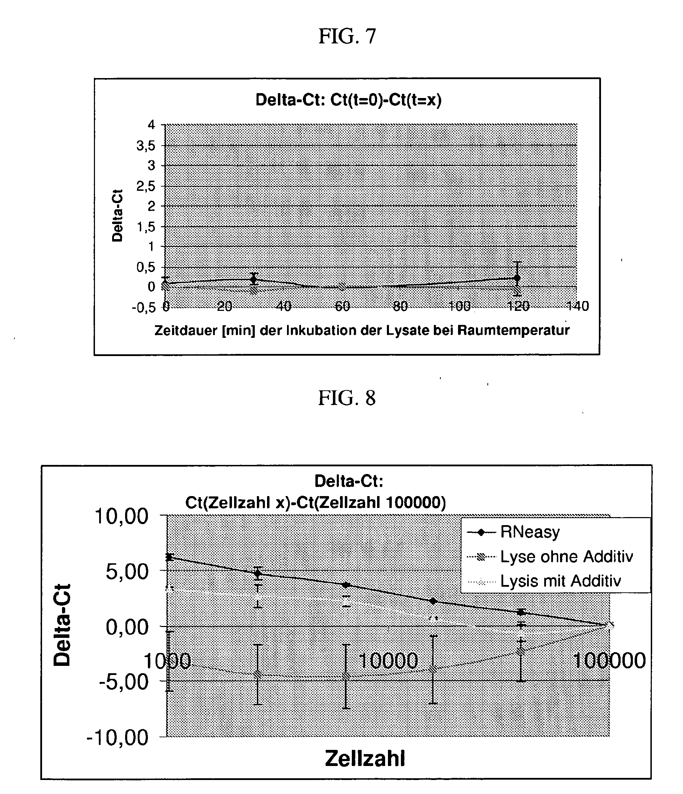

*Ergebnis:* Figur 7 zeigt ein Diagramm, in der die Abhängigkeit der Delta-Ct-Werte von der Inkubationszeit aufgezeigt ist. Sowohl Delta-Ct-Wert der Proben, die direkt aus den Lysaten vermessen wurden als auch der Delta-Ct-Wert der mittels RNeasy® (QIAGEN GmbH, Hilden, Deutschland) gereinigten RNAs blieben ungefähr bei dem Wert 0. Dies zeigte, dass die RNAs der Lysate eine vergleichbare Stabilität wie die durch RNeasy® (QIAGEN GmbH, Hilden, Deutschland) gereinigte RNA-Präparationen aufweisen.

### Beispiel 8

### Stabilisierung von zellulärer RNA bei verschiedenen Zellzahlen

Während die Nukleinsäuren, die aus Proben mit kleinen Zellzahlen gewonnen wurden, alleine durch die zellulären Substanzen stabilisiert werden können, bedarf es bei Proben, die aus großen Zellzahlen gewonnen wurden, noch zusätzlicher Agenzien, die eine ggf. stattfindende Stabilisierung von Nukleinsäuren durch zelluläre Substanzen unterstützen. In Versuchen, in denen Proben aus Kulturen mit unterschiedlichen Zellzahlen eingesetzt werden, soll die Stabilität von RNA nach Lyse mit bzw. ohne stabilisierendes Additiv bestimmt werden.

*Durchführung:* 1024-100000 Zellen wurden in 24 well-Schalen in einem geeigneten Medium ausgesät und für 3 Tage inkubiert. In einem Experiment werden die so erhaltenen Zellen in einem Lysepuffer lysiert, der Nonidet-P40, Polyethylenglycol, EGTA und EDTA sowie Imidazol als stabilisierendes Additiv enthält. Durch die Zugabe des Imidazols soll eine ggf. bereits vorliegende Stabilisierung durch zelluläre Substanzen verbessert werden. In einem weiteren Experiment wurden die Zellen in einem Lysepuffer lysiert, der Nonidet-P40, Polyethylenglycol, EGTA und EDTA, aber kein Imidazol enthielt. In jedem der oben beschriebenen Experimente wurde die zelluläre RNA als Vergleich über RNeasy® (QIAGEN GmbH, Hilden, Germany) gereinigt. Jeweils 2 µl der Lysate bzw. Eluate wurden mit Omniscript® (QIAGEN GmbH, Hilden, Germany) in einer Standard Reverse Transkriptase-Reaktion umgesetzt. Nach Abschluss der RT-Reaktion wurden 2 µl der Reaktionslösung in eine Real-time PCR überführt.
Zur Bestimmung des Grades der RNA-Stabilisierung wurde in einer vergleichenden Real-time RT-PCR der Ct-Wert eines zellulären Transkriptes bestimmt. Der Ct-Wert ist der PCR-Zyklus, an dem das PCR-Signal zum ersten Mal detektierbar wird, d.h. sichtbar wird. Der Delta-Ct-Wert berechnet sich im vorliegenden Beispiel aus der Differenz des Ct-Wertes bei einer Zellzahl X und des Ct-Wertes bei der Zellzahl 100000. Wird ein hohes Maß an Stabilisierung erreicht, steigt der Delta-Ct mit abnehmender Zellzahl.

*Ergebnis:* Figur 8 zeigt ein Diagramm, in dem die Abhängigkeit des Delta-Ct-Wertes von der Zellzahl für Proben, die über RNeasy® (QIAGEN GmbH, Hilden, Deutschland) gereinigt wurden, von Proben aus Lysaten mit Imidazol als Additiv, sowie Proben ohne Additiv. Für die über RNeasy® (QIAGEN GmbH, Hilden, Deutschland) gereinigten Proben zeigt sich deutlich, dass sich der Delta-Ct-Wert über den ganzen Zellzahlbereich entsprechend der Zellzahl verhält, d.h. ein höherer Delta-Ct-Wert bei abnehmender Zellzahl beobachtet wird. Wurden die Zellen mit dem Lysepuffer lysiert, der auch Imidazol enthielt, konnten einen solchen den Zellzahlen entsprechenden Verlauf der Delta-Ct-Werte erst ab einer Zellzahl kleiner oder gleich 40000 beobachten. Wurden die Zellen jedoch mit einem Lysepuffer ohne Imidazol lysiert, konnte man der Zellzahl entsprechende Delta-Ct Werte erst unterhalb 6400 Zellen beobachten. Oberhalb von 6400 Zellen wurden höhere Delta-Ct- Werte gemessen, was auf eine deutliche Degradierung der RNA hinweist, wenn mehr als 6400 Zellen eingesetzt wurden. Dies zeigt, dass eine ausreichende Stabilisierung ohne Additiv allein durch zelluläre Substanzen wie Proteine unterhalb einer bestimmten Zellzahl möglich ist (hier 6400 Zellen). Oberhalb dieser Zellzahl müssen Additive zusätzlich die Stabilisierung unterstützen.

### III. Verminderung inhibitorischer Effekte

### Beispiel 9

### Nachweisbarkeit von gDNA aus Nukleoproteinkomplexen

Zellen wurden in einem Lysepuffer mit bzw. ohne Additiv lysiert. Eine Lysierung von Zellen kann unter den hier gewählten Lysebedingungen zur Bildung eines Nukleoproteinkomplexes führen, so dass gDNA nur unzureichend in einer aus einem Lysat gewonnen Probe nachweisbar ist. Zur Bestimmung der gDNA wurde in einer vergleichenden Real-time PCR der Ct-Wert bestimmt. Der Ct-Wert ist der PCR-Zyklus, an dem das PCR-Signal zum ersten Mal detektierbar, d.h. sichtbar wird. Der Delta-Ct-Wert berechnet sich in diesem Experiment aus der Differenz des Ct-Wertes nach Lyse mit Additiv und dem Ct-Wert bei Lyse ohne Trennungsadditiv. Je niedriger der Delta-Ct Wert ist, desto besser ist die Nachweisbarkeit von gDNA und desto besser konnte die gDNA aus dem Nukleoproteinkomplex durch das Additiv abgetrennt werden.

*Durchführung:* HepG2-Zellen wurden in einem geeigneten Medium über einen Zeitraum von 3 Tagen inkubiert. Als Vergleichsversuch wurden Zellen wurden in einem Lysepuffer, der Nonidet-P40, Polyethylenglycol, EGTA und EDTA enthielt, lysiert. Zusätzlich wurden in weiteren Versuchen Zellen in einem Lysepuffer lysiert, der neben den oben genannten Reagenzien auch noch jeweils die Additive Arginin (1 mM), 2,3-Dimethylpyrazin (30 mM), Tyrptophan (15 mM), Histidin (15 mM), Indazol (10 mM) oder Imidazol (15 mM) in den angegebenen Konzentrationen enthielt.
Jeweils 2 µl der so gewonnenen Nucleoproteinkomplexe enthaltenden Lysate wurden in eine Real-time PCRs überführt, um gDNA nachzuweisen.

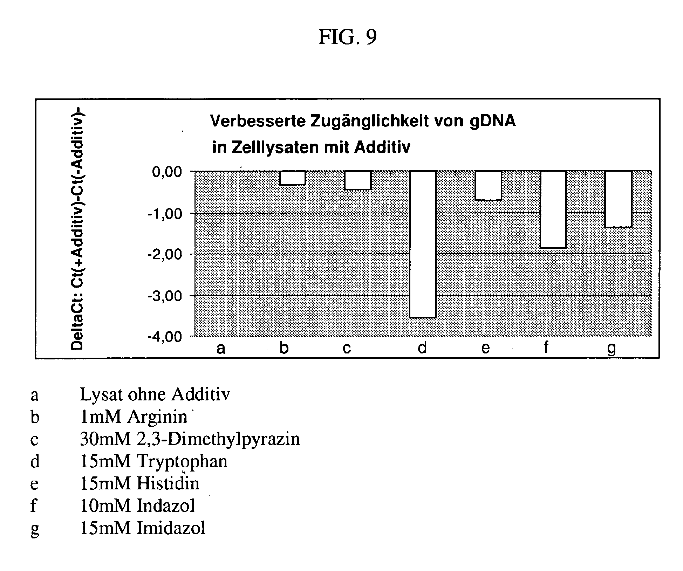

*Ergebnis:* Figur 9 zeigt einen Vergleich der jeweils ermittelten Delta-Ct-Werte. Bei allen Additiven ist sind gegenüber der Probe ohne Additiv erhöhte negative Delta-Ct-Werte gefunden worden. Daraus lässt sich schließen, dass sich zelluläre gDNA nach Trennung aus zellulären Nucleoproteinkomplexen durch die oben genannten Amine besser nachweisen lässt als die durch die Nucleoproteine komplexierte gDNA. Es konnten um 0,3 bis 3,5 bessere Delta-Ct Werte erreicht werden. Dies entspricht einer Verbesserung der Nachweisbarkeit von gDNA durch die Additiv-bedingte Trennung um ca. den Faktor 1,2 bis 10, je nach dem, welches Additiv zur Dekomplexierung der zellulären Nukleoproteinkomplexen eingesetzt wurde.

### Beispiel 10

### Verbesserung des enzymatischen Abbaus (DNase I) von gDNA aus zellulären Nukleoproteinkomplexen

Zellen wurden in einem Lysepuffer mit bzw. ohne Additiv lysiert. Eine Lysierung von Zellen, kann unter den hier gewählten Lysebedingungen zur Bildung eines Nukleoproteinkomplexes führen, so dass gDNA nur unzureichend für die DNase I zugänglich ist. Der DNase I Verdau der gDNA dient als Maß dafür, inwieweit die gDNA von den Nukleoproteinkomplexen getrennt vorliegt. Führt ein Additiv zu einer Dekomplexierung der gDNA aus den Nukleoproteinkomplexen, sollte die gDNA besser durch DNase I verdaut werden können. Der Nachweis der gDNA mittels eines Real-time Assays diente der Bestimmung des enzymatischen Abbaus durch DNase I. Bei der Real-time PCR ist der Ct-Wert der PCR-Zyklus, an dem das PCR-Signal zum ersten Mal detektierbar, d.h. sichtbar, wird. Der Delta-Ct₁-Wert berechnet sich in diesem Experiment aus der Differenz des Ct-Wertes in einer Probe unter Zugabe von Additiv aber ohne Zugabe von DNase I und des Ct-Wertes einer Probe, der sowohl Additiv als auch DNase I zugesetzt wurde. Der Delta-Ct₂-Wert berechnet sich in diesem Experiment aus der Differenz des Ct-Wertes in einer Probe ohne Zugabe von Additiv und DNase I und des Ct-Wertes einer Probe, der DNase I aber kein Additiv zugegegeben wurde. Der ΔDelta-Ct-Wert berechnet sich in diesem Experiment aus der Differenz: DeltaCt₁-DeltaCt₂. Je niedriger (hoher Betrag des negativen Wertes) der ΔDelta-Ct Wert ist, desto besser kann die gDNA durch die DNase I abgebaut werden, was ein Anzeichen einer verbesserten Zugänglichkeit der gDNA für die DNase I ist.

*Durchführung:* HepG2-Zellen wurden in einem geeigneten Medium über einen Zeitraum von 3 Tagen inkubiert. Zum einen wurden die Zellen in einem Lysepuffer, der Nonidet-P40, Polyethylenglycol, EGTA und EDTA enthielt, lysiert. Zum anderen wurden die Zellen in einem Lysepuffer, der neben den oben genannten Substanzen auch noch je eines der folgenden Additive in den jeweils angegebenen Konzentrationen enthielt: Arginin (1 mM), 2,3-Dimethylpyrazin (30 mM), Aminothazol (15 mM), Indazol (30 mM), Benzimidazol (15 mM), Imidazol (15 mM), Tryptophan (15 mM), Histidin (15 mM), Prolin (5 mM) oder Ammoniumsulfat (30 mM). Anschließend wurde wie oben beschrieben jeweils ein DNase I Verdau durchgeführt und jeweils 2 µl der so erhaltenen Reaktionslösung in Real-time PCRs überführt, um gDNA nachzuweisen.

*Ergebnis:* Figur 10 zeigt ein Diagramm, in der die verschiedenen ΔDelta-Ct-Werte für verschiedene Additive gegenübergestellt sind.
Alle eingesetzten Additve führten zu stärker negativen ΔDelta-Ct-Werten, was darauf hinweist, dass zelluläre gDNA aus zellulären Nucleoproteinkomplexen durch Dekomplexierung mittels der oben genannten Additive verstärkt durch DNase I abgebaut werden kann. Es wurden je nach Additiv 0,3 bis 10 Zyklen bessere ΔDelta-Ct Werte erhalten. Dies entspricht einer Verbesserung der enzymatischen Abbaubarkeit der gDNA durch DNase I, die durch die Zugabe der Additive erreicht wird, um den Faktor ~ 1,2 bis ~ 100.

Beispielsweise entspricht eine Ct-Differenz von 2 Zyklen einer Veränderung der für die PCR zur Verfügung stehenden Menge an Ausgangsmaterial, hier gDNA in der Probe, um den Faktor 4. Auf diese Wiese kann der Faktor mit der Verbesserung, mit dem die Zugänglichkeit der gDNA aus Nukleoproteinkomplexen durch ein Additiv verbessert wird, korreliert werden. Dieser Zusammenhang ist anhand idealisierter, hypothetischer Werte, die keinen Versuchen entstammen, in den Figuren 10.a.1 und 10.a.2 verdeutlicht.

### Beispiel 11:

Konzentrationsabhängigkeit der Verbesserung des enzymatischen Abbaus (DNase I) von gDNA aus zellulären Nukleoproteinkomplexen.

Die oben unter Beispiel 10 beschriebene Versuchsreihe wurde in analoger Weise unter Verwendung verschiedener Konzentrationen von Indazol als Additiv durchgeführt.

*Durchführung:* HepG2-Zellen wurden in einem geeigneten Medium für 3 Tage inkubiert. Die Zellen wurden in einem Lysepuffer, der Nonidet-P40, Polyethylenglycol, EGTA, EDTA und verschiedene Konzentrationen von Indazol enthielt, lysiert. Anschließend wurde wie oben beschrieben jeweils ein DNase I Verdau durchgeführt und jeweils 2 µl der so erhaltenen Reaktionslösung in Real-time PCRs überführt, um gDNA nachzuweisen.

*Ergebnis:* Figur 11 zeigt die Konzentrationsabhängigkeit des ΔDelta-Ct-Wertes von der Konzentration des dem Lysat zugegebenen Indazols. Der enzymatische Abbau der gDNA durch DNase I konnte bei einer Zugabe von 20 mM Indazol um einen Faktor von über 40 verbessert werden.

### Beispiel 12:

### Besserer DNase I Verdau von gDNA in gDNA/RNA Gemischen in Anwesenheit von Additiven

Es wird getestet, ob auch in Nukleinsäuregemischen aus RNA und DNA, die über Silica-Membranen gereinigt wurden (RNeays®, QIAamp®, QIAGEN GmbH, Hilden, Deutschland), eine Verwendung von Additiven zur Verminderung der Inhibierung durch Biomoleküle zu einem verbesserten enzymatischen Abbau durch DNase I führt. Dazu wurde gereinigte gDNA und gereinigte RNA in einem Lysepuffer mit bzw. ohne Additiv aufgenommen. Die Zugänglichkeit der gDNA für die DNase I wird durch einen enzymatischen Abbau mittels DNase I mit anschließender Feststellung des gDNA Gehaltes durch Real-time PCR bestimmt. Bei der Real-time PCR ist der Ct-Wert der PCR-Zyklus, an dem das PCR-Signal zum ersten Mal detektierbar, d.h. sichtbar wird. Der Delta-Ct-Wert berechnet sich in diesem Experiment aus der Differenz des Ct-Wertes nach Lyse mit Additiv und des Ct-Wertes bei Lyse ohne Additiv. Je niedriger, d.h. negativer, der Delta-Ct Wert ist, desto effektiver der enzyamtische Abbau, was wiederum auf eine verbesserte Zugänglichkeit der gDNA für die DNaseI hindeutet.

*Durchführung:* RNA wurde über Rneasy® (QIAGEN GmbH, Hilden, Deutschland) gereinigt. gDNA wurde über QIAamp® (QIAGEN GmbH, Hilden, Deutschland) gereinigt. Jeweils 20 ng der RNA und 20 ng der gDNA werden zu einer Probe vereinigt. Ein enzymatische Abbau durch DNase I wird ohne Additiv oder jeweils in Gegenwart von 400 µM Spermindin, 10 m Glutaminsäure, 10 mM Prolin, 10 mM Histidin, 10 mM Glycin, 10 oder 15 mM Imidazol, 5 mM Arginin, 5 mM Harnstoff, 15 mM Anthranilamid oder 15 mM Pyrimidin durchgeführt. Der DNase Verdau der Lysate wurde im QuantiTect® Real-time Puffer (QIAGEN GmbH, Hilden, Deutschland) durchgeführt, wobei 150 µM (als Endkonzentration) CaCl₂ zugesetzt wurden. Die Reaktionen enthielten jeweils 20 ng RNA und 20 ng gDNA, 0,5 U DNase (der Firma Ambion, USA), Primer, Nukleotide, HotstarTaq Polymerase und die angegebenen Additive. Gestoppt wurde die Reaktion durch Hitzeinaktivierung bei der PCR. Zur Bestimmung der restlichen gDNA wird eine Real-time PCR durchgeführt.

*Ergebnis:* Figur 12 zeigt eine Gegenüberstellung der jeweils erhaltenen Delta-Ct-Werte. Das Diagramm zeigt, dass in Gegenwart der verwendeten Additive gereinigte gDNA in gDNA/RNA Gemischen deutlich besser durch DNase I abgebaut wird. Es konnten um 2 bis 13 Zyklen verbesserte Delta-Ct-Werte gezeigt werden. Dies entspricht einer Verbesserung des Abbaus der gDNA durch die DNase I um den Faktor - 4 bis > 1000, je nach dem, welches Additiv verwendet wurde.

### Beispiel 13

### Verminderung der inhibitorischen Wirkung beim Nachweis von gDNA und RNA aus Zelllysaten.

Zellen wurden in einem Lysepuffer mit bzw. ohne Distamycin D lysiert. Eine Lysierung von Zellen, führt zur Bildung eines Nukleoproteinkomplexes, so daß gDNA nur unzureichend nachweisbar ist. Zur Dekomplexierung der gDNA oder RNA wurde Distamycin eingesetzt, dass primär doppelstrangige DNA in der kleinen Grube bindet. Zur Bestimmung der gDNA wurde in einer vergleichende Real-time PCR der Ct-Wert bestimmt. Die RNA wurde in vergleichender Real-time RT-PCR bestimmt. Der Ct-Wert ist der PCR-Zyklus, an dem das PCR-Signal zum ersten Mal detektierbar, d.h. sichtbar, wird. Der Delta-Ct-Wert berechnet sich in diesem Experiment aus der Differenz des Ct-Wertes nach Lyse mit Additiv und des Ct-Wertes bei Lyse ohne Additiv. Je niedriger, d.h. negativer, der Delta-Ct Wert ist, desto besser ist die Nachweisbarkeit von gDNA oder RNA und desto besser konnte die gDNA oder RNA aus dem Nukleoproteinkomplex durch das Additiv dekomplexiert werden.

*Durchführung:* HeLa-Zellen, die in Suspension wachsen, wurden in einem geeigneten Medium inkubiert. Zum einen wurden die Zellen in einem Lysepuffer, der Nonidet-P40, Polyethylenglycol, EGTA und EDTA enthielt, lysiert. Zum anderen wurden die Zellen in einem Lysepuffer, der neben den oben genannten Substanzen auch noch Distamycin D in verschieden Konzentrationen als Additiv enthielt, lysiert.
Jeweils 2 µl der jeweils so gewonnenen Lysate wurden in Real-time PCRs bzw. Real-time RT-PCRs überführt, um gDNA oder RNA nachzuweisen.

*Ergebnis:* Figur 13 zeigt ein Diagramm, in dem die jeweils gemessenen Delta-Ct-Werte gegenübergestellt sind. Aus dem Diagramm geht hervor, dass sowohl zelluläre gDNA als auch RNA nach der Dekomplexierung aus zellulären Nucleoproteinkomplexen durch Distamycin D wesentlich besser nachzuweisen ist, als in den Proben ohne Distamycin D. Der Grad der Verbesserung ist abhängig von der Konzentration an Distamycin. Für die gDNA konnten selbst bei kleinen Konzentration von 70 pM noch Verbesserungen des Ct-Wertes von > 4 Zyklen erreicht werden. Dies entspricht einer Verbesserung der um den Faktor > 10.
Die Ct-Wert-Verbesserungen beim Nachweis der RNA bei der Verwendung von Distamycin als Additiv, sind hier kleiner. Dies ist darauf zurückzuführen, dass Distamycin primär DNA bindet. Der erzielte Effekt beruht somit im Wesentlichen auf der Dekomplexierung von Protein-DNA-Komplexen, in welche die RNA eingewoben ist und ebenfalls freigesetzt wird. Der Effekt auf die Nachweisbarkeit von RNA ist somit im Wesentlichen indirekter Art.

### Beispiel 14

### Verminderung der inhibitorischen Wirkung auf RNA, normiert auf die Nachweisbarkeit von DNA.

Zellen wurden in einem Lysepuffer mit bzw. ohne Additiv lysiert. Die Lyse von Zellen führt zur Bildung von Nukleoproteinkomplexen, so dass RNA nur unzureichend nachweisbar ist. Zum Nachweis der RNA wurde in einer vergleichenden TwoTube Real-time RT-PCR der Ct-Wert bestimmt. Der Ct-Wert ist der PCR-Zyklus, an dem das PCR-Signal zum ersten Mal detektierbar, d.h. sichtbar, wird. Der Delta-Ct-Wert berechnet sich in diesem Experiment aus der Differenz des Ct-Wertes nach Lyse mit Additiv und des CT-Wertes bei Lyse ohne Additiv. Die Ct-Werte der RNA wurden normalisiert auf die Ct-Werte der gDNA. Eine Ct-Differenz von z.B. 2 Zyklen entspricht einer Veränderung der Zugänglichkeit der RNA durch die Reverse Transkriptase um den Faktor 4. Auf diese Weise kann der Faktor, mit dem die Zugänglichkeit der RNA aus Nukleoproteinkomplexen, verglichen mit der Zugänglichkeit der gDNA aus Nukleoproteinkomplexen, durch das jeweilige Additiv verbessert wird, bestimmt werden.

*Durchführung:* HepG2-Zellen wurden in einem geeigneten Medium für 3 Tage inkubiert. Zum einen wurden die Zellen in einem Lysepuffer, der Nonidet-P40, Polyethylenglycol, EGTA und EDTA enthielt, lysiert. Zum anderen wurden die Zellen in einem Lysepuffer, der neben den oben genannten Reagenzien jeweils noch eines der folgenden Additive in der angegebenen Konzentration enthielt, lysiert: Arginin (1 mM), 2,3-Dimethylpyrazin (30 mM), Aminothiazol (15 mM), Indazol (30 mM), Benzimidazol (15 mM), Histidin (15 mM), Prolin (5 mM), Tryptophan 10 mM, Indazol 30 mM), Ammoniumsulfat 30 mM oder Imidazol (15 mM) enthielt. Jeweils 2 µl der so gewonnenen Lysate wurden in einer Reverse Transkriptase Reaktion mit Omniscript RT ® (QIAGEN GmbH, Hilden, Deutschland) eingesetzt. Nach Abschluß der RT-Reaktion wurden jeweils ein 1 µl der Reaktionslösung in eine Real-time PCRs überführt, um die entsprechende cDNA nachzuweisen.

*Ergebnis:* Figur 14 zeigt ein Diagramm, in dem die jeweils erzielten Verbesserungen bei der Nachweisbarkeit von zellulärer RNA gegenüber gestellt sind. Das Diagramm zeigt, dass sich zelluläre RNA nach Dekomplexierung der zellulären Nucleoproteinkomplexe durch die oben genannten Additive besser nachweisen lässt, als RNA aus Lysatproben, denen kein Additiv zugegeben wurde. Es konnten Verbesserungen um den Faktor 2-1000 gemessen werden. Dies zeigt, dass es aufgrund der Verwendung der jeweiligen Additive zu einer deutlichen Dekomplexierung der RNA aus Nukleoproteinkomplexen kommt.

### IV. Maskierung

### Beispiel 15

### Differentielle Maskierung von DNA in Gemischen aus gereinigter genomischer DNA und gereinigter RNA

Maskierte DNA sollte schlechter amplifizierbar sein als RNA. Real-time PCR bzw. RT-PCR von DNA und RNA kann das Ausmaß der differentiellen Maskierung der DNA gegenüber der RNA zeigen, wenn Additive zur Maskierung der gDNA verwendet werden.
In diesem Experiment ist der Delta-Ct definiert als die Differenz des Ct-Wertes von Proben, denen Additiv zugegeben wurde, und des Ct-Wertes von Proben, denen kein Additiv zugegeben wurde. Ein hoher Delta-Ct-Wert zeigte die Maskierung der DNA oder der RNA an.

*Durchführung:* Humane gDNA und RNA wurde jeweils über QIAamp® (QIAGEN GmbH, Hilden, Deutschland) bzw. Rneasy® (QIAGEN GmbH, Hilden, Deutschland) gereinigt. 20 ng der gDNA und 20 ng der RNA wurden zu einer Probe vereinigt. Einigen Proben wurde Spermin bis zu einer Endkonzentration von 5 oder 20 µM zugegeben. 2 µl des Überstands nach Zentrifugation wurde in eine Real-time PCR oder in eine TwoTube Real-time RT-PCR überführt.

*Ergebnis:* Figur 15 zeigt ein Diagramm, in dem die jeweilig erhaltenen Delta-Ct-Werte gegenübergestellt sind. Dem Diagramm kann entnommen werden, dass während bei 20 µM Spermine beide Nukleinsäuren, gDNA und RNA, maskiert werden, bei Verwendung von 5 µM Spermine gDNA selektiv maskiert werden kann. Bei 5 µM Spermin konnte man eine Delta-Ct-Verschiebung von 6 Zyklen messen, die eine Maskierung der gDNA um den Faktor von - 500 an. Dagegen wurde für die RNA keine Delta-Ct-Verschiebung gefunden.

### Beispiel 16

### Maskierung von genomischer DNA in Zelllysaten durch verschiedene Additive

Maskierte DNA sollte schlechter amplifizierbar sein als RNA aus derselben Präparation bzw. demselben Lysat. Real-time PCR der maskierten DNA abgeglichen auf die Real-time RT-PCR von RNA sollte das Ausmaß der differentiellen Maskierung der DNA gegenüber der RNA zeigen.

In dem vorliegenden Experiment ist der Delta-Ct-Wert definiert als die Differenz von Ct-Wert von Proben, die durch Lyse mit Additiv erhalten wurden, und des Ct-Wertes von Proben, die durch Lyse ohne Additiv erhalten wurden. Der Delta-Ct Wert wurde normalisiert auf die Ct-Werte, die durch die RNA in der Real-time RT-PCR erreicht wurden.
Ein hoher Wert zeigte die Maskierung der DNA gegenüber der RNA an.

*Durchführung:* Zum einen wurde humane gDNA und RNA wurde mit einem detergenzhaltigen Lysepuffer (Nonidet-P40, Polyethylengylcol, EGTA und EDTA) aus HepG2 Zellen gewonnen. Zum anderen wurde dem Lysepuffer jeweils die Additive Ammouniumsulfat, Glycin oder Ammoniumhydrogenphosphat in einer Konzentration von 30 mM bzw. Harnstoff in einer Konzentration von 5 mM zur differentiellen Maskierung von gDNA zugegeben.
Anschließend wurde die differentielle Maskierung der DNA gegenüber RNA mittels Real-time PCR und Real-time RT-PCR bestimmt.

*Ergebnis:* Figur 16 zeigt ein Diagramm, in der die jeweils gemessenen Delta-Ct-Werte gegenübergestellt sind. Dem Diagramm kann entnommen werden, dass genomische DNA im Lysepuffer mit den genannten Additiven zu höheren Delta-Ct Werte normalisiert auf die Ct-Werte der RNA führt. Dies zeigt eine Maskierung der DNA an. Im Falle des Ammoniumsulfats wurde eine Erhöhung des Ct-Wertes um 7 Zyklen erreicht, was darauf hinwies, dass in dieser Lösung ca. 100x weniger DNA aufgrund der Maskierung nachgewiesen wurde.

### Beispiel 17

### Maskierung von genomischer DNA in Zelllysaten durch verschiedene Additive

Maskierte DNA sollte schlechter amplifizierbar sein als RNA aus derselben Präparation. Real-time PCR der maskierten DNA abgeglichen auf die Real-time RT-PCR von RNA sollte das Ausmaß der differentiellen Maskierung der DNA gegenüber der RNA zeigen.
In diesem Experiment ist der Delta-Ct-Wert definiert als die Differenz des Ct-Wertes (RNA) minus des Ct-Wertes (gDNA). Ein negativer Delta-Ct-Wert zeigt die Maskierung der DNA gegenüber der RNA an.

*Durchführung:* Zum einen wurde humane gDNA und RNA mit einem detergenzhaltigen Lysepuffer (Nonidet-P40, Polyethylengylcol, EGTA und EDTA) aus 40.000 HepG2 Zellen gewonnen. Zum anderen wurde dem Lysepuffer jeweils eines der folgenden Additive in den angezeigten Konzentrationen zur differentiellen Maskierung zugegeben: Glutaminsäure (15 mM), Arginin (5 mM), 2,3-Dimethylpyrazin (15 mM), Benzimidazol (15 mM), Imidazol (15 mM) oder Histidin (15 mM). Die Konzentrationen sind jeweils Endkonzentrationen. Anschließend wurde die differentielle Maskierung der DNA gegenüber RNA über Real-time PCR und Real-time RT-PCR bestimmt.

*Ergebnis:* Figur 17 zeigt die jeweils erhaltenden Delta-Ct-Werte. Aus dem Diagramm kann entnommen werden, dass genomische DNA im Lysepuffer mit den genannten Additiven zu negativen Delta-Ct-Werten führte. Dies zeigt eine Maskierung der DNA gegenüber der RNA an. Im Falle der Glutaminsäure sowie von Arginin, 2,3-Dimethylpyrazin und Imidazol wurde eine Maskierung von 4 Cts erreicht, was darauf hinwies, dass in dieser Lösung aufgrund der Maskierung ca. 10x weniger DNA nachgewiesen wurde.

### Beispiel 18

### Konzentrationsabhängigkeit der Maskierung von genomischer DNA in Zelllysaten

Maskierte DNA sollte schlechter amplifizierbar sein als RNA aus derselben Präparation. Real-time PCR der maskierten DNA abgeglichen auf die Real-time RT-PCR von RNA sollte das Ausmaß der differentiellen Maskierung der DNA gegenüber der RNA zeigen.
In diesem Experiment war der Delta-Ct definiert als die Differenz aus Ct-Wert (RNA) und Ct-Wert (gDNA). Ein negativer Delta-Ct-Wert zeigte die Maskierung der DNA gegenüber der RNA an.

*Durchführung:* Zum einen wurde humane gDNA und RNA mit einem detergenzhaltigen Lysepuffer (Nonidet-P40, Polyethylengylcol, EGTA und EDTA) aus HepG2 Zellen gewonnen. Zum anderen wurde dem Lysepuffer das Additiv Arginin jeweils in verschiedenen Konzentrationen zugefügt, um eine differentielle Maskierung zu erreichen. Die angegebenen Konzentrationen sind jeweils Endkonzentrationen des Arginins in dem Lysat. Anschließend wurde die differentielle Maskierung der DNA gegenüber RNA mittels Real-time PCR und Real-time RT-PCR bestimmt.

*Ergebnis:* Figur 18 zeigt ein Diagramm, in dem die jeweils erhaltenen Delta-Ct-Werte gegenüber gestellt sind. Aus dem Diagramm kann entnommen werden, dass genomische DNA im Lysepuffer bei Verwendung von Arginin in verschiedenen Konzentrationen negative Delta-Ct-Werte ergibt. Die Delta-Ct Werte sind umso negativer, je höher die Endkonzentration an Arginin im Puffer ist. Dies zeigt eine Maskierung der DNA gegenüber der RNA an. Maximale Maskierung wurde in dieser Versuchsreihe bei 6 mM Arginin erhalten. Ein Delta-Ct Wert von "-6" entspricht einem ungefähr 500fach schlechteren Nachweis der gDNA.

### Beispiel 19

### Maskierung von RNA in Zelllysaten durch verschiedene Additive

Maskierte RNA sollte schlechter amplifizierbar sein als gDNA aus derselben Präparation bzw. demselben Lysat. Two-tube Real-time RT-PCR der maskierten RNA abgeglichen auf die Real-time PCR von gDNA sollte das Ausmaß der differentiellen Maskierung der RNA gegenüber der DNA zeigen.
In diesem Experiment ist der Delta-Ct-Wert definiert als die Differenz aus Ct-Wert (DNA) und Ct-Wert (RNA). Ein negativer Delta-Ct-Wert zeigte die Maskierung der RNA gegenüber der DNA an.

*Durchführung:* Zum einen wurde humane gDNA und RNA mit einem detergenzhaltigen Lysepuffer (Nonidet-P40, Polyethylengylcol, EGTA und EDTA) aus HepG2 Zellen gewonnen. Zum anderen wurden dem Lysepuffer jeweils eines der folgenden Additive in den angegebenen Konzentrationen zugegeben, um RNA differentiell zu maskieren: Prolin (20 mM), Indazol (20 mM) oder Ammoniumsulfat (30 mM). Die Konzentrationen sind jeweils Endkonzentrationen. Anschließend wurde die RNA unter Verwendung des Lysats in einer 20 µl Reverse Transkriptase Reaktion eingesetzt. 2 µl der so erhaltenen Reaktionslösung wurde anschließend in die Real-time PCR überführt. Als Vergleich dazu wurde ein Real-time PCR ohne vorherige Reverse Transkriptase Reaktion durchgeführt.

*Ergebnis:* Figur 19 zeigt ein Diagramm, in dem die jeweils erhaltenen Delta-Ct-Werte gegenübergestellt sind. Aus dem Diagramm kann entnommen werden, dass RNA in den

Additiv enthaltenden Lysepuffern negative Delta-Ct-Werte ergab. Dies zeigt eine Maskierung der RNA gegenüber der gDNA an. Im Falle des Indazols wurde eine Maskierung von fast 6 Cts erreicht, was darauf hinweist, dass in dieser Lösung ca. 400x weniger RNA aufgrund der Maskierung nachgewiesen wurde.

### Beispiel 20:

Verwendung der erfindungsgemäßen Substanzen zur Behandlung von biologischen Proben oder Organismen und Messung der pH-Werte.

*Durchführung:* Zum einen wurde humane gDNA und RNA mit einem detergenzhaltigen Lysepuffer (EPE) aus HepG2 Zellen gewonnen. Diesem Lysepuffer wurde alternativ a) Arginin (5 mM), b) Arginin und Prolin (Konzentration je 5 mM), c) Imidazol (15 mM) oder d) Guanosin (20 mM) zugeführt. Nach Zugabe der Lösung zu den Zellen, wurde der pH-Wert des auf diese Weise gewonnenen Lysates bestimmt.

### Ergebnis: Die pH-Werte wurden wie folgt bestimmt:

| | | |
|---|---|---|
| a) | Probe behandelt mit Arginin-haltiger Lösung: | pH 7,5 bis 8 |
| b) | Probe behandelt mit Arginin-Prolin-haltiger Lösung: | pH 7,4 bis 8 |
| c) | Probe behandelt mit Imidazol-haltiger Lösung: | pH 7,5 bis 8 |
| d) | Probe behandelt mit Guanosin-haltiger Lösung: | pH > 9 |

## Patentansprüche

1. Verfahren zur Stabilisierung von Nukleinsäuren und/oder Proteinen umfassend:
A) Bereitstellen einer Probe, die zumindest eine Spezies einer Nukleinsäure und eines Proteins umfasst,
B) In-Kontakt-Bringen der Probe mit einer flüssigen oder festen Zusammensetzung, um eine flüssige Probenzubereitung zu erzeugen, wobei die Zusammensetzung zumindest eine stickstoffhaltige Verbindung enthält, die ausgesucht ist aus der Gruppe bestehend aus
a) Polyaminen,
b) Aminosäuren, sowie Oligo- und Polypeptide,
c) stickstoffhaltigen heterocyclischen Verbindungen, einschließlich Homo- oder Heteropolymeren, die diese stickstoffhaltigen Verbindungen umfassen,
d) Aminen vom Typ R¹R²NR³, wobei R¹, R² und R³ unabhängig voneinander ausgewählt sind aus der Gruppe bestehend aus H, C₁-C₅-Alkylgruppen und Arylgruppen, wobei R¹, R² und R³ nicht gleichzeitig H sind,
e) Carbonsäureamiden,
f) anorganischen Ammoniumsalzen,
g) Ammoniumgruppen enthaltenden inneren Salzverbindungen,
h) Nukleinsäure bindende Antibiotika,
i) Verbindungen, die in der kleinen Grube der DNA binden,
sowie Mischungen aus zwei oder mehreren dieser Verbindungen enthält,
**dadurch gekennzeichnet, dass** Menge der stickstoffllaltige(n) Verbindung(en) so gewählt wird, dass die in der Probe enthaltene(n) Nukleinsäure(n) und Protein(e) während des In-Kontakt-Bringens und danach nicht präzipitieren und dass sich im Vergleich von unbehandelter Probe zu Probenzubereitung die relative Konzentration der verschiedenen Spezies Nukleinsäuren und Proteine zueinander durch die Zugabe der die stickstoffhaltige Verbindung enthaltenden Zusammensetzung nicht verändert.

2. Verfahren gemäß Anspruch 1, **dadurch gekennzeichnet, dass** zumindest eine Spezies Nukleinsäure oder Protein vor, während oder nach der Zugabe der stickstoffhaltigen Verbindung auf einem Trägermaterial immobilisiert wird.

3. Verfahren gemäß Anspruch 1, **dadurch gekennzeichnet, dass** die Erzeugung der Probenzubereitung derart erfolgt, dass zumindest eine Spezies der Nukleinsäure und des Proteins in der flüssigen Probenzubereitung gelöst und/oder suspendiert wird.

4. Verfahren gemäß einem der Ansprüche 1 bis 3, **dadurch gekennzeichnet, dass** die Probe eine biologische Probe ist, die vor dem In-Kontakt-Bringen mit der Zusammensetzung zur Erzeugung der flüssigen Probenzubereitung einer Lyse und gegebenenfalls weiteren Aufreinigungsschritten unterzogen wird.

5. Verfahren gemäß einem der Ansprüche 1 bis 4, **dadurch gekennzeichnet, dass** die Probe eine biologische Probe ist, in der die zumindest eine Spezies einer Nukleinsäure und zumindest eines Proteins in einer Hülle enthalten ist und dass die Probe mit der flüssigen oder festen Zusammensetzung in Kontakt gebracht wird, um ein Lysat zu erzeugen.

6. Verfahren gemäß einem der Ansprüche 1 bis 5, **dadurch gekennzeichnet, dass** DNA und/oder RNA in der Probenzubereitung bzw. dem Lysat gelöst und/oder suspendiert oder immobilisiert wird.

7. Verfahren gemäß einem der Ansprüche 4 bis 6, **dadurch gekennzeichnet, dass** die Zusammensetzung als Lysereagenzien zumindest ein Reagenz aus der Gruppe der Komplexbildner, Detergentien, Substanzen zur Volumeneinengung und/oder Lösemittel enthält.

8. Verfahren gemäß einem der Ansprüche 4 bis 7, **dadurch gekennzeichnet, dass** die Probe vor der Lyse mit einem Waschpuffer, vorzugsweise mit einem hypotonen Waschpuffer, gewaschen wird.

9. Verfahren gemäß einem Ansprüche 1 bis 8, **dadurch gekennzeichnet, dass** die Stickstoffhaltige Verbindung ausgesucht ist aus der Gruppe
a) der Polyamine, wobei die Polyamine vorzugsweise ausgesucht sind aus der Gruppe umfassend unverzweigte Polyamine mit 2 bis 6 Aminogruppen, insbesondere Spermidin,
b) der polaren, basischen und sauren Aminosäuren, insbesondere Lysin, Arginin, Histidin, Glutaminsäure,
c) der heterocyclischen Verbindungen, die ausgesucht sind aus der Gruppe Imidazol und 2,3-Dimethylpyrazin, Pyrimidin und Guanin.

## Claims

1. Method for stabilizing nucleic acids and/or proteins comprising:
A) providing a sample which comprises at least one species of a nucleic acid and a protein,
B) contacting the sample with a liquid or solid composition in order to generate a liquid sample preparation, wherein the composition contains at least one nitrogen compound which is selected from the group consisting of
a) polyamines,
b) amino acids, an d al s O oligo- and polypeptides,
c) heterocyclic nitrogen compounds, including homo- or heteropolymers which comprise these nitrogen compounds,
d) amines of the type R¹R²NR³, wherein R¹, R² and R³, independently of one another, are chosen from the group consisting of H, C₁-C₅ alkyl groups and aryl groups, wherein R¹, R² and R³ are not simultaneously H,
e) carboxamides,
f) inorganic ammonium salts,
g) ammonium groups containing inner salt compounds,
h) nucleic-acid-binding antibiotics,
i) compounds that bind to the minor groove of DNA,
and also mixtures of two or more of these compounds,
**characterized in that** the amount of the nitrogen compound (s) is chosen such that the nucleic acid(s) and protein(s) contained in the sample do not precipitate during the contacting and thereafter, and that, compared with an untreated sample, for sample preparation the relative concentration of the various species of nucleic acids and proteins to one another is not changed by adding the composition containing the nitrogen compound.

2. Method according to Claim 1, **characterized in that** at least one species of nucleic acid or protein is immobilized on a support material before, during or after the addition of the nitrogen compound.

3. Method according to Claim 1, **characterized in that** the sample preparation is generated in such a manner that at least one species of nucleic acid and of protein is dissolved and/or suspended in the liquid sample preparation.

4. Method according to any one of Claims 1 to 3, **characterized in that** the sample is a biological sample which, before the contacting with the composition for generating the liquid sample preparation, is subjected to a lysis, and optionally other purification steps.

5. Method according to any one of Claims 1 to 4, **characterized in that** the sample is a biological sample in which the at least one species of a nucleic acid and of at least one protein is present in an envelope, and that the sample is contacted with the liquid or solid composition in order to generate a lysate.

6. Method according to any one of Claims 1 to 5, **characterized in that** DNA and/or RNA in the sample preparation or the lysate is dissolved and/or suspended or immobilized.

7. Method according to any one of Claims 4 to 6, **characterized in that** the composition, as lysis reagents, contains at least one reagent from the group of complexing agents, detergents, substances for volume reduction and/or solvents.

8. Method according to any one of Claims 4 to 7, **characterized in that** the sample, before lysis, is washed with a wash buffer, preferably with a hypotonic wash buffer.

9. Method according to any one of Claims 1 to 8, **characterized in that** the nitrogen compound is selected from the group consisting of
a) the polyamines, wherein the polyamines are preferably selected from the group consisting of unbranched polyamines having 2 to 6 amino groups, in particular spermidine,
b) the polar, basic and acidic amino acids, in particular lysine, arginine, histidine, glutamic acid,
c) the heterocyclic compounds which are selected from the group imidazole and 2,3-dimethylpyrazine, pyrimidine and guanine.

## Revendications

1. Procédé pour la stabilisation d'acides nucléiques et/ou de protéines, comprenant :
A) la mise à disposition d'un échantillon, qui comprend au moins une espèce d'un acide nucléique et d'une protéine,
B) la mise en contact de l'échantillon avec une composition liquide ou solide, pour produire une préparation échantillon liquide, la composition contenant au moins un composé azoté, qui est choisi dans le groupe consistant en :
a) les polyamines,
b) les acides aminés, ainsi que les oligo- et polypeptides,
c) les composés hétérocycliques azotés, y compris les homo- ou hétéropolymères qui comprennent ces composés azotés,
d) les amines du type R¹R²NR³, où R¹, R² et R³ sont chacun indépendamment des autres choisis dans le groupe consistant en H, les groupes alkyle en C₁-C₅ et les groupes aryle, R¹, R² et R³ ne représentant pas simultanément H,
e) les carboxamides,
f) les sels d'ammonium inorganiques,
g) les composés sels internes contenant des groupes ammonium,
h) les antibiotiques se liant aux acides nucléiques,
i) les composés qui se lient dans le petit sillon de l'ADN,
ainsi que les mélanges de deux ou plusieurs de ces composés,
**caractérisé en ce que** la quantité du ou des composés azotés est choisie de telle sorte que le ou les acides nucléiques et protéines contenus dans l'échantillon ne précipitent pas pendant la mise en contact et après cette dernière, et que, dans le cadre d'une comparaison de l'échantillon non traité et de la préparation échantillon, la concentration relative des différentes espèces d'acides nucléiques et de protéines, les unes par rapport aux autres, ne subit pas de modification sous l'effet de l'addition de la composition contenant le composé azoté.

2. Procédé selon la revendication 1, **caractérisé en ce qu'**on immobilise sur un matériau support au moins une espèce d'acide nucléique ou de protéine, avant, pendant ou après l'addition du composé azoté.

3. Procédé selon la revendication 1, **caractérisé en ce que** la production de la préparation échantillon a lieu par dissolution et/ou mise en suspension d'au moins une espèce de l'acide nucléique et de la protéine dans la préparation échantillon liquide.

4. Procédé selon l'une des revendications 1 à 3, **caractérisé en ce que** l'échantillon est un échantillon biologique, qui, avant la mise en contact avec la proposition destinée à la production de la préparation échantillon liquide, est soumis à une lyse et éventuellement à de s étapes supplémentaires de purification.

5. Procédé selon l'une des revendications 1 à 4, **caractérisé en ce que** l'échantillon est un échantillon biologique, dans lequel la ou les espèces d'un acide nucléique et d'au moins une protéine est contenue dans une enveloppe, et que l'échantillon est mis en contact avec la composition liquide ou solide pour produire un lysat.

6. Procédé selon l'une des revendications 1 à 5, **caractérisé en ce que** l'ADN et/ou l'ARN est dissous et/ou mis en suspension ou immobilisé dans la préparation échantillon ou dans le lysat.

7. Procédé selon l'une des revendications 4 à 6, **caractérisé en ce que** la composition contient, en tant que réactifs de lyse, au moins un réactif du groupe des complexants, des détergents, des substances pour la diminution du volume et/ou des solvants.

8. Procédé selon l'une des revendications 4 à 7, **caractérisé en ce que** l'échantillon est, avant la lyse, lavé avec un tampon de lavage, de préférence avec un tampon de lavage hypotonique.

9. Procédé selon l'une des revendications 1 à 8, **caractérisé en ce que** le composé azoté est choisi dans le groupe
a) des polyamines, les polyamines étant de préférence choisies dans le groupe comprenant les polyamines non ramifiées ayant 2 à 6 groupes amino, en particulier la spermidine,
b) des acides aminés polaires, basiques et acides, en particulier la lysine, l'arginine, l'histidine, l'acide glutamique,
c) des composés hétérocycliques, qui sont choisis dans le groupe de l'imidazole e t de la 2,3-diméthylpyrazine, de la pyrimidine et de la guanine.
